# EUROPEAN PATENT APPLICATION

(11) **EP 1 815 862 A1**
(43) Date of publication of application: **08.08.2007**
(21) Application number: 07008345.6
(22) Date of filing: 24.02.1999
(51) Int. Cl.: A61K 35/00, C12N 15/85, C12N 15/86

(54) **Isolated stromal cells for use in the tretment of diseases of the central nervous system**

(30) Priority: 24.02.1998 US 28395
(62) Divisional of application: 99908377.7
(71) Applicant: MCP Hahnemann University, Philadelphia, PA 19104 (US)
(72) Inventor: Prockop, Darwin J., Philadelphia, PA 19104 (US); Stokes, David G., Philadelphia, PA 19090 (US); Azizi, S. Austin, Philadelphia, PA 19129 (US); Phinney, Donald G., Maple Glen, PA 19002 (US)
(74) Representative: Grünecker, Kinkeldey, Stockmair & Schwanhäusser Anwaltssozietät

(57) **Abstract**

Methods of treating a human patient having a disease, disorder or condition of the central nervous system are disclosed. The methods include obtaining a bone marrow sample from a human donor, isolating stromal cells from the bone marrow sample, and administering the isolated stromal cells to the central nervous system of the human patient, wherein the presence of the isolated stromal cells in the brain effects treatment of the disease, disorder or condition. Stromal cells which are isolated may be cultured *in vitro,* they may be genetically engineered to produce therapeutic compounds, and/or they may be predifferentiated prior to administration into the central nervous system.

## Description

### FIELD OF THE INVENTION

The invention relates to compositions and methods of treating a mammal suffering from a disease, disorder or a condition associated with the central nervous system, using isolated stromal cells.

### BACKGROUND OF THE INVENTION

Neurological damage in a mammal having as its genesis trauma, tumor formation or a genetic or other component, is very difficult to treat and/or reverse in the mammal. One treatment for neurological damage to the central nervous system is neurotransplantation. Over the last few decades, neurotransplantation has been used to explore the development, plasticity, and regeneration of the central nervous system (McKay, 1997, Science 276:66-71). Also, neurotransplantation has been used to effect the repair and functional restoration of diseased and damaged nervous tissues (Bjorklund, 1993, Nature 362:414-415; Olson, 1997, Nature Med. 3:1329-1335; Spencer et al., 1992, N. Engl. J. Med. 327:1541-1548: Freed et al., 1992, N. Engl. J. Med 327:1549-1555; Kordower et al., 1995, N. Engl. J. Med. 332:1118-1124; Defer et al., 1996, Brain 119:41-50; Lopez-Lozano et al., 1997, Transp. Proc. 29:977-980; Rosenstein, 1995, Exp. Neurol. 33:106; Turner et al., 1993, Neurosurg. 33:1031-1037; Kang et al., 1993, J. Neurosci. 13:5203-5211; Andersson et al., 1993, Int J. Dev. Neurosci. 11:555-568; Sanberg et al., 1997, Nature Med. 3:1129-1132). In particular, a series of human patients with Parkinson's disease have been treated by neurotransplantation of mesencephalic cells obtained from 6 to 9 week old abortuses of human fetuses (Spencer et al., 1992, N. Engl. J. Med. 327:1541-1548: Freed et al., 1992, N. Engl. J. Med 327:1549-1555; Kordower et al., 1995, N. Engl. J. Med. 332:1118-1124; Defer et al., 1996, Brain 119:41-50; , Lopez-Lozano et al., 1997 Transp. Proc. 29:977-980). Some of the patients exhibited significant improvement both in clinical symptoms and in the synthesis of dopamine, as assessed by fluorodopa uptake using positron-emission tomography (Spencer et al., 1992, N. Engl. J. Med. 327:1541-1548: Freed et al., 1992, N. Engl. J. Med 327:1549-1555; Kordower et al., 1995, N. Engl. J. Med. 332:1118-1124; Defer et al., 1996, Brain 119:41-50). However, the process of obtaining fetal tissue for therapeutic uses has presented major logistic and ethical barriers (Rosenstein, 1995, Exp. Neurol. 33:106; Turner et al., 1993, Neurosurg. 33:1031-1037). Also, only about 5 to 10% of dopaminergic neurons survive, apparently because of adverse immune reaction to the same (Lopez-Lozano et al., 1997, Transp. Proc. 29:977-980) and because the fetal tissue is primarily dependent on lipid instead glycolytic metabolism (Rosenstein, 1995, Exp. Neurol. 33:106). For these reasons, attempts have been made to develop alternative cells such as fibroblasts (Kang et al., 1993, J. Neurosci. 13:5203-5211), fetal astrocytes (Andersson et al., 1993, Int. J. Dev. Neurosci. 11:555-568), and sertoli cells (Sanberg et al., 1997, Nature Med. 3:1129-1132) which are suitable for neurotransplantation.

In order to treat diseases, disorders, or-conditions of the central nervous system, such as for example brain tumors, brain trauma, Huntington's disease, Alzheimer's disease, Parkinson's disease, and spinal cord injury, by transplantation, donor cells should be easily available, capable of rapid expansion in culture, immunologically inert, capable of long term survival and integration in the host brain tissue, and amenable to stable transfection and long-term expression of exogenous genes (Bjorklund, 1993, Nature 362:414-415; Olson, 1997, Nature Med. 3:1329-1335). Donor cells meeting these criteria are not currently available.

In addition to hematopoietic stem cells, bone marrow contains stem-like precursors for non-hematopoietic cells, such as osteoblasts, chondrocytes, adipocytes and myoblasts (Owen et al., 1988, in Cell and Molecular Biology of Vertebrate Hard Tissues, Ciba Foundation Symposium 136, Chichester, UK, pp. 42-60; Caplan, 1991, J. Orthop. Res. 9:641-650; Prockop, 1997, Science 276:71-74). Non-hematopoietic precursors of the bone marrow have been variously referred to as colony-forming-unit-fibroblasts, mesenchymal stem cells, and marrow stromal cells (MSCs).

MSCs are mesenchymal precursor cells (Friedenstein et al., 1976, Exp. Hemat. 4:267-274) that are characterized by their adherence properties when bone marrow cells are removed from a mammal and are transferred to plastic dishes. Within about four hours, MSCs adhere to the plastic and can thus be isolated by removing non-adherent cells from the dishes. Bone marrow cells, i.e., MSCs, that tightly adhere to plastic have been studied extensively (Castro-Malaspina et al., 1980, Blood 56:289-30125; Piersma et al., 1985, Exp. Hematol 13:237-243; Simmons et al.,1991, Blood 78:55-62; Beresford et al.,1992, J. Cell. Sci. 102:341- 351; Liesveld et al.,1989, Blood 73:1794-1800; Liesveld et al., Exp. Hematol 19:63-70; Bennett et al.,1991, J. Cell. Sci. 99:131-139). The terms "MSCs" and "stromal cells" are used interchangeably herein.

Stromal cells are believed to participate in the creation of the microenvironment within the bone marrow in vivo. When isolated, stromal cells are initially quiescent but eventually begin dividing so that they can be cultured in vitro. Expanded numbers of stromal cells can be established and maintained. Stromal cells have been used to generate colonies of fibroblastic adipocytic and osteogenic cells when cultured under appropriate conditions. They can also be made to differentiate into cartilage cells and myoblasts. If the adherent cells are cultured in the presence of hydrocortisone or other selective conditions, populations enriched for precursors or osteogenic cells are obtained (Carter et al., 1992, Blood 79:356-364 and Bienzle et al., 1994, Proc. Natl. Acad. Sci USA, 91:350-354).

There are several examples of the use of stromal cells for treatment of disease. For example, European Patent EP 0,381,490, discloses gene therapy using stromal cells. In particular, a method of treating hemophilia is disclosed. Stromal cells have been used to produce fibrous tissue, bone or cartilage when implanted into selective tissues in vivo (Ohgushi et al., 1989, Acte. Orthop. Scand. 60:334-339; Nakahara et al., 1992, J. Orthop. Res. 9:465-476; Niedzwiedski et al., 1993, Biomaterials 14:115-121; and Wakitani et al., 1994, J. Bone & Surg. 76A:579-592). In some reports, stromal cells have been used to generate bone or cartilage in vivo when implanted subcutaneously with a porous ceramic (Ohgushi, et al., 1989, Acta. Orthop. Scand. 60:334-339), intraperitoneally in a diffusion chamber (Nakahara et al., 1991, J. Orthop. Res. 9:465-476), percutaneously into a surgically induced bone defect (Niedzwiedski, et al., 1993, Biomaterials 14: 115-121), or transplanted within a collagen gel to repair a surgical defect in a joint cartilage (Wakitani et al., 1994, J. Bone Surg. 76A: 579-592). Piersma et al. (1983, Brit. J. Hematol. 94:285-290) disclose that after intravenous bone marrow transplantation, the fibroblast colony-forming cells which make up the hemopoietic stroma lodge and remain in the host bone marrow. Stewart et al. (1993, Blood 81:2566-2571) recently observed that unusually large and repeated administrations of whole marrow cells produced long-term engraftment of hematopoietic precursors into mice that had not undergone marrow ablation. Also, Bienzle et al. (1994, Proc. Natl. Acad. Sci. USA, 91:350-354) successfully used long-term bone marrow cultures as donor cells to permanently populate hematopoietic cells in dogs without marrow ablation. In some reports, stromal cells were used either as cells that established a microenvironment for the culture of hematopoietic precursors (Anklesaria, 1987, Proc. Natl. Acad. Sci. USA 84:7681-7685) or as a source of an enriched population of hematopoietic stem cells (Kiefer, 1991, Blood 78(10):2577-2582).

Given the paucity of successful treatments for diseases, disorders and conditions of the central nervous system, there remains a need for additional methods of treating patients affected by a disease, disorder, or condition of the central nervous system. The present invention satisfies this need and overcomes the deficiencies of prior art treatments.

### SUMMARY OF THE INVENTION

The invention includes a method of treating a human patient having a disease, disorder or condition of the central nervous system. The method comprises obtaining a bone marrow sample from a human donor, isolating stromal cells from the bone marrow sample, and administering the isolated stromal cells to the central nervous system of the human patient, wherein the presence of the isolated stromal cells in the central nervous system effects treatment of the disease, disorder or condition.

In one aspect, wherein the human donor is not suffering from a disease, disorder or condition of the central nervous system and wherein the human donor is synergeneic with the patient.

In another aspect, the human donor is the human patient.

In another aspect, the disease, disorder or condition of the central nervous system is selected from the group consisting of a genetic disease, a tumor, trauma and stroke.

In another aspect, the disease, disorder or condition is injury to the tissues or cells of the central nervous system.

In yet another aspect, the disease, disorder or condition is a brain tumor.

In one embodiment, the isolated stromal cells administered to the central nervous system remain present or replicate in the central nervous system.

In another embodiment, prior to administering the isolated stromal cells, the cells are cultured in vitro.

In yet another embodiment, prior to administering the isolated stromal cells, the isolated stromal cells are transfected with an isolated nucleic acid encoding a therapeutic protein, wherein when the protein is expressed in the cells the protein serves to effect treatment of the disease, disorder or condition.

Preferably, the therapeutic protein is selected from the group consisting of a cytokine, a chemokine, a neurotrophin and antibody and a glioma toxic protein.

In another embodiment, prior to administering the isolated stromal cells, the isolated stromal cells are transfected with an isolated nucleic acid encoding a therapeutic protein wherein when such protein is secreted by the cells the protein serves to effect treatment of the disease, disorder or condition.

In one embodiment, the isolated nucleic acid is operably linked to a promoter/regulatory sequence.

In another embodiment, the therapeutic protein is selected from the group consisting of a cytokine, a chemokine, a neurotrophin an antibody and a glioma-toxic protein.

The isolated nucleic acid in the method of the invention may be a wild type copy of a mutated, non-functioning or under-expressed gene, wherein the isolated nucleic acid is operably linked to a promoter/regulatory sequence and is expressed in the isolated stromal cells.

In yet another aspect of the invention, prior to administrating the stromal cells, the cells are pre-differentiated by coculturing the stromal cells in the presence of a substantially homogeneous population of differentiated cells or in the presence of an inducer of cell differentiation, whereby the isolated stromal cell differentiates and acquires the phenotypic characteristics of the differentiated cells or of the differentiation phenotype characteristic of a cell treated with the inducer.

In another aspect, prior to administration of the isolated stromal cells at least one of the steps of culturing the cells in vitro, introducing isolated nucleic acid into the cells, and pre-differentiating the cells, is performed.

In a further aspect, the isolated stromal cells are immunologically isolated.

The invention also includes a method of directing the differentiation of an isolated stromal cell. The method comprises culturing the isolated stromal cell in the presence of a substantially homogeneous population of differentiated cells or in the presence of an inducer of cell differentiation, whereby the isolated stromal cell differentiates and acquires the phenotypic characteristics of the differentiated cells or of the differentiation phenotype characteristic of a cell treated with the inducer.

Preferably, the differentiated cells are selected from the group consisting of astrocytes, macroglial cells and neurons.

When a brain tumor is to be treated, in one embodiment, the brain tumor is a glioma.

When a therapeutic protein is used, in one embodiment, the therapeutic protein is a glioma-toxic protein. Preferably, the glioma-toxic protein is Fas ligand, a bispecific single chain antibody directed against epidermal growth factor receptor (EGFR) and CD3, or an antibody directed against epidermal growth factor receptor (EGFR).

In certain embodiments wherein transfection of cells is performed, the transfection is conducted using a retroviral vector.

In certain other embodiments, the method of administering the isolated stromal cells is by engraftment of the cells directly into the brain.

Further, in other embodiments, the isolated stromal cells administered to the central nervous system remain present or replicate in the central nervous system for up to about 150 days.

In yet other embodiments, after administering the isolated stromal cells to the central nervous system, about 0.1% of the isolated stromal cells administered to the central nervous system are capable of expressing a marker for differentiation into macroglial cells, astrocytes or neurons. Preferably, the marker for differentiation is glial fibrillary acidic protein.

In one aspect, the disease of the central nervous system is Parkinson's disease.

In other embodiments when a therapeutic protein is used, the therapeutic protein is an enzyme required in the biosynthesis of a neurotransmitter that is deficient or not produced in a disease, disorder or condition of the central nervous system. Preferably, the neurotransmitter is L-DOPA and the treatment results in a decrease in an observed phenotype for Parkinson's disease. Also preferably, the observed phenotype for Parkinson's disease is apomorphine-induced rotation in a rat model for Parkinson'd disease. Further preferably, the isolated stromal cells used in the method of the invention continue to synthesize and secrete L-DOPA for at least about 3 days, and the L-DOPA is converted to metabolites of L-DOPA.

### BRIEF DESCRIPTION OF THE DRAWINGS

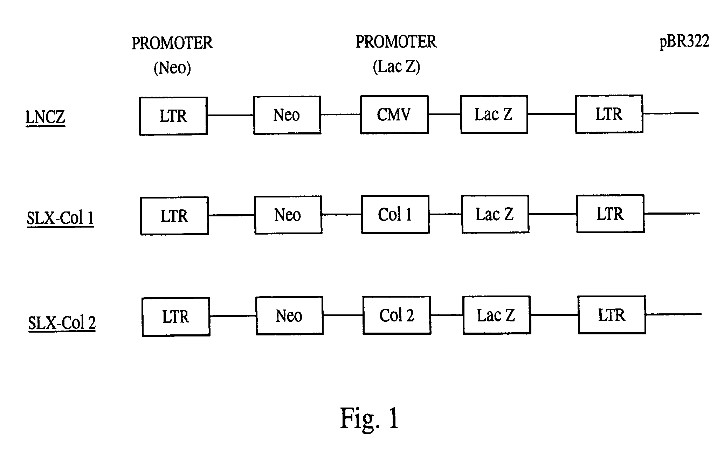

Figure 1 is a schematic of the retroviral constructs pCMV-lac Z, pCOLI-lac Z, and pCOL2-lac Z. The cassettes of the gene constructs are: LTR-Neo-promoter-Lac Z-30 LTR.

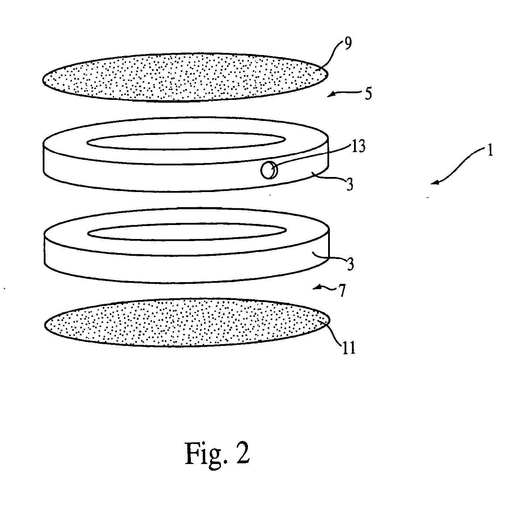

Figure 2 is a schematic illustration of a diffusion chamber.

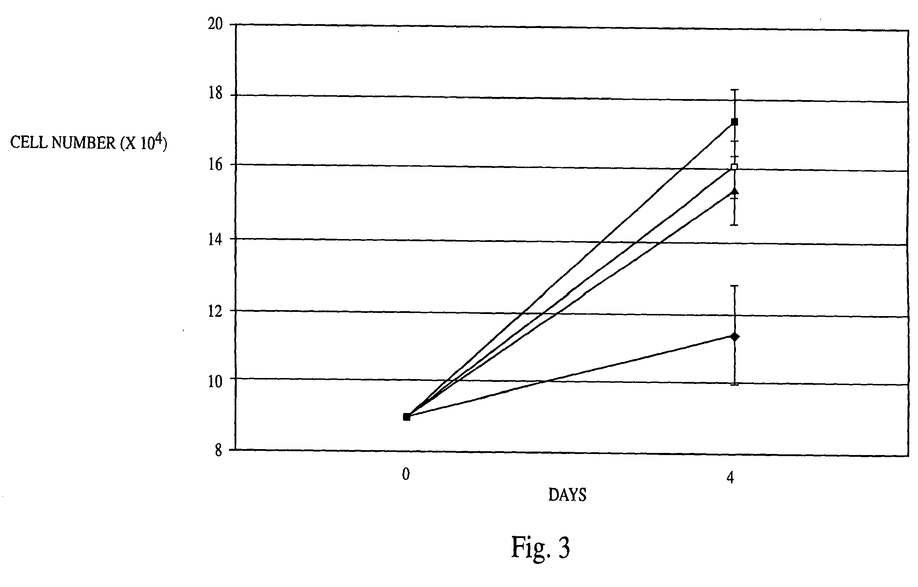

Figure 3 is a graph depicting the effect of PDGF-AA on the growth of human of MSCs. The following symbols are represented: ◆, control; ▲, 2-5 ng/ml PDGF; □, 2-5 ng/ml PDGF; ■, 2-5 ng/ml PDGF.

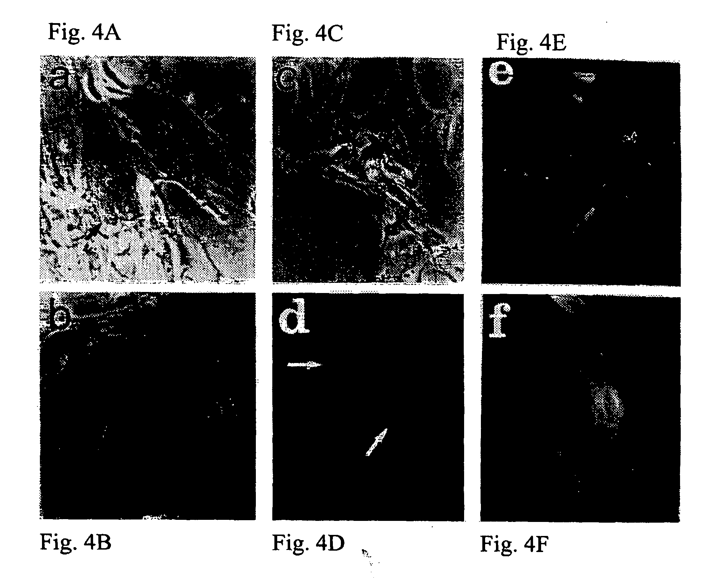

Figures 4A-4F, are images of a series of photomicrographs illustrating the morphological characteristics of MSCs and astrocytes in culture. Figures 4A and 4B depict two types of human MSCs, flat and elongated. Figure 4C depicts rat MSCs. Figure 4D depicts fluorescent labeled nuclei of human MSCs prior to implantation. Figures 4E and 4F depict indirect immunofluorescent staining of astrocytes with antibodies against vimentin and glial fibrillary acidic protein.

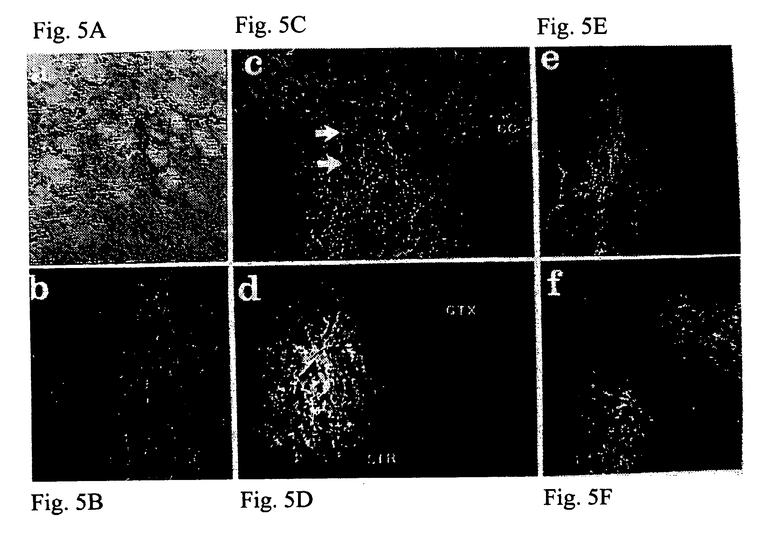

Figures 5A-5F, are images of a series of photomicrographs depicting the site of implantation of bone marrow stromal cells in the striatum of adult rats. Figure 5A depicts the site of implantation of human MSCs after 14 days. Figure 5B depicts an adjacent section to that shown in Figure 5A, wherein human MSCs were stained with antibodies to HLA-ABC. Figure 5C depicts an adjacent section to that in Figure 5A, wherein cells were examined for nuclear fluorescence of human MSCs. Figure 5D depicts the site of implantation of human MSCs after 72 days. Figure 5E depicts the same site of implantation as in Figure 5D of human MSCs after 30 days. Figure 5F depicts the site of implantation of human MSCs after 14 days. These cells were examined by nuclear fluorescence.

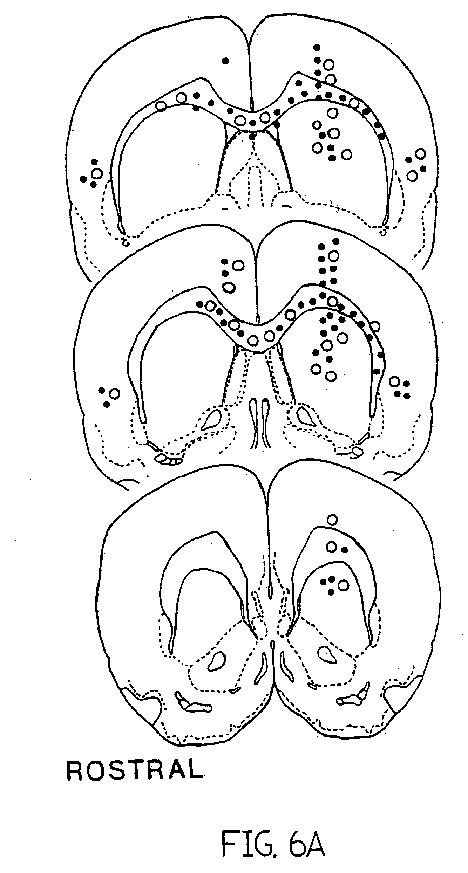

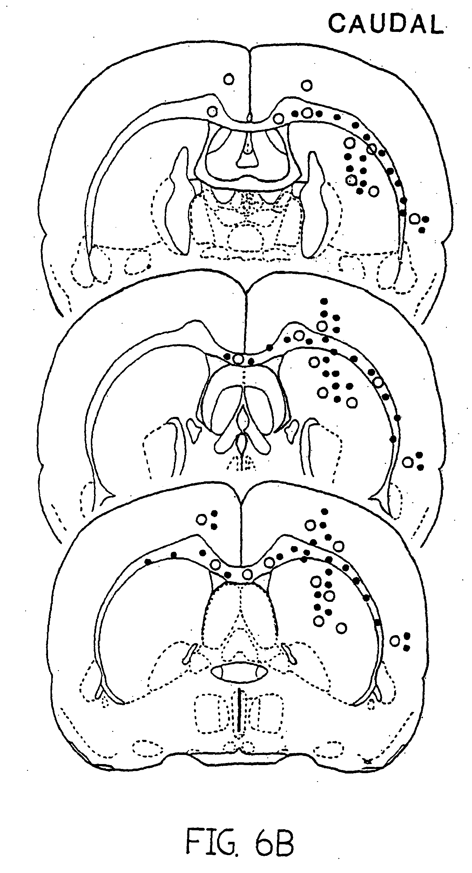

Figure 6, comprising Figures 6A and 6B, is a series of line drawings of rat forebrain, illustrating the migration of MSCs through the brain. The drawing is a composite from brains examined at 4, 14, 30 and 72 days after cell infusion. The pattern of implantation and migration of human MSCs is similar to that of rat astrocytes. The outlying cells, located in the temporal cortex and areas of corpus callosum farthest from the injection sites, were first to disappear. The following symbols are represented: •, human MSCs; o, rat astrocytes.

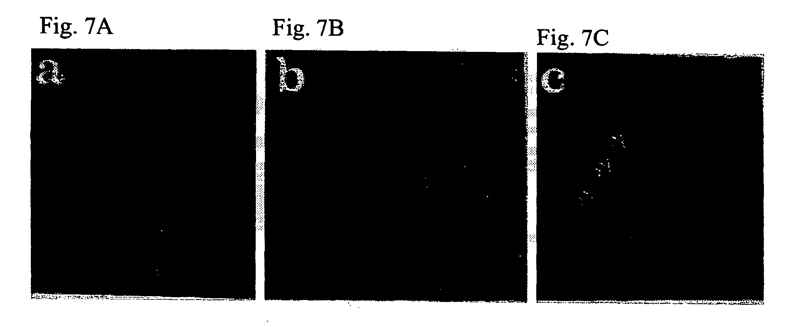

Figures 7A-7C, are images of a series photomicrographs depicting antibody staining to collagen I and fibronectin. Figure 7A depicts human MSCs stained with antibodies to collagen I prior to implantation. Figure 7B depicts human MSCs stained with antibodies to fibronectin. Figure 7C depicts a section of a rat brain stained for fibronectin 5 days after implantation of human MSCs.

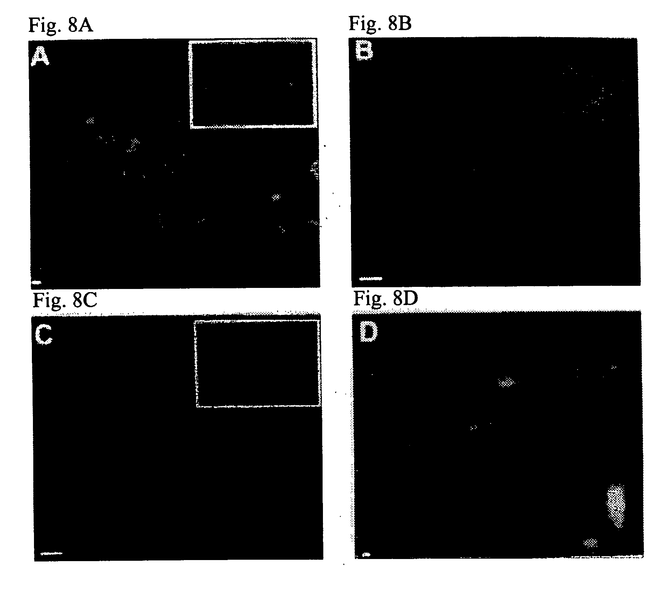

Figures 8A-8D are images of a series of photomicrographs depicting the immunostaining of rat MSCs in culture. Figure 8A depicts indirect immunofluorescent staining for Vimentin at a magnification of 20 x, (scale bar = 0.1 mm) where the insert is an image of a positive control of fibroblasts at a magnification of 20 x. Figure 8B depicts indirect immunofluorescent staining for Nestin at a magnification of 40 x , where the insert is a phase overlay of the same field (scale bar = 0.1 mm). Figure 8C depicts indirect immunofluorescent staining for GFAP at a magnification of 40 x, where the insert is an image of a positive control of astrocytes at a magnification of 20 x (scale bar = 0.1 mm). Figure 8D depicts indirect immunofluorescent staining for S100 with phase overlay at a magnification of 20 X (scale bar = 0.1 mm).

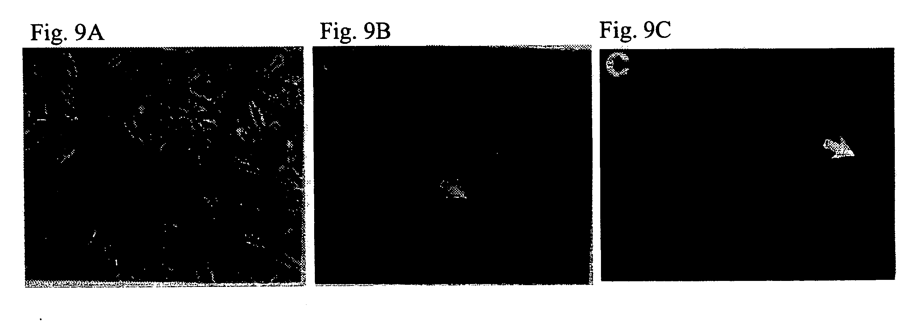

Figures 9A-9C are images of a series of photomicrographs depicting quantification of primary cultures of rat forebrains after implantation ofHMSCs. Figure 9A depicts a non-confluent region of rat forebrain mixed cell culture. HMSCs cannot be differentiated morphologically from endogenous brain cells. Figure 9B depicts harvested cultured cells in a hemocytometer viewed under a phase microscope. Figure 9C depicts the same cells as in Figure 9b visualized with a fluorescent microscope demonstrating *bis-*benzamide labeled hMSCs, which were implanted into the rat brain 30 days prior to culture.

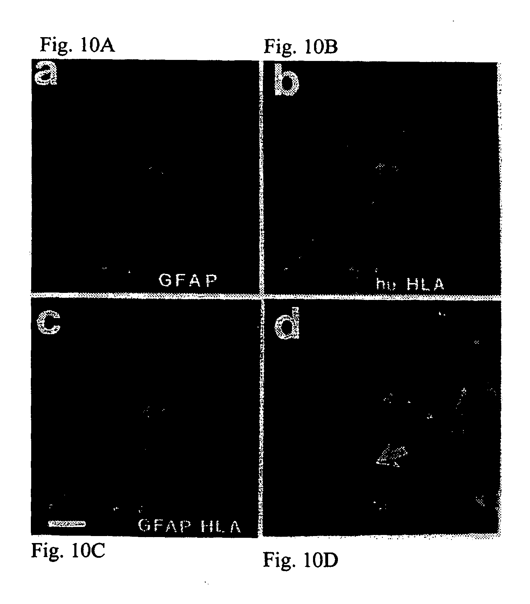

Figures 10A-10D, are images of a series of photomicrographs depicting recovery of human cells from astrocyte-enriched cultures of rat brain. Cultures of rat brain were prepared two weeks after implantation of hMSCs. Samples were immunocytochemically stained. Figures 10A-D depict an isolated cell in culture stained as follows: Figure 10A depicts staining with antibodies to GFAP (against human GFAP raised in rabbit, DAKO); Figure 10B depicts staining with antibodies to human HLA-ABC (raised in mouse, Pharmingen); Figure 10C depicts staining with double labeled antibodies (magnification 40x, scale bar 25 µm); and Figure 10D depicts staining with double labeled antibodies in different sample of cultured rat brain cells.

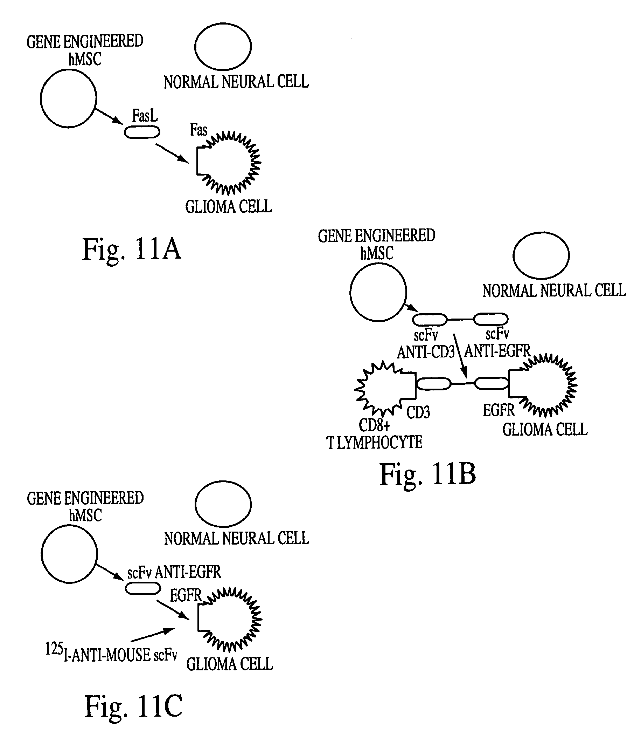

Figure 11, comprising Figures 11A, 11B, and 11C, is a trio of schematic illustration of the strategies used to engineer hMSCs to secrete tumor-toxic proteins.

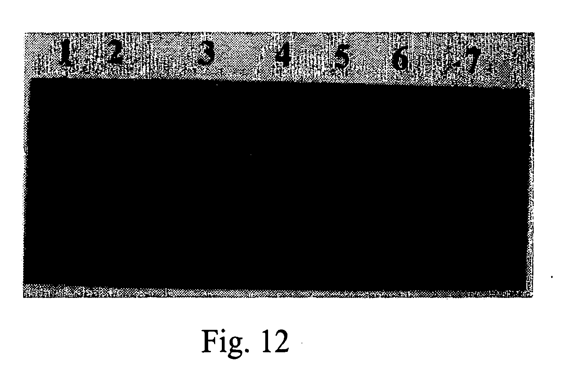

Figure 12 is an image of a Western blot assay demonstrating synthesis and secretion of soluble FasL by COS cells transfected with a cDNA for soluble (truncated) FasL. The cDNA was contained within the plasmid pSecTag²/Hyg. The cells were transiently transfected with lipofectamine and assayed by Western blotting as described by Rensing-Ehl et al., (1995, Eur. J. Immunol. 25:2253-2258). Lane 1 depicts cell lysate (14 µg protein) obtained from transfected cells (about 5 x 10⁵ in 2 ml medium) incubated for 2 days. Lane 2: Same as in Lane 1 following 4 days of incubation. Lane 3: Cell lysate obtained from mock transfected cells incubated for 4 days. Lane 4: Medium (18 µl) not concentrated from transfected cells after incubation for 1 day. Lane 5: Same as lane 4 following 2 days of incubation. Lane 6: Same as lane 4 following 4 days of incubation. Lane 7: Medium obtained from mock transfected cells following 4 days of incubation. Note: Diffuse white bands in lanes 4 to 7 are large concentrations of albumin in the samples of medium that reduce the background for the Western blot.

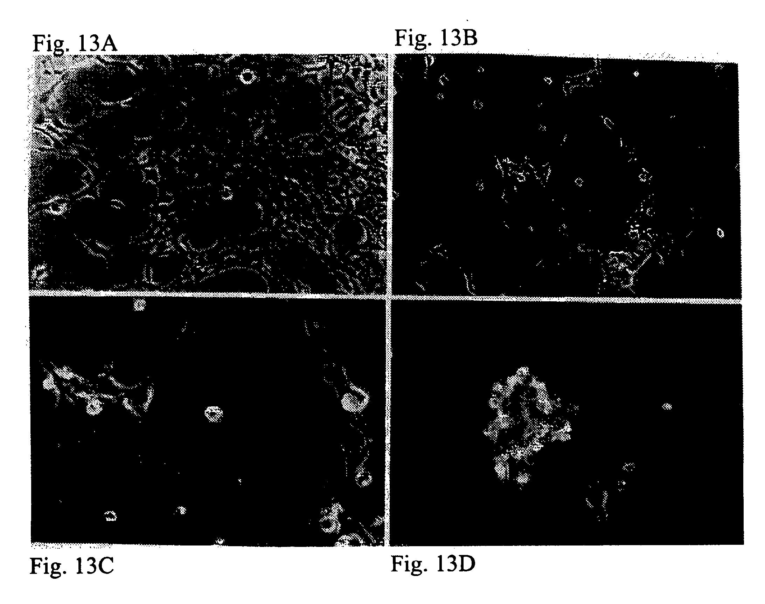

Figures 13A-13D, are images of a series of photomicrographs depicting inhibition of growth and killing of 293 cells (Fas⁺) by medium obtained from COS cells transiently expressing a cDNA encoding soluble FasL. About 5 x 10⁵ 293 cells were incubated with 1 ml fresh medium plus 1 ml of medium obtained from transfected COS cells at 48 hours following transfection. The 293 cells are shown at 3 days of incubation. The treatment reduced the viable cells by over 90%. Figure 13A depicts control 293 cells incubated with 1 ml of medium obtained from mock transfected COS cells. Figure 13B depicts test 293 cells incubated with 1 ml of medium from COS cells expressing soluble FasL. Phase contrast magnification was x 100. Figures 13C and 13D depict the same cultures as Figure 13B at the higher magnification of x 200. The appearance of the cells suggests that they are apoptotic.

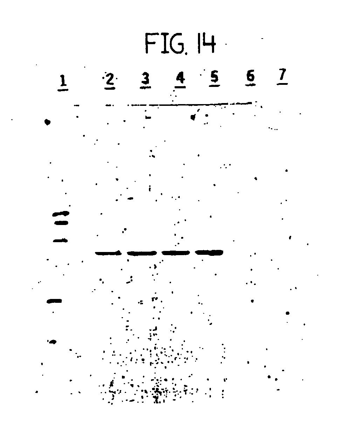

Figure 14 is an image of a Western blot of rat MSCs transduced with a retrovirus (pLNCX) containing a test gene (tyrosine hydroxylase). Conditions for retrovirus transduction were as described herein at Example 10. The Western blot assay was carried out with cell lysates and an antibody for tyrosine hydroxylase. Lane 1: MW markers. Lanes 2-5: Cell lysates of transduced MSCs (5 or 10 µg protein). Lane 6: Cell lysates of untransduced MSCs. Lane 7: Cell lysate of control cells (293).

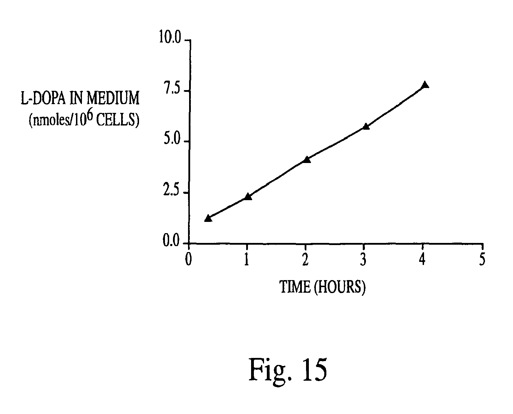

Figure 15 is a graph depicting the synthesis and secretion of the product (L-DOPA) by human MSCs transduced with a retrovirus containing a test cDNA (tyrosine hydroxylase). The cells were incubated with the essential cofactor 50 pM tetrahydropteridine, and L-DOPA produced in the medium was assayed by HPLC. No L-DOPA was detected in medium obtained from control MSCs.

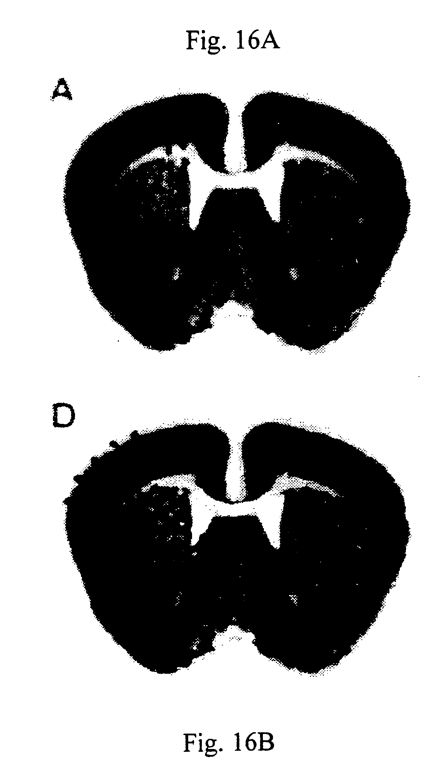

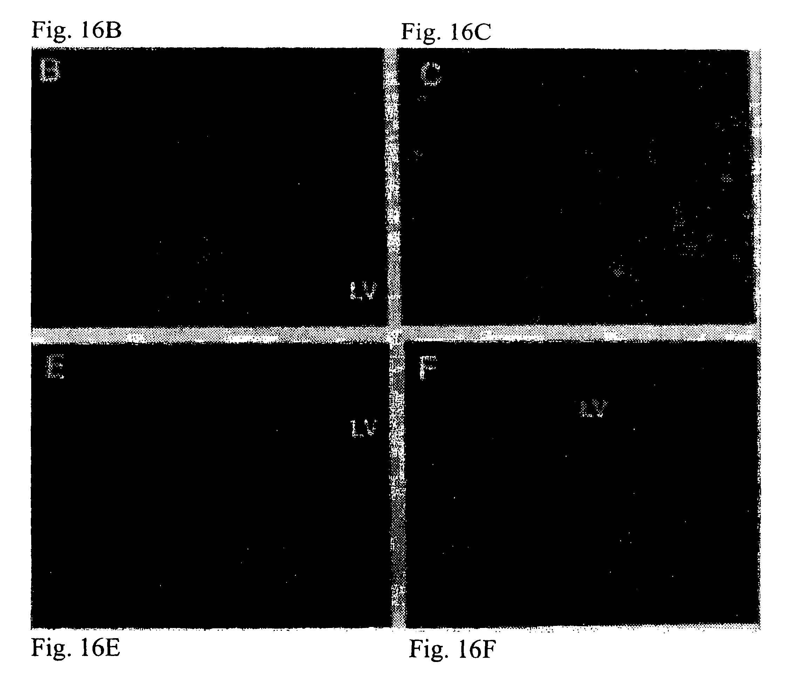

Figures 16A-16F, are images of schematics and a series of images depicting the engraftment of mouse MSCs after injection into the brains of neonatal mice. Figure 16A is a schematic depicting the distribution of fluorescently labeled nuclei at 12 days after infusion. Figure 16B is an image depicting fluorescently labeled nuclei at the injection site adjacent to the left lateral ventricle. Figure 16C is an image at a higher magnification that was used to count the freshly labeled nuclei of donor cells. Twelve serial sections were counted. Figure 16D is a schematic of the distribution of fluorescently labeled donor cells at 45 days. Figure 16E is an image showing the appearance of donor cells along the injection track adjacent to the left lateral ventricle throughout the striatum. Figure 16F is an image depicting the detection of donor cells expanding into the sematosensory cortex along the arachnoid mater and migrating into the ventral hippocampal commissure.

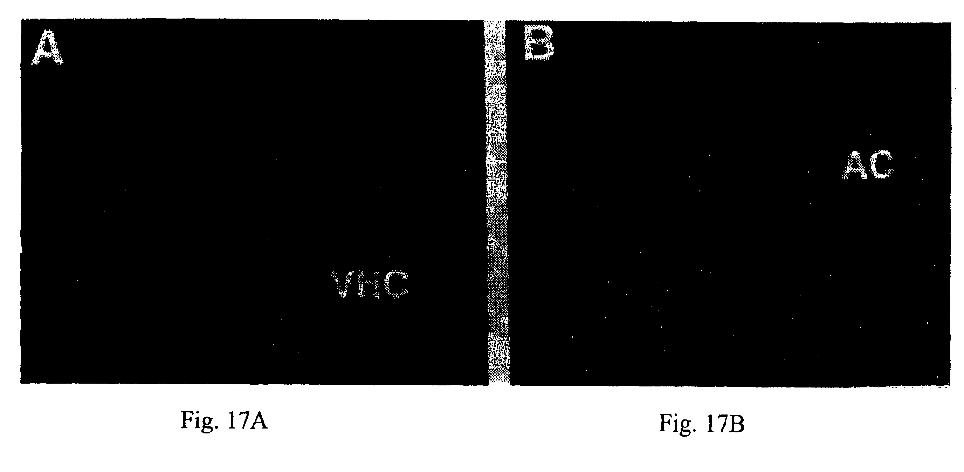

Figures 17A and 17B, are images of a pair of images further demonstrating the migration of the donor mouse MSCs 45 days after infusion into neonatal brain. The images are at higher magnification than that shown previously and demonstrate that the cells align themselves in linear arrays and appear to be migrating along white metered tracs within the external capsule, corpus callosum, anterior commisssure (AC), ventral hippocampal commissure (VHC), and along fiber bundles of the striatum.

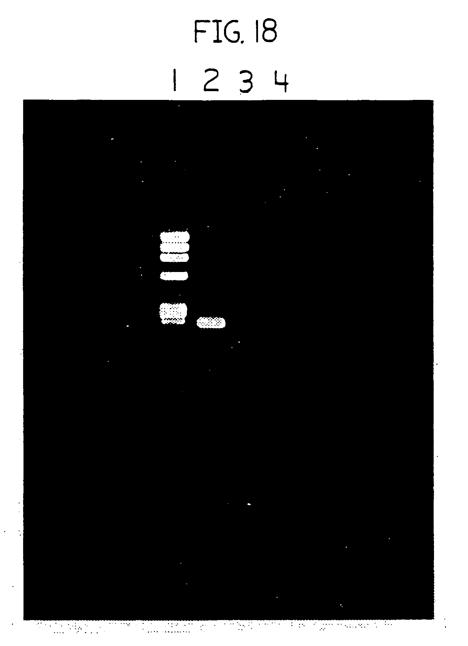

Figure 18 is an image of a gel depicting RT-PCR assays demonstrating low levels of mRNA encoding FasL and no detectable mRNA encoding Fas in hMSCs. Primers were reported by Kitagawa et al. (1998, Luekemia 12:486-492) were used and the PCR was for 30 cycles). Lane 1: Markers. Lane 2: Control of β-actin. Lane 3: Assay for FasL. Lane 4: Assay for Fas.

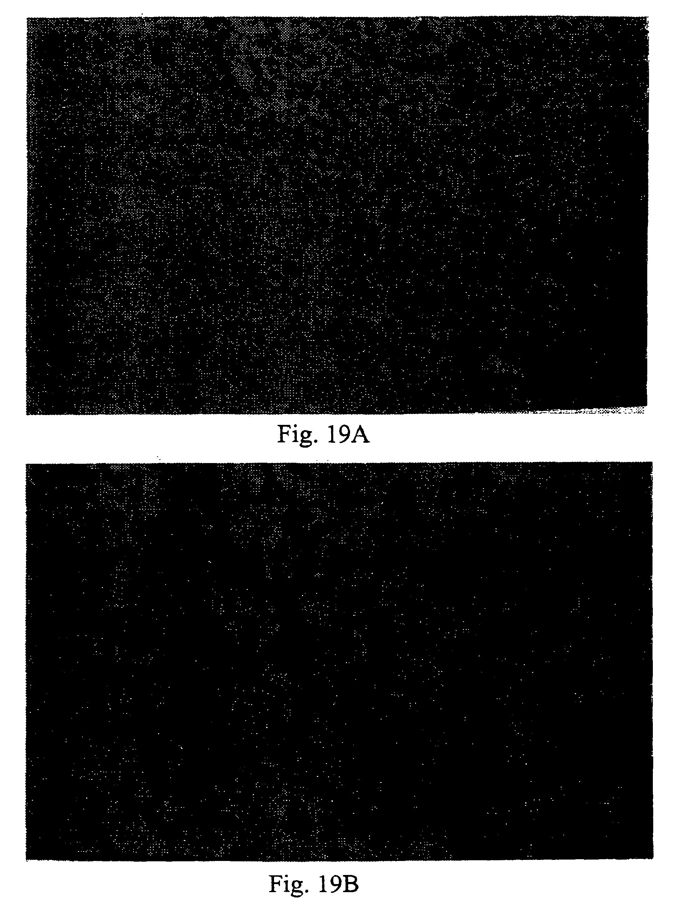

Figures 19A and 19B are a pair of images depicting successful implantation of C6-glioma cells in the rat brain. The brain sections were stained with H and E. Figure 19A depicts at low power the border between the implanted tumor and normal cerebral cortical tissue (CTX). Figure 19B depicts glioma cells with bizarre nuclei (shown by the arrow), at high power (40 x).

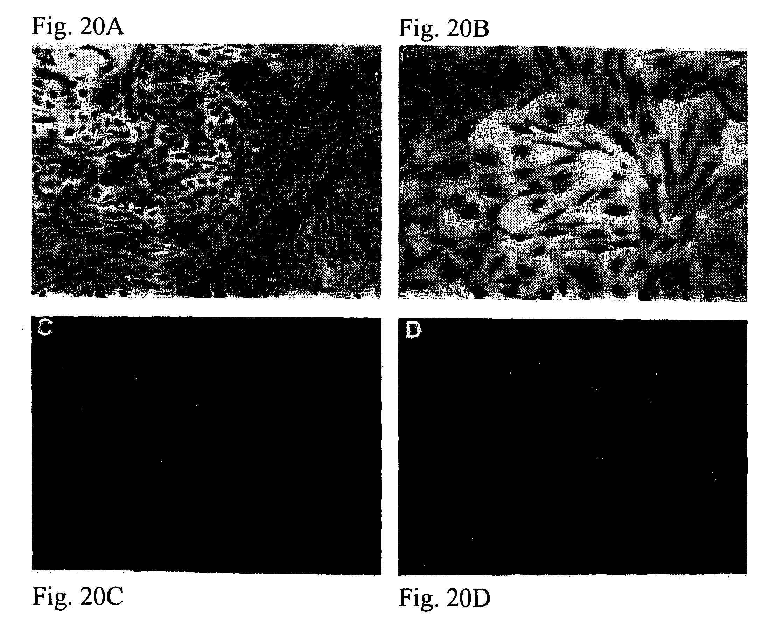

Figures 20A-20D are a quartet of images depicting mouse MSC (mMSC) cultures before and after immunodepletion. Figures 20A and 20C depict seven day old mMSC cultures before immuno-depletion stained with Giemsa or with an FITC-conjugated anti CD11b antibody and counterstained with DAPI, respectively. Figures 20B and 20D depict seven day old mMSC cultures after immuno-depletion stained with Giemsa or with an FITC-conjugated anti CD11b antibody and counterstained with DAPI, respectively.

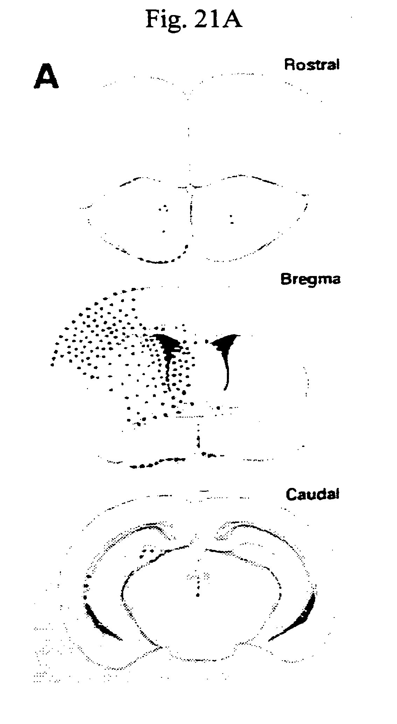

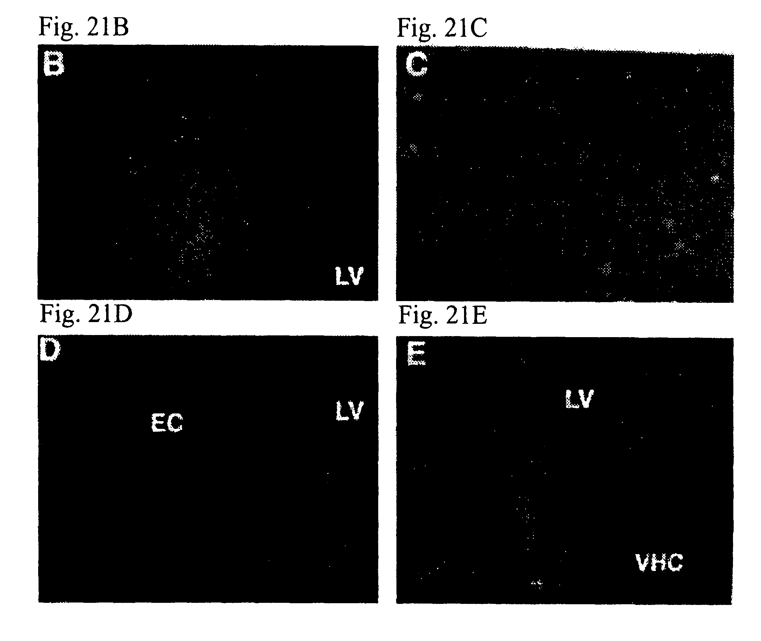

Figures 21A-21E are schematics and images depicting the time course of mouse MSC engraftment into neonatal mouse brains. Figure 21A is a schematic depicting the dissemination of *bis*-benzamide labeled MSCs at 3 days (yellow), 12 days (red), and 45 days (black) after injection. Regions in the brain where the distribution of donor cells was non-overlapping are indicated by the dots for each time point. Figure 21B is an image depicting MSCs adjacent to the lateral ventricle at approximately +0.9 mm to the bregma at 12 days after injection. Magnification was x 100. Figure 21C is an image depicting the same as in Figure 21 B at a higher magnification of x 400. Figure 21 D is an image depicting MSCs evident throughout the striatum and the external capsule at -0.2 mm to the bregma at 45 days after transplantation of the cells. Magnification was at x 100. Figure 21E is an image depicting donor cells lining the ventral hippocampal commissure and choroid plexus at a magnification of x 100. (LV=lateral ventricle; EC=external capsule; VHC= ventral hippocampal commissure).

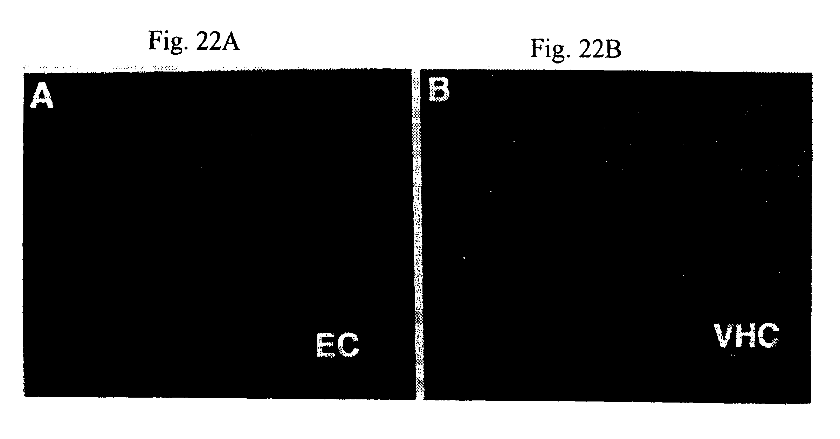

Figures 22A and 22B are a pair of images depicting the orientation of MSCs along established white matter tracts at 45 days after injection. Figure 22A depicts *bis*-benzamide labeled MSCs oriented in parallel, radial arrangements within the external capsule (EC). Figure 22B depicts *bis*-benzamide labeled MSCs oriented in parallel, radial arrangements within the ventral hippocampal commissure (VHC). Figures 22A and 22B are at a magnification of x 400.

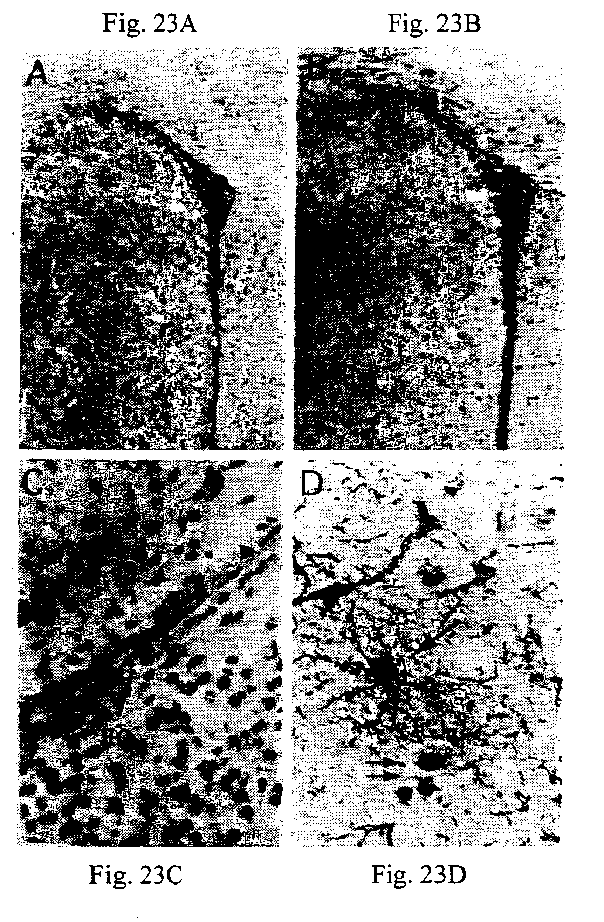

Figures 23A-23D are a quartet of images depicting the immunohistochemical localization of BrdU-labeled MSCs in forebrain at 12 days after injection. Figure 23A depicts Hemylotoxin and Eosin stained and Figure 23B depicts anti-BrdU stained serial sections of striatum and lateral ventricle, ipsilateral to the injection site, at approximately +0.9 bregma. The magnification is x 40. Figure 23C is an image of a photomicrograph of the sections in Figure 23B at the higher magnification of x 400, depicting BrdU labeled donor cells lining the external capsule. Figure 23D depicts an MSC derived astrocyte (indicated by the arrow) double labeled with antibodies against glial fibrillary acidic protein (GFAP) at x 1000 magnification in the hippocampal molecular layer at approximately -2.4 bregma. Donor cells which have not differentiated into astrocytes are indicated by the double arrows.

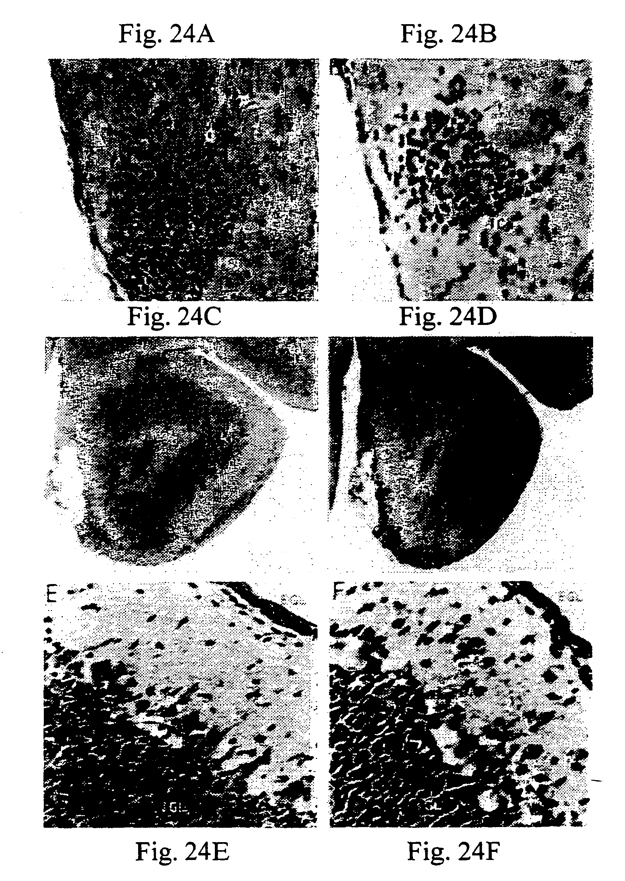

Figures 24A-24F are a series of images depicting 8-bromo-2-deoxyuridine (BrdU) labeled MSCs in neural rich regions of the forebrain and cerebellum. Hemylotoxin and Eosin (H&E) staining, depicted in Figure 24A, or anti-BrdU staining, depicted in Figure 24B, of serial sections indicates donor derived cells in the Islands of Calleja (ICj) in the ventral forebrain cortex. H&E staining, depicted in Figure 24C, or anti-BrdU staining, depicted in Figure 24D, of serial sections indicates donor derived cells in the subependyma of the olfactory bulb (OB). H&E staining, depicted in Figure 24E, or anti-BrdU staining, depicted in Figure 24F, of serial sections indicates donor derived cells in the external and internal granular layers (EGL, IGL) of the cerebellum. Magnification was at x 400.

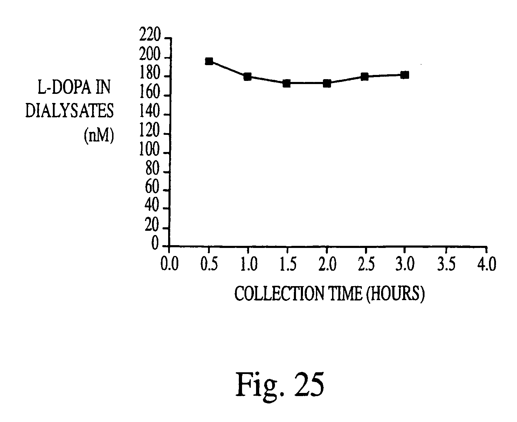

Figure 25 is an graph depicting the levels of L-DOPA produced in the brain of a rat treated with genetically engineered rat MSCs which secrete L-DOPA. Microdialysis was carried out in striatum of a Sprague Dawley 6-hydroxydopamine lesioned rat 3 days after engraftment of genetically engineered rat MSCs (TH+GC+). Approximately 100,000 cells were engrafted. Dialysates were taken every 30 minutes for 3 hours. Prior to engraftment, the rat MSCs were transduced with one retrovirus containing the gene for tyrosine hydroxylase (TH) and then a second retrovirus with the gene for GTP-cyclohydrase (GC). The GC gene provides tetrahydropteridine, an essential cofactor for tyrosine hydroxylase. No L-DOPA was detected in control rats.

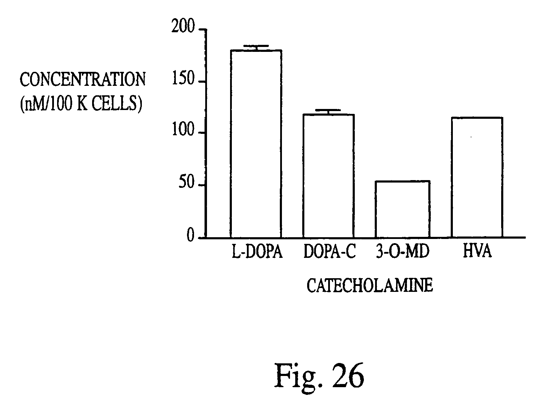

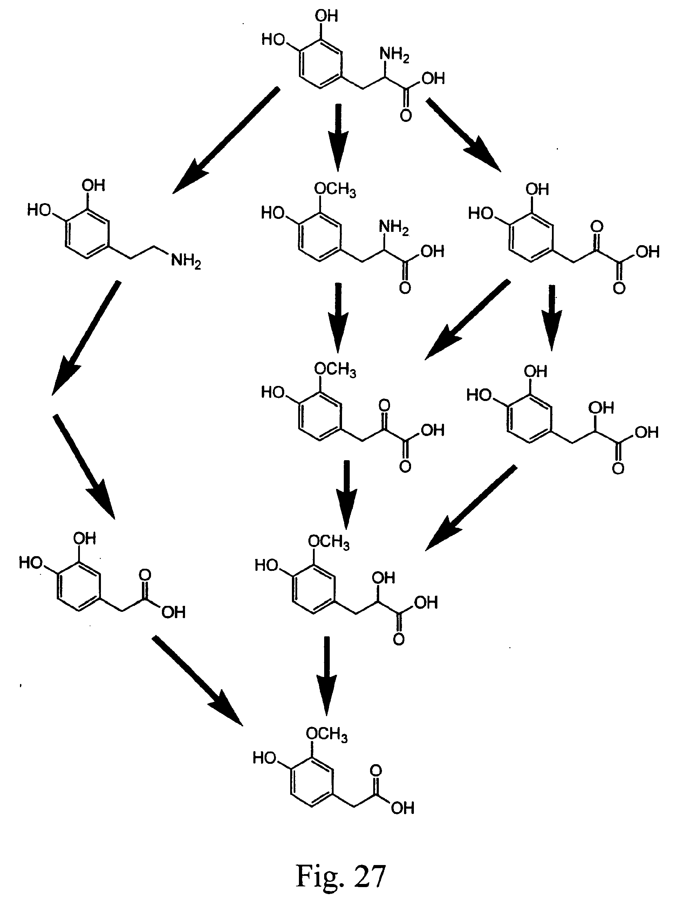

Figure 26 is a graph depicting the levels of L-DOPA detected by HPLC in samples from the microdialysis experiments depicted in Figure 25. As indicated in Figure 27, 3-O-methyl DOPA (3-O-MD) and homovanillic acid (HVA) can arise from an altered pathway of DOPA metabolism. 3,4-dihydroxyphenylacetic acid (DOPA-C) is a metabolite of dopamine.
Figure 27 is a schematic illustration depicting the metabolites of L-DOPA.

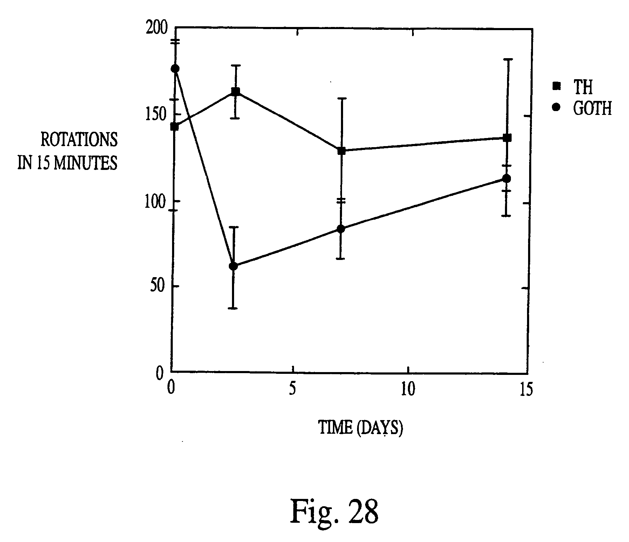

Figure 28 is a graph depicting the reduction of apomorphine-induced rotations in a rat model for parkinsonism. The rats were prepared by infusion of 6-hydroxydopamine into the corpus striatum to produce the parkinsonism model. Rotation was induced by injection of apomorphine (0.05 mg/kg, sc). Values depicted are from 2 to 5 rats infused with rMSCs transduced to synthesize L-DOPA (+GCTH) and 3 control rats infused with rMSCs transduced only with the gene for tyrosine hydroxylase (TH), rendering the cells unable to produce L-DOPA in culture without added tetrahydropteridine. The graph indicates that the effect on rotation disappeared after about 7 days, the same time at which L-DOPA was no longer detected by the microdialysis depicted in Figure 25.

### DETAILED DESCRIPTION OF THE INVENTION

The invention includes compositions and methods for treating patients affected by a disease, disorder, or condition of the central nervous system. The composition is isolated marrow cells which may or may not be genetically altered using recombinant DNA technology. The method comprises the steps of obtaining a bone marrow sample from a donor, isolating stromal cells from the bone marrow sample and administering the isolated stromal cells directly into the central nervous system of the patient.

### Definitions

The articles "a" and "an" are used herein to refer to one or to more than one (i.e. to at least one) of the grammatical object of the article. By way of example, "an element" means one element or more than one element.

As used herein, "central nervous system" should be construed to include brain and/or the spinal cord of a mammal. The term may also include the eye and optic nerve in some instances.

As used herein, "stromal cells", "colony forming fibroblasts", "marrow stromal cells", "adherent cells" and "MSCs" are used interchangeably and are meant to refer to the small fraction of cells in bone marrow which can serve as stem cell like precursors of osteocytes, chondrocytes, and adipocytes and which can be isolated from bone marrow by their ability adhere to plastic dishes. Stromal cells may be derived from any animal. In some embodiments, stromal cells are derived from primates, preferably humans.

As used herein, the term "adherent cells" is meant to refer to stromal cells.

The term "non-adherent cells" as used herein, is meant to refer to hematopoietic precursor cells.

As used herein, the term "disease, disorder or condition of the central nervous system" is meant to refer to a disease, disorder or a condition which is caused by a genetic mutation in a gene that is expressed by cells of the central nervous system such that one of the effects of such a mutation is manifested by abnormal structure and/or function of the central nervous system, such as, for example, neurodegenerative disease or primary tumor formation. Such genetic defects may be the result of a mutated, non-functional or under-expressed gene in a cell of the central nervous system. The term should also be construed to encompass other pathologies in the central nervous system which are not the result of a genetic defect per se in cells of the central nervous system, but rather are the result of infiltration of the central nervous system by cells which do not originate in the central nervous system, for example, metastatic tumor formation in the central nervous system. The term should also be construed to include trauma to the central nervous system induced by direct injury to the tissues of the central nervous system. The term should additionally be construed to include behavioral diseases, such as, but not limited to drug and alcohol addiction, depression, schitzophrenia, diseases which involve seratonin uptake or opiate receptors, metabolism of drugs, and the like. The term should also be construed to include stroke and emboli.

As used herein, "a disease, disorder or condition characterized by a gene defect" is meant to refer to a disease, disorder and condition in which a defective gene and/or insufficient gene expression is causally linked to the disease, disorder or condition. Individual who have any of several well known diseases, disorders and conditions characterized by a gene defect can be identified by those having ordinary skill in the art.

As used herein, "a disease, disorder or condition characterized by a defect in a gene which encodes a secreted protein" is meant to refer to a disease, disorder or condition characterized by a gene defect in which the gene that is defective or insufficiently expressed encodes a protein that is abnormally secreted from the cell.

By the term "abnormal secretion" is meant that the protein is processed in the cell in a manner which differs from the normal, i.e., wild type, non-defective protein. For example, the normal protein may be secreted from the cell in a particular structural configuration; abnormal secretion of this protein may occur when the protein is secreted in a different configuration. Further by way of example, when the normal protein is normally secreted from the cell, abnormal secretion of the protein may occur if the protein is not secreted from the cell, or is secreted from the cell at a level which differs markedly, i.e., is higher or lower, from the normal level of secretion of the normal protein.

As used herein, "a disease, disorder or condition which can be treated with a beneficial protein" is meant to refer to a disease, disorder or condition that can be treated or prevented by the presence of a protein which alleviates, reduces, prevents or causes to be alleviated, reduced or prevented, the causes and/or symptoms that characterize the disease, disorder or condition. Diseases, disorders and conditions which can be treated with a beneficial protein include diseases, disorders and conditions characterized by a gene defect as well as those which are not characterized by a gene defect but which nonetheless can be treated or prevented by the presence of a protein which alleviates, reduces, prevents or causes to be alleviated, reduced or prevented, the causes and/or symptoms that characterize the disease, disorder or condition.

As used herein, "beneficial protein" and "heterologous protein" are interchangeable and are meant to refer to a protein which can compensate for the protein encoded by a defective gene and/or insufficient gene expression that is causally linked to the disease or symptoms of the disease, disorder or condition characterized by a gene defect. The presence of the protein alleviates, reduces, prevents or causes to be alleviated, reduced or prevented, the causes and/or symptoms that characterize the disease, disorder or condition.

As used herein, "injury to the tissues or cells of the central nervous system caused by a tumor," is meant to refer to a disease, disorder or a condition of the central nervous system, wherein new growth of tissue occurs, usually in the brain and includes multiplication of cells which is uncontrollable and progressive. The cells which multiply may originate from, or may not originate from the central nervous tissue.

As used herein, "immunologically isolated", "immunologically protected", "immunologically neutralized", and "a manner that physically isolates cells from the recipient's immune system" are meant to refer to the encapsulation, containment or other physical separation of an implanted cell from the body into which it is implanted such that the cell is not exposed to and-cannot be eliminated by the immune system of the body, such that cells which are immunologically isolated are administered in a manner that physically isolates them from the recipient's immune system. Examples of immunological isolation methods include, but are not limited to, well known technologies and devices such as microencapsulation, biocompatible matrices, diffusion chambers, implantable cartridges, implant devices with membrane assemblies and other containers with membranes. It is preferred that cells are immunologically isolated by maintaining them in the body within an implant device.

The term "isolated nucleic acid" should be construed to refer to a nucleic acid sequence, or segment, or fragment which has been purified from the sequences which flank it in a naturally occurring state, e.g., a DNA fragment which has been removed from the sequences which are normally adjacent to the fragment e.g., the sequences adjacent to the fragment in a genome in which it naturally occurs. The term also applies to nucleic acids which have been substantially purified from other components which naturally accompany the nucleic acid, e.g., RNA or DNA or proteins which naturally accompany it in the cell.

As used herein, "an isolated nucleic acid molecule" may be one which either is not present in stromal cells or is not expressed as a protein in sufficiently high levels in stromal cells until it is introduced into the cell by means such as, but not limited to, classical transfection (calcium phosphate or DEAE dextran-mediated transfection), electroporation, microinjection, liposome-mediated transfer, chemical-mediated transfer, ligand mediated transfer or recombinant viral vector transfer.

As used herein, "gene construct" is meant to refer to an isolated nucleic acid molecule which includes coding sequences that encode a beneficial protein operably linked to a promoter/regulatory sequence having elements sufficient for expression of the coding sequence in stromal cells.

As used herein, "promoter/regulatory sequence" means a DNA sequence which is required for specific expression of a gene operably linked to the promoter/regulator sequence. In some instances, this sequence may be the core promoter sequence and in other instances, this sequence may also include an enhancer sequence and other regulatory elements which are required for expression of the gene in a tissue-specific or otherwise inducible or constitutive manner.

By describing two nucleic acid sequences as "operably linked" as used herein, is meant that a single-stranded or double-stranded nucleic acid moiety comprises each of the two nucleic acid sequences and that the two sequences are arranged within the nucleic acid moiety in such a manner that at least one of the two nucleic acid sequences is able to exert a physiological effect by which it is characterized upon the other.

As used herein, "heterologous gene" is meant to refer to the coding sequence of the gene construct.

A "heterologous protein" as used herein, is one which is encoded by a heterologous gene.

As used herein, the terms "recombinant genetic material" and "recombinant gene" are used interchangeably and meant to refer to genomic DNA, cDNA, synthetic DNA and RNA, mRNA and antisense DNA and RNA which is introduced into the stromal cell. The recombinant genetic material may be heterologous genetic material or may be an additional copy or copies of genetic material normally found in the individual or animal. When cells are used as a component of a pharmaceutical composition in a method for treating a human disease, disorder or condition, the recombinant genetic material that is used to transform the cells may encode a protein selected as a therapeutic used to treat the individual and/or to render the cells more amenable to transplantation.

As used herein, "transfected stromal cells" is meant to refer to stromal cells to which a gene construct has been provided using any technology used to introduce nucleic acid molecules into cells such as, but not limited to, classical transfection (calcium phosphate or DEAE dextran mediated transfection), electroporation, microinjection, liposome-mediated transfer, chemical-mediated transfer, ligand mediated transfer or recombinant viral vector transfer.

As used herein, the term "pre-differentiated" should be construed to mean isolated stromal cells which are cocultured with a substantially homogeneous population of differentiated cells, or products of differentiated cells, or inducers of cell differentiation, such that the isolated stromal cells differentiate and acquire phenotypic characteristics of the differentiated cells.

As used herein, the term "phenotypic characteristics" should be construed to mean at least one of the following characteristics: morphological appearance, the expression of a specific protein, a staining pattern, and the ability to be stained with a substance.

The phrase "substantially homogeneous population of differentiated cells" as used herein should be construed to mean a population of cells wherein at least 75% of the cells exhibit the same differentiated phenotype.

The phrase "directing differentiation" as used herein, should be construed to mean the induction of a differentiated phenotype in an undifferentiated cell by coculturing the undifferentiated cell in the presence of a substantially homogeneous population of differentiated cells, in the presence of products of differentiated cells or in the presence of an inducer of cell differentiation..

As used herein, the term "glioma-toxic protein" should be construed to mean a protein which is capable of arresting the growth of, and/or killing glioma cells/

### Description

The present invention is based on the discovery that MSCs which are isolated by adhesion to plastic and are infused into mammal brain, engraft, migrate, and differentiate into cells of the central nervous system, while other cells remain as precursor cells or throw off daughter cells that differentiate into cells of the central nervous system. This discovery allows for the successful treatment of an individual, i.e., a mammal and preferably, a human patient, suffering from a disease, disorder or a condition associated with the central nervous system, by either providing the individual with stromal cells obtained from a normal, matched syngeneic donor, or by isolating stromal cells from the individual, culturing the cells and genetically modifying them to correct whatever genetic defect is responsible for the disease, disorder or condition associated with the defect in the central nervous system.

In a preferred embodiment of the invention, stromal cells obtained from a matched donor or the same individual, are administered to an individual suffering from a disease, disorder or condition involving the central nervous system, in order to augment or replace the diseased and damaged nervous cells. Stromal cells are preferably administered to a human. Stromal cells are further preferably administered to the brain of the human. In some instances, the cells are administered to the corpus striatum portion of the human brain. The precise site of administration of the stromal cells will depend on any number of factors, including but not limited to, the site of the lesion to be treated, the type of disease being treated, the age of the human and the severity of the disease, and the like. Determination of the site of administration is well within the skill of the artisan versed in the administration of such cells.

The mode of administration of the stromal cells to the central nervous system of the human may vary depending on several factors including the type of disease being treated, the age of the human, whether the stromal cells are differentiated or not, whether the stromal calls have heterologous DNA introduced therein, and the like. An example of administration of stromal cells directly into brain tissue is provided herein in the experimental details section. In that example, cells are introduced into the brain of a mammal be first creating a hole in the cranium through which the cells are then passed into the brain tissue. Cells may be introduced by direct injection, by using a shunt, or by any other means used in the art for the introduction of compounds into the central nervous system.

There are several ways in which stromal cells may be used in a mammal, preferably, a human, to treat diseases of the central nervous system. For example, stromal cells may be used as precursor cells that differentiate following introduction the central nervous system or as cells which have been differentiated into neural cells prior to introduction into the central nervous system or simply as cells which do not differentiate and serve as vectors for gene products. In either situation, as the data presented herein establishes, the cells become permanent residents of the central nervous system. These cells may therefore replace cells in the central nervous system which have been lost as a result of a genetic disease, trauma, or other injury. In addition, prior to introduction into the central nervous system, stromal cells may be genetically engineered to produce molecules such as cytokines, neurotrophins, and the like, which will beneficially influence cells which are already present in the central nervous system. For example, genetically engineered stromal cells which are then introduced into the central nervous system may be used to repair any central nervous system damage, and/or to combat tumors of the central nervous system.

The data presented herein establish that human stromal cells which are cocultured with rat astrocytes become positive for glial fibrillary acidic protein, a marker for early astrocytes. In other words, it is possible to direct the differentiation of stromal cells by coculture of stromal cells with a desired cell type. Further, it is possible to convert human stromal cells into macroglial cells according tot eh data presented herein. Thus, it is possible, according to the data presented herein, to pre-differentiate isolated stromal cells to evolve into a desired phenotype prior to their introduction into the central nervous tissue. It is further possible that isolated stromal cells which are introduced into the central nervous system can differentiate in brain tissue or in spinal cord tissue into oligodendrocytes, Schwann cells, neurons and astrocytes.

Based on these considerations, the types of diseases which are treatable using isolated stromal cells which are introduced directly into the central nervous system are many. For example, among neonates and children, the cells may be used for treatment of a number of genetic diseases of the central nervous system, including, but not limited to, Tay-Sachs disease and the related Sandhoff's disease, Hurler's syndrome and related mucopolysaccharidoses and Krabbe's disease. To varying extents, these diseases also produce lesions in the spinal cord and peripheral nerves and they also have non-neurological effects. While the non-neurological effects of these diseases may be treatable by bone marrow transplantation, the central nervous system effects do not improve despite bone marrow transplantation. The method of the present invention may therefore be used to address the central nervous system effects of these types of diseases. In addition, in neonates and children, treatment of head trauma during birth or following birth is treatable by introducing stromal cells directly into the central nervous system of the children. Central nervous system tumor formation in children is also treatable using the methods of the present invention.

With respect to adult diseases of the central nervous system, isolated stromal cells are useful for treatment of Parkinson's disease, Alzheimer's disease, spinal cord injury, stroke, trauma, tumors, degenerative diseases of the spinal cord such as amyotropic lateral sclerosis, Huntington's disease and epilepsy. Treatment of multiple sclerosis may also be possible.

Treatment of spinal cord injuries is possible using the method of the present invention. Some prior art methods of treating spinal cord injuries involve using fibroblast cells to deliver of neurotrophins to the site of spinal cord lesions in animals. The neurotrophins delivered in this manner serve to reduce the lesion or otherwise treat the injury. However, unlike stromal cells, fibroblasts continue to produce large amounts of collagen which causes fibrosis at the site of the lesion, thereby negating the beneficial effects of the treatment. Thus, delivery of neurotrophins to spinal cord lesions using genetically engineered stromal cells has significant advantages over prior art methods because stromal cells do not produce large amounts of collagen and therefore will not cause fibrosis.

Although the data presented herein establish that the direct introduction of stromal cells into the central nervous system is useful for treatment of diseases of the central nervous system, in certain instances, isolated stromal cells may also be useful for treatment of diseases or injury associated with the eye. Stromal cells, when injected directly into the eye may differentiate into retinal pigmented epithelium, i.e., the cells that line the posterior surface of the retina and that appear to serve as nutrient cells for cones and rods in the eye. Alternatively, it may be possible to pre-differentiate isolated stromal cells into retinal pigmented epithelium by coculturing stromal cells with cells of the eye. That stem cells give rise to cells of the eye has been observed in fish. Fish eyes continue to grow throughout the life of the fish. In that instance, there are stem cells in the anterior edges of the eye that differentiate into both retinal pigmented epithelium and the light sensitive cells of the retina. Isolated stromal cells may be used to treat a variety of degenerative diseases of the eye, including degenerative diseases of the optic nerve retinal pigmented epithelial cells. The diseases include, but are not limited to, macular degeneration, a process of unknown origin which leads to a central visual loss in the elderly. Also included is blindness resulting from diabetes or arterial sclerosis, i.e., diseases of the eye which are the result of lesions in the vascular supply of the retina. In addition, rare diseases such as, for example, Laber's congenital amaurosis, may also be treatable using the methods of the invention.

In some aspects of the invention, an individual suffering from a disease, disorder, or a condition that affects the central nervous system and that is characterized by a genetic defect may be treated by supplementing, augmenting and/or replacing defective or deficient neurological cells with cells that correctly express a normal neurological cell gene. The cells which are to be introduced into the individual may be derived from stromal cells obtained from a normal matched donor or they may be stromal cells obtained from the individual to be treated. The cells may also be genetically modified to correct the defect. But this is not the only instance where the cells can be genetically modified.

In another embodiment of the invention, an individual suffering from a disease, disorder or a condition that affects the central nervous system and that is characterized by a genetic defect can be treated by supplementing, augmenting and/or replacing defective cells with cells that correctly express a normal gene. The cells may be derived from stromal cells obtained from a normal matched donor or stromal cells obtained from the individual to be treated. The cells may also be genetically modified to correct the defect.

An example of a disease, disorder or a condition that affects the central nervous system and that is characterized by a genetic defect is a brain tumor. An individual suffering from a brain tumor may be administered stromal cells obtained from a normal matched donor, which stromal cells differentiate into normal brain cells that may be used to replace or supplement the brain cells in the individual which has the tumor cells. The normal cells will compensate for the defective cells in the brain.

In an alternative embodiment, stromal cells are isolated from an individual suffering from a brain tumor and a gene capable of killing or otherwise arresting the replication of the tumor cells is inserted into the isolated stromal cells. The transfected cells are then reintroduced into the individual. The growth and/or replication of the tumor cells is arrested and/or apoptosis of the tumor cells is induced.

In one aspect of the invention, an individual suffering from a disease, disorder or a condition of the central nervous system can be treated as follows. Isolated stromal cells are obtained, they are expanded and are systemically administered to the individual. Some of the isolated/expanded stromal cells will develop into normal brain cells. Normal stromal cells expand more quickly than defective stromal cells and the expanded rejuvenated population will reflect a greater proportion of normal cells. Thus, repopulation of the central nervous system tissue with an expanded and rejuvenated population of stromal cells serves to provide a population of normal central nervous system cells which facilitate correction of the defect in the central nervous system tissue. Also, stromal cells may be pre-differentiated into, for example, astrocytes, by following the protocols provided herein, prior to administration of the stromal cells to the central nervous system.

In addition to replacing defective cells with repaired cells or normal cells from matched donors, the method of the invention may also be used to facilitate expression of a desired protein that when secreted in the central nervous system, has a beneficial effect. That is, stromal cells may be isolated, furnished with a gene encoding a desired protein and introduced into the central nervous system tissue of an individual. Expression of the desired protein in the central nervous system of the individual exerts a therapeutic effect in the individual. This aspect of the invention relates to gene therapy in which therapeutic proteins are administered to an individual.

According to some aspects of the present invention, immunologically isolated transfected stromal cells may be used as cell therapeutics to treat a disease, disorder or a condition characterized by a gene defect and/or a disease, disorder or a condition which can be treated using a recombinant protein in a gene therapy approach. In particular, a gene construct that comprises a heterologous gene which encodes a beneficial protein is introduced into stromal cells. The transfected stromal cells are then immunologically isolated and implanted into an individual who will benefit when the protein is expressed and secreted by the cell into the tissue of the central nervous system, preferably the brain.

Immunologically isolated stromal cells are particularly useful in cell therapeutic compositions, because in addition to being suitable hosts for expressing heterologous genes and producing heterologous proteins, stromal cells perform favorably when they are immunologically isolated. Immunologically isolated stromal cells have a very high viability when implanted in locations that lack a direct vascular blood supply. Moreover, stromal cells can be easily and readily obtained, they rapidly expand in culture making them a good source of an adequate supply of useful cells for immunologically isolated cell therapeutics.

According to the present invention, gene constructs which comprise nucleotide sequences that encode heterologous proteins are introduced into stromal cells. That is, the cells are genetically altered to introduce a gene whose expression has therapeutic effect on the individual. According to some aspects of the invention, stromal cells obtained from the same individual to be treated or from another individual, or from a non-human animal, may be genetically altered to replace a defective gene and/or to introduce a gene whose expression has therapeutic effect on the individual.

Further, according to the present invention, stromal cells are useful to prepare transfected cells that can be immunologically isolated and express heterologous beneficial proteins thereby providing a means to correct genetic defects and/or to produce therapeutic proteins in the individual. Stromal cells may be isolated with relative ease and isolated stromal cells may be cultured to increase the number of cells available. Stromal cells can be transfected, immunologically isolated and implanted with a high degree of viability into locations that lack direct blood supply such as subcutaneous locations.

In some embodiments, stromal cells may be immortalized, such as by using SV40 virus, a retrovirus, an adenovirus or other transforming virus, or by using proteins having transforming properties. In some aspects of the invention, an individual suffering from a disease, disorder or a condition may be treated by supplementing, augmenting and/or replacing defective or deficient genes by providing immunologically isolated stromal cells containing gene constructs that include normal, functioning copies of the deficient gene. This aspect of the invention relates to gene therapy in which the individual is provided with genes for which they are deficient in presence and/or function. The gene provided by the cell compensates for the defective gene of the individual, because, when the gene is expressed in the central nervous system tissue, a protein is produced-which serves to alleviate or otherwise treat the disease, disorder or condition in the individual. Such genes preferably encode proteins that are secreted.

The stromal cells are transfected and are administered to the central nervous system of the individual "as is" or in an immunologically isolated form. In some embodiments, stromal cells are transfected with genes for which the individual to be treated suffers from a complete absence of a non-mutated copy of the gene, or suffers from an absence or insufficient expression of a nonmutated form of the protein. Stromal cells are transfected with a non-mutated copy of the gene in an expressible form. That is, the protein encoded by the transfected gene will be expressed by the stromal cells, preferably as a secreted protein.

In addition to replacing defective genes with functional genes, the invention may also be used to express desired secreted proteins which exert a biologically active therapeutic or prophylactic effect. Such proteins are preferably secreted by the cells. That is, stromal cells may be isolated, furnished with a gene encoding a desired protein, they may then be administered to the individual as is, or in an immunologically isolated form, and the desired protein is expressed therein. According to this aspect of the invention, the isolated stromal cells serve as vectors for introducing therapeutic genes into the individual as well as hosts for such genes when the cells are administered to the individual.

In such embodiments, stromal cells are transfected with genes that encode proteins which have a therapeutic effect when expressed in the individual to be treated. Rather than administering the therapeutic protein directly to the individual which may require repeated administrations, the present invention provides a means of administering a therapeutic protein to the individual in a continuous manner by administering to the individual cells which produce the protein. Stromal cells are transfected with a gene that encodes the protein in an expressible form. That is, the protein encoded by the transfected gene will be expressed in the stromal cells, preferably as a secreted protein. Thus, the invention includes a method of treating a disease, disorder or condition of the central nervous system wherein stromal cells that are transfected with genes that encode a protein are administered to the individual. The protein is expressed and has a therapeutic effect. Examples of therapeutic proteins include, but are not limited to cytokines, chemokines, neurotrophins, and the like. In addition, it is contemplated that genes involved in behavioral disorders, such as, but not limited to disorders involving opiate receptors, serotonin uptake, and the like, including drug and alcohol addiction, genes involved in drug metabolism, genes involved in treatment of schitzophrenia, depression, may be used in the invnetion for delivery of their protein products for treatment of these diseases and disorders.

In all cases in which a gene construct is transfected into a stromal cell, the heterologous gene is operably linked to an appropriate promoter/regulatory sequence which is required to achieve expression of the gene in the stromal cell. Such promoter/regulatory sequences include but are not limited to, constitutive and inducible and/or tissue specific and differentiation specific promoters. Constitutive promoters include, but are not limited to, the cytomegalovirus immediate early promoter and the Rous sarcoma virus promoter. In addition, housekeeping promoters such as those which regulate expression of housekeeping genes may also be used. Other promoters include those which are preferentially expressed in cells of the central nervous system, such as, but not limited the promoter for the gene encoding glial fibrillary acidic protein. In addition, promoter/regulatory elements may be selected such that gene expression is inducible. For example, a tetracycline inducible promoter may be used (Freundlich et al., 1997, Meth. Enzymol. 283:159-173).

The gene construct is preferably provided as an expression vector which includes the coding sequence of a heterologous protein operably linked to essential promoter/regulatory sequences such that when the vector is transfected into the cell, the coding sequence is expressed by the cell. The coding sequence is operably linked to the pmmoter/regulatory elements necessary for expression of the sequence in the cells. The nucleotide sequence that encodes the protein may be cDNA, genomic DNA, synthesized DNA or a hybrid thereof or an RNA molecule such as mRNA.

The gene construct, which includes the nucleotide sequence encoding the beneficial protein operably linked to the promoter/regulatory elements, may remain present in the cell as a functioning episomal molecule or it may integrate into the chromosomal DNA of the cell. Genetic material may be introduced into cells where it remains as separate genetic material in the form of a plasmid. Alternatively, linear DNA which can integrate into a host cell chromosome may be introduced into the cell. When introducing DNA into the cell, reagents which promote DNA integration into chromosomes may be added. DNA sequences which are useful to promote integration may also be included in the DNA molecule. Alternatively, RNA may be introduced into the cell.

The elements in the promoter/regulatory sequences that are necessary for gene expression include: a promoter, an initiation codon, a stop codon, and a polyadenylation signal. It is necessary that these elements be operable in the stromal cells or in cells that arise from the stromal cells after infusion of the cells into an individual. Moreover, it is necessary that these elements be operably linked to the nucleotide sequence that encodes a protein such that the nucleotide sequence can be expressed in the stromal cells and thus the protein can be produced. Initiation codons and stop codon are generally considered to be part of a nucleotide sequence that encodes the protein. However, it is necessary that these elements are functional in the stromal cells or cells that arise from stromal cells. Similarly, the promoters and polyadenylation signals used must be functional within the stromal cells or cells that arise from stromal cells.

Examples of promoter/regulatory sequences useful to practice the present invention include but are not limited to promoter/regulatory sequences that are active in many cells such as the cytomegalovirus promoter, the SV40 promoter and many retroviral promoters. Other examples of promoter/regulatory sequences useful to practice the present invention include but are not limited to tissue-specific promober/regulatory sequences, i.e. promoter/regulatory sequences that function in some tissues but not in others; also, promoter/regulatory sequences of genes normally expressed in stromal cells with or without specific or general enhancer sequences. In some embodiments, promoter/regulatory sequences are used which constitutively express genes in stromal cells with or without enhancer sequences. Enhancer sequences are provided in some embodiments when appropriate or desirable.

Examples of polyadenylation signals useful to practice the present invention include but are not limited to the human collagen I polyadenylation signal, the human collagen II polyadenylation signal, and the SV40 polyadenylation signal.

In order for genetic material in an expression vector to be expressed, the promoter/regulatory elements must be operably linked to the nucleotide sequence that encodes the protein. In order to maximize protein production, promoter/regulatory sequences may be selected which are well suited for gene expression in the desired cells. Moreover, codons may be selected which are most efficiently transcribed in the cell. One having ordinary skill in the art can produce recombinant genetic material as expression vectors which are functional in the desired cells.

It is also contemplated that promoter/regulatory elements may be selected to facilitate tissue specific expression of the protein. Thus, for example, specific promoter/regulatory sequences may be provided such that the heterologous gene will only be expressed in the tissue where the immunologically isolated stromal cells are implanted. In addition, promoter/regulatory elements may be selected such that gene expression is inducible. For example, a tetracycline inducible promoter may be used (Freundlich et al., 1997, Meth. Enzymol. 283:159-173).

The heterologous protein preferably includes a signal sequence which directs the transport and secretion of the heterologous protein in the stromal cell. The signal sequence is generally processed and removed upon secretion of the mature protein from the cell.

In addition to providing cells with recombinant genetic material that either corrects a genetic defect in the cells, that encodes a protein which is otherwise not present in sufficient quantities and/or functional condition so that the genetic material corrects a genetic defect in the individual, and/or that encodes a protein which is useful as a therapeutic in the treatment or prevention of a particular disease, disorder or condition associated therewith, genetic material may also be introduced into the stromal cells used in the present invention to provide a means for selectively terminating such cells should such termination become desirable. Such means for targeting cells for destruction may be introduced into stromal cells which are to be otherwise genetically modified as well as those to which no other recombinant genetic material is to be introduced.

According to the invention, isolated stromal cells are furnished with genetic material which renders them specifically susceptible to destruction. For example, stromal cells may be provided with a gene that encodes a receptor that can be specifically targeted with a cytotoxic agent. An expressible form of a gene that can be used to induce selective cell death can be introduced into the cells. In such a system, cells expressing the protein encoded by the gene are susceptible to targeted killing under specific conditions or in, the presence or absence of specific agents. For example, an expressible form of a herpes virus thymidine kinase (herpes tk) gene can be introduced into the cells and used to induce selective cell death. When the introduced genetic material that includes the herpes tk gene is introduced into the individual, herpes tk will be produced. If it is desirable or necessary to kill the implanted cells, the drug gangcyclovir can be administered to the individual which will cause the selective killing of any cell producing herpes tk. Thus, a system can be provided which allows for the selective destruction of implanted cells.

Stromal cells may be obtained by removing bone marrow cells from a donor and placing the cells in a sterile container with a plastic surface or other appropriate surface that the cells come into contact with. The stromal cells will adhere to the plastic surface within 30 minutes to about 3 days. After at least 30 minutes, preferably about four hours, the non-adhered cells may be removed and discarded. The adhered cells are stromal cells which are initially non-dividing. However, after about 2-4 days, the cells begin to proliferate and can be cultured to increase their numbers using standard cell culture techniques.

According to preferred embodiments, stromal cells are cultured in medium supplemented with 2-20% fetal calf serum or serum-free medium with or without additional supplements. Isolated stromal cells may be transfected using well known techniques readily available to those having ordinary skill in the art. Foreign genes may be introduced into stromal cells using standard methods which are employed for introducing a gene construct into cells which express the protein encoded by the gene. In some embodiments, cells are transfected by calcium phosphate precipitation transfection, DEAE dextran transfection, electroporation, microinjection, liposome-mediated transfer, chemical-mediated transfer, ligand mediated transfer or recombinant viral vector transfer.

In some embodiments, recombinant adenovirus vectors are used to introduce DNA having a desired sequence into the stromal cell. In some embodiments, recombinant retrovirus vectors are used to introduce DNA having a desired sequence into the stromal cell. In some embodiments, standard calcium phosphate, DEAE dextran or lipid carrier mediated transfection techniques are employed to incorporate a desired DNA into dividing cells. Standard antibiotic resistance selection techniques can be used to identify and select transfected cells. In some embodiments, DNA is introduced directly into cells by microinjection. Similarly, well known electroporation or particle bombardment techniques can be used to introduce foreign DNA into isolated stromal cells. A second gene is usually co-transfected or linked to the therapeutic gene. The second gene is frequently a selectable antibiotic-resistance gene. Transfected cells can be selected by growing the cells in an antibiotic that kills cells that do not take up the selectable gene. In most cases where the two genes are unlinked and co-transfected, the cells that survive the antibiotic treatment contain and express both genes.

After isolating the stromal cells, the cells can be administered to the human upon isolation or following a period of in vitro culture. Isolated stromal cells may be administered upon isolation, or may be administered within about one hour after isolation. Generally, stromal cells may be administered immediately upon isolation in situations in which the donor is large and the recipient is an infant. It is preferred that stromal cells are cultured prior to administration. Isolated stromal cells can be cultured from 1 hour to up to over a year. In some preferred embodiments, the isolated stromal are cultured prior to administration for a period of time sufficient to allow them to convert from non-cycling to replicating cells. In some embodiments, the isolated stromal cells are cultured for 3-30 days, preferably, 5-14 days, more preferably, 7-10 days. In other embodiments, the isolated stromal cells are cultured for 4 weeks to a year, preferably, 6 weeks to 10 months, more preferably, 3-6 months.

In other embodiments, the isolated stromal cells are cocultured so that they differentiate into astrocytes or other neural cells prior to administration of the central nervous system.

If the cells are to be transfected, either isolated, non-cycling stromal cells are first transfected and then are administered as non-cycling cells; isolated, non-cycling stromal cells are first transfected, then cultured for a period of time sufficient to convert them from non-cycling to replicating cells and then are administered; isolated, non-cycling stromal cells are first cultured for a period of time sufficient to convert them from non-cycling to replicating cells, they are then transfected, and then are administered; or isolated, non-cycling stromal cells are first cultured for a period of time sufficient to convert them from non-cycling to replicating cells, they are then transfected, they are then cultured and then administered to the human. In some embodiments, stromal cells are isolated, transfected and immediately administered to the human.

It is preferred that stromal cells are cultured prior to transfection and/or administration. Isolated stromal cells can be cultured from cultured for 3-30 days, in some embodiments, 5-14 days, in other embodiments, 7-10 days prior to transfection. Transfected stromal cells can be cultured for 3-30 days, in some embodiments, 5-14 days, in some embodiments, 7-10 days prior to administration. Isolated stromal cells can be cultured from 3-30 days, in some embodiments, 5-14 days, in some embodiments, 7-10 days prior to transfection, and upon transfection, additionally cultured for 3-30 days, in some embodiments, 5-14 days, in some embodiments, 7-10 days prior to administration. In some embodiments, the isolated stromal cells are cultured for 4 weeks to a year, in some embodiments, 6 weeks to 10 months, in some embodiments, 3-6 months prior to transfection. Transfected stromal cells can be cultured for 4 weeks to a year, in some embodiments, 6 weeks to 10 months, in some embodiments, 3-6 months prior to administration. In some embodiments, the isolated stromal cells are cultured for 4 weeks to a year, in some embodiments, 6 weeks to 10 months, in some embodiments, 3-6 months prior to transfection and upon transfection, further cultured for 4 weeks to a year, in some embodiments, 6 weeks to 10 months, in some embodiments, 3-6 months prior to administration.

For administration of stromal cells to the human, the isolated stromal cells are removed from culture dishes, washed with saline, centrifuged to a pellet and resuspended in a glucose solution which is infused into the patient. In some embodiments, bone marrow ablation is undertaken prior to infusion in order to make space in the bone for introduced cells. The immune immune responses suppressed by agents such as cyclosporin must also be considered.. Bone marrow ablation may be accomplished by X-radiating the individual to be treated, administering drugs such as cyclophosphamide or by a combination of X-radiation and drug administration. In some embodiments, bone marrow ablation is produced by administration of radioisotopes known to kill metastatic bone cells such as, for example, radioactive strontium, ¹³⁵Samarium or ¹⁶⁶Holmium (see Applebaum et al., 1992, Blood 80(6):1608-1613).

If bone marrow ablation precedes administration of stromal cells, the administration of stromal cells must be accompanied by the administration of non-adherent cells which comprise blood cell precursors necessary for survival. Such non-adherent cells may be saved from the same sample used as starting materials in the isolation of stromal cells and stored or they can be derived from a different sample. In some preferred embodiments, the non-adherent cells are provided by the recipient/patient. Prior to procedures which generate bone marrow ablation, a sample of the patient/recipients bone marrow is obtained and stored. The entire sample may be used or the non-adherent cells may be isolated and used to administer in conjunction with isolated stromal cells. Non-adherent cells administered in conjunction with administration of stromal cells may be administered separately before or after stromal cell administration or may be mixed with isolated stromal cells prior to administration.

In some embodiments, isolated stromal cells are administered to the brain by direct infusion as described herein in the experimental examples section. In other embodiments, isolated stromal cells are administered to the central nervous system, i.e., the spinal cord, by simple injection, etc.

Between about 10⁵ and about 10¹³ cells per 100 kg person are administered per infusion. In some embodiments, between about 1.5 X 10⁶ and about 1.5 X 10¹² cells are infused intravenously per 100 kg person. In some embodiments, between about 1 X 10⁹ and about 5 X 10¹¹ cells are infused intravenously per 100 kg person. In some embodiments, between about 4 X 10⁹ and about 2 X 10¹¹ cells are infused per 100 kg person. In some embodiments, between about 5 X 10⁸ cells and about 1 X 10¹ cells are infused per 100 kg person.

In some embodiments, a single administration of cells is provided. In some embodiments, multiple administrations are provided. In some embodiments, multiple administrations are provided over the course of 3-7 consecutive days. In some embodiments, 3-7 administrations are provided over the course of 3-7 consecutive days. In some embodiments, 5 administrations are provided over the course of 5 consecutive days.

In some embodiments, a single administration of between about 10⁵ and about 10¹³ cells per 100 kg person is provided. In some embodiments, a single administration of between about 1.5 X 10⁸ and about 1.5 X 10¹² cells per 100 kg person is provided. In some embodiments, a single administration of between about 1 X 10⁹ and about 5 X 10¹¹ cells per 100 kg person is provided. In some embodiments, a single administration of about 5 X 10¹⁰ cells per 100 kg person is provided. In some embodiments, a single administration of 1 X 10¹⁰ cells per 100 kg person is provided.

In some embodiments, multiple administrations of between about 10⁵ and about 10¹³ cells per 100 kg person are provided. In some embodiments, multiple administrations of between about 1.5 X 10⁸ and about 1.5 X 10¹² cells per 100 kg person are provided. In some embodiments, multiple administrations of between about 1 X 10⁹ and about 5 X 10¹¹ cells per 100 kg person are provided over the course of 3-7 consecutive days. In some embodiments, multiple administrations of about 4 X 10⁹ cells per 100 kg person are provided over the course of 3-7 consecutive days. In some embodiments, multiple administrations of about 2 X 10¹¹ cells per 100 kg person are provided over the course of 3-7 consecutive days. In some embodiments, 5 administrations of about 3.5 X 10⁹ cells are provided over the course of 5 consecutive days. In some embodiments, 5 administrations of about 4 X 10⁹ cells are provided over the course of 5 consecutive days. In some embodiments, 5 administrations of about 1.3 X 10¹¹ cells are provided over the course of 5 consecutive days. In some embodiments, 5 administrations of about 2 X 10¹¹ cells are provided over the course of 5 consecutive days.

Stromal cells in diffusion chambers are described in Benayahu et al.; 1989, J. Cell Physiol. 140:1-7; Mardon et al., 1987, Cell Tissue Res. 250:157-165. After introducing the gene construct into the stromal cells, the cells can be immunologically isolated immediately or following a period of in vitro culture. Stromal cells can be implanted after they are immunologically isolated. Stromal cells may be immunologically isolated using any number of well known methods using readily available starting materials and/or devices. Stromal cells may be microencapsulated using many such microencapsulation protocols including those disclosed, for example, in U.S. Patent Number 4,391,909, U.S. Patent Number 4,806, 355, U.S. Patent Number 4, 942,129, and U.S. Patent Number 5,334,640.

Stromal cells may be administered to an individual in chambers using diffusible membranes or they may be encapsulated in microbeads. In another embodiment, the stromal cells are contained in hollow fibers such as those available from Amicon, Inc. (Beverly MA). These fibers are used for example to make cartridges for dialysis. One end can be pulled out from under the skin and reduced in size if dosages of the protein made by the cells are to be reduced. The surface area of the fibers is very high. Further, cells in the fiber can be flushed out and replaced periodically. Hollow fibers are described on pages 50-51 of Amicon, Inc. Publication No. 323.

Similarly, incorporation of transfected stromal cells in biocompatible matrices facilitates secretion of a beneficial protein to the individual while maintaining the cells in an immunologically isolated condition. Examples of biocompatible matrices are disclosed, for example, in U.S. Patent Number 4,902,295 and U.S. Patent Number 4,997,443. In some embodiments, transfected stromal cells are immunologically isolated by encasing them within tissue implant systems that are membrane assemblies. That is, cells are maintained in containers that include at least one porous membrane. The cells within the membrane assembly are immunologically isolated while beneficial proteins may be made available to the individuals by passing through the membrane. Implant devices which are membrane assemblies, include, but are not limited to, those described in U.S. Patent Number 5,314,471 and U.S. Patent-Number 5,344,454. According to one embodiment of the invention, an implant device is provided which comprises two ten ring assembles. Each ring assembly comprises a circular plastic ring and a 0.3 micron millipore membrane covering the area of the circle. Transfected stromal cells are disposed between the two ring assembly which are connected to each other at the circumference. The constructed implant device is preferably implanted subcutaneously.

In some preferred implant devices, about 10⁵ to about 10¹³ cells are provided. Preferred ranges of cells to be administered when immunologically isolated are as described herein when the cells are not immunologically isolated. Immunologically isolated cells may be implanted into the ventricles of the subdural space, or into the subarachnoid space of the brain and spinal column.

It is preferred that stromal cells are cultured prior to immunological isolation. Stromal cells can be cultured from 1 hour to over a year. In some preferred embodiments, the stromal cells are cultured for a period of time sufficient to allow them to convert from non-cycling to replicating cells. In some embodiments, the stromal cells are cultured for 3-30 days, preferably 5-14 days, more preferably 7-10 days. In some embodiments, the stromal cells are cultured for 4 weeks to a year, preferably 6 weeks to 10 months, more preferably 3-6 months. In preferred embodiments, cells are either isolated, non-cycling stromal cells that are first transfected and then immunologically isolated, then implanted as noncycling cells; isolated, non-cycling stromal cells that are first transfected, then cultured for a period of time sufficient to convert from non-cycling to replicating cells, then immunologically isolated and then implanted; isolated, non-cycling stromal cells that are first cultured for a period of time sufficient to convert from non-cycling to replicating cells, then transfected, then immunologically isolated and then implanted; or isolated, non-cycling stromal cells that are first cultured for a period of time sufficient to convert from non-cycling to replicating cells, then transfected, then cultured, then immunologically isolated and then implanted. In some embodiments, stromal cells are isolated, transfected, immunologically isolated and implanted. It is preferred that stromal cells are cultured prior to and after transfection, prior to immunological isolation. Isolated stromal cells can be cultured from 3-30 days, in some embodiments, 5-14 days, in some embodiments, 7-10 days prior to transfection. Transfected stromal cells can be cultured from 3-30 days, in some embodiments, 5-14 days, in some embodiments, 7-10 days prior to administration. Isolated stromal cells can be cultured from 3-30 days, in some embodiments, 5-14 days, in some embodiments, 7-10 days prior to transfection and upon transfection, additionally cultured for 3-30 days, in some embodiments, 5-14 days, in some embodiments, 7-10 days prior to administration. In some embodiments, the isolated stromal cells are cultured for 4 weeks to a year, in some embodiments, 6 weeks to 10 months, in some embodiments, 3-6 months prior to transfection. Transfected stromal cells can be cultured for 4 weeks to a year, in some embodiments, 6 weeks to 10 months, in some embodiments, 3-6, months prior to implantation.

The invention will be further described by reference to the following experimental examples. These examples are provided for the purposes of illustration only and are not intended to be limiting unless otherwise specified. Thus, the invention shall in no way be construed as being limited to the following examples, but rather, shall be construed to encompass any and all variations which become evident as a result of the teaching provided herein.

### Examples

### Example 1: Stromal Cells as Precursor Cells of Connective Tissues

Cells from a transgenic mouse line that expresses a human mini-gene for collagen I in a tissue-specific manner were used to determine whether precursor mesenchymal cells from marrow that are expanded in culture can serve as long-term precursors of bone and other connective tissues after intravenous infusion into irradiated mice. The marker gene consisted of an internally deleted mini-gene encoding the human proα1 (I) chain of procollagen I that causes synthesis of shortened proα1 (I) chains (Khillan et al., 1991, J. Biol. Chem. 266:23373-23379; Pereira et al., 1983, J. Clin. Invest. 91:709-716; and Sokolov et al., 1993, Biochemistry 32:9242-. 9249). Cells expressing the gene were obtained from a line of transgenic mice in which the copy number of the human mini-gene relative to the endogenous mouse gene was about 100 to 1, and the steady-state levels of mRNA expressed by the human mini-gene relative to mRNA expressed by the endogenous mouse gene was about 0.5:1 in most tissues.

Donor cells obtained from marrow partially enriched for mesenchymal precursors were prepared using standard protocols (Friedenstein et al., 1976, Exp. Hemat. 4:267-274; Castro-Malaspina et al., 1980, Blood 56:289-301; Piersma et al., 1985, Exp. Hematol 13:237-243; Simmons et al., 1991, Blood 78:55-62; Beresford et al., 1992, J. Cell. Sci.102:341-351; Liesveld et al., 1989, Blood 73:1794-1800; Liesveld et al., 1990, Exp. Hematot. 19:63-70; Bennett et al.,1991, J. Cell. Sci. 99:131-139). Briefly, the ends of long bones from the transgenic mice were cut, and the marrow was extracted with a pressurized syringe filled with α-MEM (Sigma Chemical Company, St. Louis, MO) containing 10% fetal bovine serum (Atlanta Biologicals). About 10⁷ nucleated cells were plated onto 175 cm² plastic culture flasks in 25 ml of α-MEM containing 10% fetal bovine serum. After 4 hours, the non-adherent cells were discarded by replacing the medium. Foci containing two to four fibroblast-like cells appeared in 2 to 3 days, and the foci grew to near-confluent colonies in about 1 week. The yield was about 10⁷ cells per flask after trypsin digestion. Most of the cells were fibroblast-like, but a few macrophages and adipocytes were also seen when the cells were examined under phase contrast microscopy.

About 10⁵ of the cultured adherent cells were mixed with 6 X 10⁵ non-adherent cells obtained by incubation of marrow from normal mice for 4 hours on 175 cm² flasks under the same conditions used for the initial isolation of the adherent cells. The mixture of about 7 X 10⁵ cells in 0.2 to 0.4 ml of α-MEM and 10% fetal bovine serum was injected into the tail vein of each recipient mouse.

Eight-week old mice from the same inbred FVB/N line were prepared to receive the donor cells by irradiation with a ¹³⁷Cu irradiator (Atomic Energy of Canada, Ltd.). The unit had a dose rate of 116 cG/min with a parallel opposed beam configuration. Each animal received 9.0 Gy in two fractions with a 4 hour interval (4.5 Gy + 4.5 Gy) (O'Hara et al., 1991, Exp. Hemat. 19:878-881). One to 2 hours after the second radiation fraction, the mixture of marked adherent cells and normal non-adherent cells was injected intravenously. Control irradiated mice that did not receive a cell infusion died after 10 to 13 days of marrow failure.

To follow the fate of the donor cells, two PCR assays for the human COL1A1 mini-gene and the mouse endogenous COL1A1 gene were developed. Using a two-primer assay, the values for the ratio of the human to mouse genes were linear over a range of 10⁻⁴ to about 10⁺¹ and, therefore, resulted in about 10⁻⁶ to 10⁻¹ donor cells per recipient cell. Using the three-primer assay, the values were linear over a range of about 10⁻³ to 10⁺² and, therefore, resulted in about 10⁻⁵ to 1 donor cell per recipient call.

Assays of irradiated mice after one day indicated only trace amounts of the donor cells in marrow, spleen, bone, lung or brain (Table 1). Slightly higher levels were seen at seven days. At 30 days and 150 days, progeny of the donor cells accounted for 2.0 to 12% of the cells in marrow, spleen, bone and lung (Table 1). At 150 days, they also accounted for 1.5 to 5.0% of the cells in xiphoid cartilage that was dissected free of any mineralized or fibrous tissue under a microscope. Although the mean values appeared to show a decrease between 1 and 5 months, there was no statistically significant decrease in the combined values for marrow, spleen, bone and lung between these two time periods (Table 1). Assays of non-irradiated mice revealed only very low levels of the donor cells at the same time points (< 0.0001 to 0.05%). PCR in situ assay of tissue sections of lung demonstrated that progeny of the donor cells were evenly distributed in the parenchyma of both alveoli and bronchi.

To confirm that progeny of the donor cells were present in cartilage, chondrocytes were isolated from xiphoid and articular cartilage by digestion at 37°C overnight with 0.5 mg/ml bacterial collagenase (Sigma Chemical Company, St. Louis, MO) in DMEM. PCR assays indicated that progeny of the donor cells accounted for 2.5% of the isolated chondrocytes.

To determine whether the donor cells became functional mesenchymal cells in the tissues they populated, tissues obtained the recipient mice were assayed by RT-PCR for expression of the human mini-gene for collagen I contained in the donor cells. In three mice assayed at 150 days, the mini-gene was expressed in bone, a tissue in which over half the protein synthesized in collagen I. The expression in bone was confirmed using a similar assay on bone cells isolated from femur and cultured for 1 week. Expression of the mini-gene for collagen I was more variable in marrow, spleen and lung, tissues in which the rate of collagen I synthesis is less than in bone. As expected, the mini-gene was not expressed in cartilage, a tissue in which about half the protein is synthesized in collagen II but in which there is no synthesis of collagen 1. The mini-gene for collagen I was also not expressed in cultures of chondrocytes obtained from the recipient mice that contained the marker gene and that synthesize collagen II but not collagen I.

The results presented herein demonstrate that after intravenous injection into irradiated mice, the expanded cultures of adherent cells efficiently populate several connective tissues. The results also demonstrate that the cells serve as true precursor cells for these tissues, since they expressed the marker gene for collagen I in a tissue-specific manner, and they were diffusely incorporated into the mesenchymal parenchyma of lung.

### Example 2: Conditions for Isolation and Culture of MSCs

Conditions for culture of MSCs so that they expand but retain the stem-cell-like phenotype were examined. Table I provides data which establishes that co-culture of MSCs with pieces of bone increased the number of cells obtained after 1 week. At the same time, co-culturing with bone decreased the alkaline phosphatase (APase) levels in the cells, an observation which suggests that the cells did not differentiate into osteoblasts. Also, there was a decrease in the levels of tartrate-resistant acid phosphatase (TRAP), an observation which suggests that the cells did not differentiate into osteoclasts. Similar effects were observed with secondary cultures of the MSCs. Therefore, the results suggest that co-culturing of MSCs with pieces of bone may provide improved conditions for expansion of MSCs. Also, the medium of cultured bone pieces may be an important source of cytokines and growth factors for expansion of MSCs in culture.

In related experiments, it has been found that secondary cultures of MSCs can be maintained for long periods of time. MSCs can be passed in culture for over 4 months by trypsinization and re-plating. The cells are remarkably stable in stationary phase cultures. In one experiment, stationary cultures remained viable for over 4 months with re-feeding about once per week. In another experiment, the cells remained viable when, through an oversight, they were left in an incubator without re-feeding for 1 month.

### Stable Transfection of MSCs with a Retrovirus Vector

To obtain virus for infection of MSCs, the LNCZ retroviral vector (Miller et al., 1989, BioTechniques 7:980-990) was modified so that the promoter for cytomegalovirus (pCMV) drove expression of the lacZ gene (Figure 1). The vector was stably transfected into an amphotropic murine packaging cell line (PA317). Constitutive virus producer clones were isolated by G418 selection, and supernatant obtained from the clones was used for infection of MSCs. Primary cultures of MSCs (3 days old) were infected for three successive days with fresh supernatant obtained from the producer line with the highest titer. Staining of the cells 5 days later indicated that about 15-20% of the cells typically expressed the lacZ gene. Several cultures of the infected cells were placed under selection with G418 (0.44 µg/ml active concentration) for 5 days. Most of the cells that recovered continued to express the lacZ gene. Modifications of LNCZ were also constructed so that expression of the lacZ gene was driven by the promoter of the COL Al gene-and the promoter of the COL2 Al gene (pCOL2A1). Expression of the lacZ gene was successfully obtained in primary cultures of MSCs using both constructs.zz

### Example 3: Lone-term Expression of Human Genes Using hGH. factor IX or Ob in Stably Transfected MSCs

MSCs are isolated from mice and cultured under the conditions described in Pereira et al., 1995, Proc. Natl. Acad. Sci: USA 92:4857-4861, which is incorporated herein by reference. MSCs are infected with retroviral vectors or transfected with naked DNA to obtain clones that express the genes for human growth hormone (hGH), the human obesity protein (Ob), or the gene for human factor IX. Because a lacZ gene has been successfully introduced into mouse MSCs with a retroviral vector, variants of the same vector are used. At the same time, MSCs are stably transfected with electroporation (Andreason et al., 1988, BioTechniques 6:650-660; Toneguzzo et al., 1986, Mol. Call. Biol. 6:703-706), lipofectamine and nuclear injection (Mercer et al., 1992, In: Antisense Strategies, Ann. IV. Y. Acad. Sci. Biol. 660:209-218) so that larger endogenous genes can be used. Further, some of the potential disadvantages of retroviruses are avoided using alternative introduction methodology.

Standard conditions for isolation and culture are: Whole marrow is obtained from the tibias and femurs of 6- to 10-week old FVB/N mice by cutting the ends of the bones and extruding the marrow with a syringe that contains 1 to 2 ml of ice-cold α-MEM and 10% fetal bovine serum (FBS). The pooled marrow cells are dispersed by gentle shaking and counted in an automatic counter (Coulter model ZM). From 5 X 10⁶ to 5 X 10⁷ nucleated cells in 25 ml of α-MEM and 10% FBS are plated onto 75-cm² culture flasks. After 4 hours or 3 days, the non-adherent cells are removed by replacing the medium. The adherent cells are expanded as primary cultures for 10 to 12 days with a refeeding about every 4 days. The cells are recovered by digestion with 0.25% trypsin and 1 to 5 mM EDTA for 5 minutes at 37°C followed by gentle scraping. The cells are diluted with α-MEM with 10% FBS and replated at a density of from 3 X 10⁴ to 1 X 10⁵ cells per 9.5 cm² in 6-well plates. Under these conditions, the doubling time of the cells is 19 to 22 hours. The secondary cultures are re-fed about every 4 days, and passed by trypsinization and replating under the same conditions.

### Preparation of Gene Constructs

The retrovirus vector LNCX is used as the parent construct. Convenient cloning sites in the construct are used to prepare the modified constructs pRSV-lacZ, pCMV-lacZ, pCOL1/lacZ and pCOL2-lacZ (Figure 1). The pCOL1 promoter is a 1.4 kb fragment that contains 476 bp of the promoter, the first exon and most of the first intron of the human COL1A1 gene. The promoter has been shown in transgenic mice to express a promoterless form of the COL2A1 gene in a highly tissue specific and developmental specific manner (Sokolov et al., 1995, J. Biol. Chem. 270:9622-9629). The COL2A1 promoter is a 1 kb fragment from the human COL2A1 gene (Ala-Kokko et al., 1991, J. Biol. Chem. 266:14175-14178) that confers tissue-specificity of expression (Bradham et al., 1994, J. Cell Physiol.158:61-68). The lacZ gene is replaced with the hGH gene (Nichols Laboratories); the OB gene (Considine et al., 1995, J. Clin. Invest. 95:2986-2988) or the human factor IX gene (Genetic Therapy, Inc.).

### Use of the Retrovirus Vector

### Retrovirus Producer Cell Lines

To establish producer cell lines, amphotrophic retrovirus packaging murine cells PA317 were used. The cells were transfected at 20% confluency in 100 mm dishes by the calcium phosphate precipitation procedure (Promega) using 15 µg of plasmid DNA that was linearized by digestion with ScaI that cuts in the pBR322 region of the retrovirus vector. One day post-transfection G418 (GIBCOBRL) was added to the medium at an active concentration of 1 mg/ml. Neomycin-resistant colonies appeared at 7 to 10 days of selection and were isolated by cloning with mechanical rings. The clones were expanded and individual clones were tested for the ability to express lacZ by direct staining of duplicate wells. The titer of the virus produced by the positive cells was assayed by single addition of 50 µl of medium to HT-1080 human tumor cells grown to 20% confluency in 6-well microliter plates with 3 ml medium per well and in the presence of 4 µg/ml of polybrene. The titer was assayed by determining the number of HT-1080 cells that stained positively for expression of the lacZ gene. Typically, the titer was 1 X 10⁵ to 1 X 10⁶.

### Retrovirus Infection of Mouse MSCs

Primary cultures of mouse MSCs were prepared as described above. After 3 days, the non-adherent marrow cells were discarded and fresh medium was added. The cells were then infected with the retrovirus in the presence of 4 µg/ml of polybrene by addition of 1/4 volume of fresh supernate medium from stably transfected producer cells that had the highest titer of virus production. The infection was repeated on two additional successive days. The cells were then either stained directly for lacZ expression or were divided into larger dishes and placed under selection with 0.4 mg/ml of G418 (active concentration). About 15 to 20% of primary cultures were positive for lacZ and most of the cells that survived G418 selection were positive for lacZ.

### Lipofectamine Transfection

Primary cultures of MSCs were grown for 10 days in α-MEM containing 10% FBS. After trypsinization and light scraping, the cells were seeded in a 6-well plate at a density of 10⁵ cells per well. The cells were grown for 2 days, then washed 2 times with PBS and incubated with a DNA-lipofectamine complex. The DNA-lipofectamine complex was prepared as follows: 6 µl of lipofectamine (GIBCO/BRL) were mixed with 1 µg of LNCZ DNA in 200 µl of α-MEM, incubated at room temperature for 30 min, and added to one well of a 6-well plate containing MSCs in 800 microliter α-MEM. After 6 hour incubation at 37°C, the DNA-lipofectamine complex was replaced with 2 ml of α-MEM containing 10% FBS. The cells were stained for lacZ or placed under G418 selection after 18 hour incubation in FBS-containing medium. Positive clones were obtained, but they grew slowly, apparently because the cell density was too low after the G418 selection. To circumvent this situation, three different strategies can be used: cells are plated at higher densities; co-culture cell culture inserts are placed over surviving clones early in the selection process and fresh MSCs or pieces of bone in the inserts are placed (see Table 1) on a daily basis to provide the necessary cell factors to stimulate growth; at the time that selection with G418 has killed many of the non-transfected cells, the cultures are reseeded with MSCs that have been infected with a variant of the retrovirus LNCX (Figure 1) in which the lacZ gene is replaced with a selectable gene for thymidine kinase. Therefore, the MSCs stably transfected with retrovirus are used to provide the necessary cytokines, growth factors, and cell interactions required for the initial growth of the transfected MSCs during selection in G418. The cells infected with the retrovirus may be then removed by negative selection with gangcyclovir.

### Delivery methods

### Nuclear Injections

Nuclear injections are highly efficient as a means of transfecting some cells. Cells were plated in 60-mm dishes containing a 22 x 22 mm coverslip marked with a circle to delineate the area for microinjection. Cells were incubated in medium containing 0.1% CS for 5 days to induce growth arrest before microinjection. Under these conditions between 8 and 15% of the cells incorporated [³H] thymidine during continuous labeling for 24 hours between days 5 and 6. Microinjection was performed using a Zeiss Axiovert inverted-microscope equipped with an Eppendorf microinjector and micromanipulator using commercially purchased glass-capillary femtotips (Eppendorf). All cells within a delineated area of the coverslip (usually 150-200) were microinjected into the nucleus with DNA at concentrations ranging from 0.01-10 µg/µl in 10 mM Tris buffer (pH 7.6). The injected cells are then expanded and assayed as described above.

### Electroporation

MSCs are treated with 0.25% trypsin and 1 to 5 mM EDTA for 5 minutes at room temperature and then harvested by scraping. The cells are pelletted by centrifugation at 4,000 x g for 10 minutes, and then are washed twice by resuspending the pellet in ice cold PBS (pH 7.4). MSCs are resuspended at 2 X 10⁶ cells per 0.8 ml and aliquoted into an electroporation cuvette (0.4 cm gap). The cells are incubated for 10 minutes on ice, DNA is added to the suspension (5 - 50 µg), and the cells are chilled for an additional 10 minutes. The cell suspension is then electroporated using a commercial instrument (BioRad Gene Pulser; model 1652076) at an empirically determined field strength which yields the greatest percentage of cells that retain the exogenously added DNA. To determine the appropriate field strength for MSCs, titrations have been performed ranging from 0.25 - 2.5 kv/cm. Electroporation efficiency was monitored by introducing a lacZ gene (LNCZ vector) and then staining cells 48 to 72 hours after electroporation.

### Assays

### hGH

Expression of the hGH gene is monitored by assaying medium from clones of cells grown in 6-well microliter plates with an enzyme linked immunoabsorbent assay with a commercially available kit (GIBCO/BRL). In this assay; 0:1 ml of 2 X diluent buffer is added per well of a microtiter plate. After 5 minutes, 0.1 ml of test sample is added and the plate is incubated at 37°C for 30 minutes. The wells are washed 5 times and 0.2 ml of primary antibody is added per well. The samples are incubated at 37°C for 30 minutes, and washed 5 times. Then 0.2 ml of substrate buffer containing 0-phenylenediamine substrate is added. Samples are incubated at room temperature for 30 minutes and the reaction is stopped by addition of 0.1 ml of 2 N sulfuric acid. The absorbance of the sample is assayed at 490 nm.

### Ob Protein

Cells are assayed for expression of the OB gene using a protein radioimmunoassay of cell medium. The primary antibody for human OB protein was raised in rabbits against recombinant protein synthesized in an *E. coli* expression system and purified to homogeneity. The human protein is highly homologous to the mouse and, therefore, anti-human antibodies should cross-react with the mouse protein. If they do not, the short mouse cDNA (619 nt) is expressed in *E. coli,* the protein is purified and antibodies are prepared. Alternatively, synthetic peptides having the mouse sequence are purchased and these are used to prepare antibodies. For the assay, recombinant human Ob protein was radiolabeled with ¹²⁵Iodine by the BoltonHunter method followed by gel filtration purification using Sephadex G-25. The specific activity obtained was 30 µCi/µg. Samples for assay (0.2 ml) were preincubated with primary antiserum (1:2000 dilution) in phosphate buffered saline containing 0.1% Triton X-100 for 16 hours at 4°C in a total volume of 0.4 ml. ¹²⁵I-Ob protein (30,000 cpm carried in 100 µl) was then added and the incubation was continued for an additional 24 hours. The bound Ob protein (12±1%; nonspecific binding 1.4±0.1%) was immunoprecipitated by the addition of 0.1 ml sheep anti-rabbit IgG serum (Antibodies, Inc., Davis, CA), 0.1 ml normal rabbit serum (GIBCO/BRL, Gaithersburg, MD), and 0.1 ml of 10% polyethylene glycol. The tubes were centrifuged for 15 minutes (2200 rpm), unbound label was decanted and the radioactivity in the pellet was counted in a Packard 5000 gamma counter (Downers Grove, IL). The concentration of Ob protein in unknown samples was calculated using Rodbard's unweighted four parametric logistic model. The limit of detection of this assay is 0.39 ng/ml. The intra-assay variance is 11.6% at 12 ng/ml with an interassay variance of 20.8% at 13.1 ng/ml.

### Human Factor IX

Expression of the gene encoding factor IX is assayed using a commercially available ELISA (American Bioproducts Company) under conditions similar to those used in the hGH assay (above). The standard curve ranged from 1-50 ng/ml⁻¹ and the limit of sensitivity was 1 ng/ml⁻¹. The assay did not cross-react with mouse factor IX.

### Example 4: Sustained expression of the three genes at physiologically important levels by systemic infusion of stably transfected MSCs into mice

Experiments presented herein have demonstrated that cultured MSCs can serve as stem-cell-like precursors of bone, cartilage and other mesenchymal tissues after systemic infusion. Therefore, MSCs expressing hGH, the Ob protein or factor IX are infused into irradiated and nonirradiated mice to evaluate sustained expression of the genes in vivo.

### Infusion of MSCs

Initially, MSCs are infused into mice under conditions described herein (3-week old mice: 300 or 700 Gray irradiation; intraperitoneal injection; 1 x 10⁶ MSCs; and 2 x 10⁸ whole marrow cells). In addition, intravenous infusion is compared to intraperitoneal; and lower levels of X-ray irradiation are employed. Also, the cells are infused into embryos by Cesarean section. In preliminary trials, 50 µl of 5 X 10⁴ ES were injected into the amnion of seven 13-day embryos; 6 of 7 were delivered as viable pups. Therefore, intrauterine injection of MSCs is feasible.

### Growth Curves

Effective in vivo expression of hGH should increase the growth rate of mice and expression of the Ob protein should induce starvation. Therefore, the weight and size of the treated mice and of control littermates are monitored.

### Assays for Gene Expression

Blood is obtained from the retro-orbital plexus of mice at 1 week, 1 month, 3 months, 5 months, 10 months, and 20 months after infusion of the MSCs. hGH and factor IX are assayed by ELISA, and the Ob protein is assayed with a radioimmune assays. In addition, if measurable increases in human factor IX are obtained with ELISA, the procedure described in Smith et al. (1993) Mature Genet. 5:397-402, to assay biologically active human Factor IX. In this procedure, human Factor IX was first captured in a microtiter well with the monoclonal antibody, BGIX1, and then activated by Factor XIa. The active Factor IX, in combination with Factor VIII, converted Factor X to Xa. Factor Xa cleaved the chromogenic substrate, S2765, yielding a yellow product. BGIX1-coated microliter plates and Factor VIII were purchased from Elcatech, Inc. (Winston-Salem, NC). Factor Xia was purchased from Enzyme Research Labs, Inc. (South Bend, IN).

Factor X, phospholipid solution, S-2765, and the thrombin inhibitor, 1-2581, were purchased from Kabi Pharmacia Hepar, Inc. (Franklin, OH). Four buffers were prepared: A, 50 mM Tris, 150 mM NaCl, 1% BSA, pH 7.5; B, 150 mM Tris, 5 mM CaCl₂, 10 mg/ml gelatin, pH 7.6; C, 50 mM Tris, 10 mM CaCl₂, pH 7.5; D, 50 mM Tris, 150 mM NaCl, pH 8.4. The Factor VIII/X reaction mix was prepared fresh by mixing equal quantities of the following stocks: Factor VIII, 5 U/ ml in buffer A; Factor X, 1 U/ml in buffers; 1-2581, 34 µg/ml in buffer A; CaCl₂, 25 mM in water; and phospholipid. Plasma samples were diluted in buffer A and 100 µl was added to each microtiter well. The plate was incubated for 90 minutes at room temperature and then washed five times with buffer B. 100 µl of Factor XIa (2 µg/ml in buffer C) was added to each well. After 30 minutes at 37°C, 100 µl of S2765 (0.5 mM in buffer D) was added to each well and the plate was incubated for 10 minutes at room temperature before the reaction was stopped by adding acetic acid to a final concentration of 10%. Absorbances at 405 nm were determined using a Bio-Rad microplate reader. The standard curve, prepared with dilutions of human normal pooled plasma, was linear from 3-25 ng/ml. The assay did not cross react with mouse Factor IX. Factor IX, levels of 250 ng/ml or 5% of normal are generally considered therapeutic and 100 to 150 ng/ml are considered beneficial.

### Example 5: Sustained expression of the genes at physiologically important levels by placing the MSCs in subcutaneous diffusion chambers

Cells implanted in subcutaneous diffusion chambers have at least two distinct advantages for use in therapy of human patients: Immune responses are circumvented; and when implanted in capsules in mice (Benayahu, 1989, J. Cell Physiol. 140:1-7), rats (Mardon et al., 1987, Cell Tissue Res. 250:157-165) or rabbits (Friedenstein et al., 1987, Cell Tissue Kinet. 20:263-272), they survive for at least 6 weeks (Wakitani, 1994, J. Bone and J.T. Surgery 76A:579-592), apparently because they persist as bone, fibrous tissue or cartilage that does not require vascularization (Benayahu et al., 1989, Supra; Mardon, et al., 1987, Supra; Owen et al., 1988, In: Cell and Molecular Biology of Invertebrate Hard Tissues, Wiley Chicester, CIBA Foundation Symposium, 136:42-60; Friedenstein et al.,1987, Supra).

### Preparation of Chambers

Diffusion chambers are assembled from commercially available components (Millipore Corp.) and used as described in previous reports (Benayahu et al., 1989, Supra; Mardon et al., 1987, Supra). Briefly, membrane filters with pore size are glued to one side of each of two plastic rings with acryloid glue. The two rings are then glued together to form a chamber, the dimensions are 9 mm inner diameter and 2 mm thick with a volume of about 127 mm³. From 10⁴ to 10⁷ MSCs are inoculated into the chambers through a hole in one ring and the hole is sealed with a tapered plastic plug coated with glue. The chambers are implanted into mice either subcutaneously on the back or intraperitoneally under anesthesia. Initially, one or more chambers are inserted into freshly weaned mice (3 weeks). Subsequently, chambers are inserted in 1 week old mice. For the experiments with the I week old mice, smaller chambers are prepared from discs (5 mm, inner diameter) cut from plastic tips for micropipettes.

### Assays

Blood is obtained from the retro-orbital plexus at 1 week, 1 month, 3 months, 5 months, 10 months and 20 months after implantation of the chambers. The plasma is assayed for hGH, Ob protein and factor IX as described above.

### Example 6: The Diffusion Chamber

Referring to Figure 2, the diffusion chamber (1) may have a chamber barrel (3) having two ends, a first end (5) and a second end (7). The barrel may be comprised of one or more rings secured together by non-toxic means. The chamber is fitted at each end with a filter, a first filter (9) and a second filter (11). The filters are porous to factors, for example, proteins, such that the factors may pass between the chamber and the mammal. The filter pores size may be about 0.25 µm or smaller, preferably about 0.1 µm. The filters may be made of plastic, teflon, polyester, or any inert material which is strong, flexible and able to withstand chemical treatments. The filters may be secured in position with rubber gaskets which may also provide a tighter seal. Optionally, the barrel portion of the chamber may have an opening (13) which may be covered by a cap (not shown). The cap may be a screw on type of self sealing rubber and fitted to the opening. Inserting cells into the chamber contents may thus be performed by accessing the opening by removing the cap and inserting cells using an ordinary needle and syringe. The chamber may be made of any substance, such as but not limited to, plastic, teflon, lucite, titanium, or any inert material, which is non-toxic to, and well tolerated by, mammals. In addition, the chambers should be able to survive sterilization.

The chamber may be implanted in the following nonlimiting ways: subcutaneously or intraperitoneally, for example. The chamber may be removed about 24 to about 30 hours after implantation. Alternatively, a refillable chamber may be employed such that the chamber may be re-used for treatments and emptied following treatments.

### Example 7: Administration of Isolated MSCs Directly into Rat Brains

### Preparation of Donor MSCs and Astrocytes

Human MSCs (Owen et al., 1988, in Cell and Molecular Biology of Vertebrate Hard Tissues, Ciba Foundation Symposium 136, Chichester, UK, pp. 42-60; Caplan, 1991, J. Orthop. Res. 9:641-650; Prockop, 1997, Science 276:71-74; Pereira et al., 1995, Proc. Natl. Acad. Sci. 92:4857-4861; Pereira et al., 1998, Proc. Natl. Acad. Sci. 95:1142-1147) were grown from aspirates taken from the iliac crest of normal male and female volunteers, aged 19 to 46 years old. Aspirates were diluted 1:1 with α-MEM/10% FBS and were centrifuged through a density gradient (Ficoll-Paque Plus; 1.077 g/ml, Pharmacia, LKB Biotechnology Inc., Piscataway, NJ) for 30 minutes at 1000 X g. After the supernatant and the interface were combined, the mixture was diluted to about 40 ml with α-MEM/10% FBS, and was again centrifuged. The nucleated cells, i.e., the MSCs, were suspended at a concentration of 1 X 10⁷/ml in α-MEM/10% FBS and the cells were plated at 3 X 10⁶/cm² in 25 cm² culture dishes. The MSCs were incubated for 3 days and the non-adherent cells were removed by replacing the medium in the culture. After the MSC cultures reached confluency, the MSCs were lifted from the plates by incubation with 0.25% trypsin and 1 mM EDTA at 37°C for 3 to 4 minutes. The MSCs were diluted 1:2 or 1:3 and the procedure for preparing the MSCs was repeated for 3 to 5 passages. Beginning with the second passage, 5 ng/ml of platelet derived growth factor (PDGF-AA; GIBCO/BRL, Grand Island, NY) was added to the medium.

For the isolation of rat MSCs, tibias and femurs obtained from 8-12 week old Lewis/SsNHsd female rats were dissected (Harlan Sprauge Dawley, IN). The ends of the bones were cut, and the marrow was extruded using 5 ml of DMEM (GIBCO/BRL, Grand Island, NY) using a needle and syringe. Between 100-200 X 10⁶ whole marrow cells were plated on a 175 cm² tissue culture flask in DMEM/10% FBS. After 24 hours, the non-adherent cells were removed by replacing the medium. The medium was replaced every 2 to 3 days as the cells were grown to confluency. The cells were lifted as before by incubation in the presence of 0.25% trypsin and 1 mM EDTA, following which the cells were passed three or four times and were then stored frozen until use.

Primary cultures of astrocytes (Azizi, 1996, Ann. Neurol. (suppl) 121;T236) were obtained from the brains of albino Sprague-Dawley adult rats (Harlan Sprague Dawley). After the heads of the mice were decapitated, the brains were removed under aseptic conditions and were floated in cold PBS over ice. The meninges and the brain stem were removed and discarded. The forebrain was mechanically dissociated into small pieces of tissue and the tissues were incubated at 37°C for 30 minutes in 2.4 units/ml of dispase (GIBCO/BRL, Grand Island, NY). At 10 minute intervals, the tissues were dissociated by flushing them through a large bore pipette. The dissociated tissues were centrifuged and the supernatant was discarded. The residue from each brain was suspended in α-MEM and was plated in two 175 cm² culture flasks, each in a medium of α-MEM/10% FBS. The non-adherent cells and the debris were removed by changing the medium after 48 hours, and then changing the medium every 4 days for about 2 weeks until the cultures were confluent. To remove loosely adherent cells, the confluent cultures were treated with 2.4 units/ml dispase for 15 minutes at 37°C, and the cultures were shaken on a rotary shaker at 120 rpm for 2 hours. The detached cells were discarded. Fresh medium was added to the adherent cells and the cultures were reincubated for 24 to 48 hours. Treatment with dispase to remove loosely adherent cells was repeated three times over a period of about 1 week.

For antibody staining, the cells were lifted from the plates by incubation with 0.25% trypsin for 1 to 3 minutes. The cells were then subcultured in chambered slides. To label the nuclei fluorescently, the MSCs and the astrocytes were incubated in the presence of 1 µg/ml bis-benzamide (Sigma Chemical Company, St. Louis, MO) for 24 hours prior to implantation. At one to two hours prior to implantation, the cultures were washed three times with sterile buffered saline and were lifted from the plates by incubation with 0.25% trypsin for 1 to 3 minutes. The trypsin was neutralized by adding medium containing 20% serum, and the cells were isolated by centrifugation. The cells were suspended as a slurry of about 10,000 cells/liter in α-MEM without serum.

### Neurotransplantation of Cells

Adult Sprague-Dawley,albino rats (weighing 200 to 300 grams each) were anesthetized in a sealed chamber using 3% halothane in oxygen. Anesthesia was maintained by intramuscular injection of a mixture of 6 mg/kg xylozine and 60 mg/kg ketamine. The animals were transferred to a sterotaxic apparatus in a clean field. A 2 to 5 mm incision was made in the scalp, 2 mm lateral to the bregma. A burr hole was made in the bone at a position 3 mm lateral to the bregma using a dental drill. About 10 µl of the cell suspension was slowly injected over 30 minutes into the striatum at a depth of 4 to 5 mm deep from the surface of the brain. The wound was closed with interrupted surgical sutures, and the animals were treated with 0.6 mg/kg of xylozine and 6 mg/kg of ketamine. At 5, 14, 30 and 72 day intervals, the rats were sacrificed by intracardiac perfusion under deep anesthesia using xylozine and ketamine. Perfusion was performed using ice-cold phosphate buffered saline, followed by 3% buffered paraformaldehyde, and then followed by 10% sucrose. The brains were removed, the forebrains were trimmed, and samples comprising sections of the brain were frozen immediately.

### Immunohistology of Implanted Cells.

Ten micron tissue sections were prepared using a cryostat. The transplant site in the brain was located by microscopically identifying the fluorescently labeled cells in the tissue sections. Frozen sections were attached to the gelatin coated slides and were quickly immersed in cold acetone for 5 minutes and stored at -20°C for further processing. The cells in the chambered slides were fixed with acetone for 5 minutes. Immunocytochemistry was performed at room temperature. The cells and the tissue sections were treated with blocking antibodies comprising 2% goat serum and 5% fetal bovine serum for 30 minutes. In experiments requiring the labeling of collagen and vimentin, the cells were further treated with Triton X-100 for 30 minutes and were then were rinsed in PBS. Primary antibodies were applied (Table 2) for 1 to 2 hours to cells in the chambered slides. These antibodies were applied for 24 hours in the case of tissue sections. The secondary antibodies used were species-specific IgGs coupled to either FITC or rhodamine.

Fluorescently labeled cells were visualized and photographed using a fluorescent microscope. The number of fluorescently labeled nuclei were counted in eight to ten tissue sections, cut from rostral to caudal limits of the striatum. This procedure was repeated on each brain by two individuals, who used alternate sections. Only the clearly labeled nuclei were counted. Dead and lysed cells left a bluish hue in the surrounding tissue and no clear nuclear staining. The observed numbers were extrapolated to the total number of sections in order to estimate the number of surviving engrafted cells.

### Donor MSCs and Astrocyte Precursors

MSCs obtained from human bone marrow were isolated by their adherence to plastic and were grown for 3 to 5 passages. As indicated in Figure 3, the addition of PDGF-AA increased the growth rate of the cells. Therefore, PDGF-AA was added to cells at passages 2 to 5 in order to obtain adequate numbers of human MSCs. However, rat MSCs grew adequately without the addition of PDGF-AA. Astrocytes were also isolated because of their tight adherence to plastic cultures dishes. Astrocytes were harvested after primary culture for about 3 weeks.

As indicated in Table 2, human MSCs stained heavily for fibronectin, collagen I and human HLA-ABC. Human MSCs stained faintly for vimentin and were negative for glial fibrillary acidic protein, whereas rat astrocytes were positive for both. Rat astrocytes stained poorly for fibronectin and were negative for collagen I and human HLA-ABC. Rat astrocytes were also negative for von Willebrand factor and galactocerebrosidase C.

It has been previously described that human MSCs become relatively homogeneous in appearance as these cells are passaged in culture (Bruder et al., 1997, J. Cell Biochem. 64:278-294). However, in the present study, two distinct populations were seen. These included a population of large flattened cells and a population of relatively elongated or spindle shaped cells (Figure 4, Panels A and B). In rat MSCs, two distinct populations of cells having similar morphologies were also seen (Figure 4, Panel C). The rat brain astrocytes were more dendritic in appearance (Figure 4, Panels E and F).

### Survival of Cells after Injection in the Striatum

The MSCs and the astrocytes were injected in the corpus striatum of rat brains using minimal perfusion pressure. Five to 72 days later, the rats were killed and sections of their brains were obtained. Examination of sections stained with hematoxylin and eosin indicated that there was no significant gliosis around the implantation site of either the rat astrocytes or the MSCs (Figure 5, Panel a). Fluorescently labeled cells were readily detected in the brain sections. Rat astrocytes, as previously shown in the prior art, readily engrafted (Andersson et al., 1993, Int. J. Dev. Neurosci.11:555-568; Azizi, 1996, Ann. Neurol. (suppl)121:T236; Zhou et al., 1992, J. Comp. Neurol. 317:145-155). Similar results were obtained using rat MSCs (Figure 5, Panel f) and human MSCs (Figures 5, Panels b-e). As indicated in Table 3, from about 20,000 to 42,000 cells were present in the recovered brains. Since the number of the cells injected varied from 100,000 to 120,000, about 20% of the infused human MSCs were recovered after 5 to 72 days. Moreover, there appeared to be a decrease in the number of the infused human MSCs recovered between days 30 and 72. In the case of rat MSCs, 33,000 cells or about 30% were recovered 14 days after infusion (Figure 5f).

### Migration of the Implanted Cells

It has been previously described that implanted rat astrocytes migrate through layers of the brain (Andersson et al., 1993, Int. J. Dev. Neurosci. 11:555-568; Zhou et al., 1992, J. Comp. Neurol. 317:145-155) in a manner similar to the migration seen with implanted neural stem cell or with a transformed line of the neural stem cells (McKay,1997, Science 276:66-71). In the present invention it has been discovered that MSCs migrated in a similar fashion. Donor cells were found in multiple areas of the brain, including the contralateral cortex (Figure 6). The cells persisted in the sites to which they migrated. The heaviest concentration of cells was found around the rostrocaudal axis in the striatum and along the corpus callosum. There were fewer cells in the cerebral cortex. Clusters of labeled cells were consistently observed in the temporal lobe regions at all time points examined. At day 72, fewer cells were found in the outlying cortical regions, an observation consistent with the apparent decrease in cell number between day 30 and 72 (Table 3).

### Immunohistology of Implanted Cells

Immunostaining of sections demonstrated that engrafted human MSCs were also detected throughout the brain (Figure 6) when antibodies to HLA-ABC were used (Figure 5, Panel b). Although human MSCs stained with antibodies to collagen I prior to implantation (Figure 7, Panel a), no staining with the same antibodies was seen after implantation. Thus, the MSCs ceased synthesis of type I collagen after integration into brain tissue. Staining of the cells with antibodies specific for fibronectin was observed prior to implantation (Figure 7, Panel b) and was also observed 5 days after implantation (Figure 7, Panel c).

The results of neurotransplantation experiments establish that human MSCs infused into rat brain can engraft, migrate, and survive in a manner similar to engraftment, migration and survival of rat astrocytes. Rat MSCs behaved in a similar manner to rat astrocytes. Thus, the rat and human cells behave in a similar manner. The data presented herein has established that at least a subset of cells that are isolated from the bone marrow by their adherence characteristics to plastic culture dishes, may also participate in the same pathway as astrocytes. The results establish that MSCs are therefore useful vehicles for both cell and gene therapy for treatment of a variety of diseases in humans. In addition, since the transplanted MSCs ceased synthesis of collagen type I, these cells are much more suitable for neurotransplantation than are fibroblasts which are known to continue to produce large amounts of collagen following transplantation and which therefore induce fibrosis at the site of transplantation.

MSCs have several advantages as cells for neurotransplantation. In particular, they can be readily obtained from bone marrow and expanded in culture. Also, a patient's own MSCs can be employed for the therapy, thus circumventing any attending problems of host immunity and graft versus host disease. In addition, the cells may be genetically engineered for treatment of diseases in human.

### Example 8: Coculture of MSCs with Astrocytes: Differentiation of MSCs into Astrocytes

Human MSCcs at a concentration of 2 X 10⁵ cells were cocultured with rat astrocytes at a concentration of 1 X 10⁵ cells in 10 ml of medium that consisted of MEM plus 30% FBS (Sigma Chemical Company, St. Louis, MO). The human cells were fluorescently labeled with α-human HLA-ABC prior to coculture. Following 5 days of coculture, about 2% of the human cells also stained positive for glial fibrillary acidic protein. Since glial fibrillary acidic protein is a marker for early astrocytes, these results demonstrate that a fraction of the human MSCs had differentiated into astrocytes during coculture with rat astrocytes.

**Table 1. Conditions for Growth of Primary and Secondary Cultures of MSCs**

| MSCs | Culture Conditions | Cells per Well X 105 | APase^{a} (mmol min/mg) | TRAP^{a} (mmol min/mg) |
|---|---|---|---|---|
| Primary | Standard^{a} | 2.0 | 42.6 | 144 |
| | Co-cultured^{b} | 6.41 | 22.3 | 102 |
| | Co-cultured^{c} (Matrigel) | 6.94 | 42.0 | 105 |
| Secondary^{d} | Standard | 1.33 | 2,052 | 72 |
| | Co-cultured | 7.40 | 362 | 60.6 |
| | Co-cultured (Matrigel) | 5.08 | 506 | 59.2 |

| | | | | |
|---|---|---|---|---|
| ^{a}Whole marrow cells (20 X 10⁶) from 6-week old mice were cultured in individual 9.5 cm² wells in a 2 ml of 10% FCS and α-MEM. Non-adherent cells were removed on day 3 and the incubation was continued in fresh medium until day 7. APase and TRAP were assayed. ^{b}Co-cultured with pieces of bone (one-half femur and one-half tibia) in cell culture inserts (23 mm; 3 µm pore size; Becton Dickinson). ^{c}same as^{b}, with inserts coated with Matrigel. ^{d}Primary cultures on day 10 were detached with 0.25% trypsin and 1 mM EDTA for 5 minutes at 37°C followed by gentle scraping. Cells from one well (2 X 10⁵) were diluted 1:4 and cultured in 9.5 cm² wells for 7 days with changes of medium on day 3 and day 6. ^{e}APase and TRAP activities were per mg total protein. | | | | |

**Table 2. Immunostaining of Human MSCs and Rat Astrocytes**

| Antibodies | Dilution | Human MSCs | Rat astrocytes |
|---|---|---|---|
| Fibronectin (Sigma) | 1:400 | + + + | - |
| Collagen I (Biodesign) | 1:10 | + + | - |
| HLA-ABC (Pharmingen) | 1:100 | ++ | - |
| Vimentin (Sigma) | 1:40 | +/- | + + + |
| Glial Fibrillary Acidic Protein (Sigma) | 1:20 | - | ++ |
| Galactocerebrosidase C (Sigma) | 1:50 | NA^{a} | - |
| von Willebrand Factor (Sigma) | 1:200 | NA | - |

| | | | |
|---|---|---|---|
| ^{a}NA, not assayed | | | |

**Table 3. Recovery of Fluorescently Labeled Human MSCs from Rat Brain**

| Days after infusion | Labeled cells per brain |
|---|---|
| 5 | 27,300^{a} |
| 14 | 30,200 |
| 30 | 41,800 |
| 72 | 19,900 |

| | |
|---|---|
| ^{a}Total counts on alternative sections by two different observers differed by less than 20%. | |

### Example 9: Implanting human MSCs into rat brain: Differentiation of MSCs into macroglial cells

Neuronal and glial cells in the central nervous system are derived from multipotential periventicular precursors. Marrow stromal cells are a subset of multipotential cells from bone marrow that can differentiate into osteoblasts, chondrocytes and myotubes, and may be the source of some of the glial cells or their precursors.

The data presented in this Example demonstrate that a fraction of human marrow stromal cells implanted into rat brains can express the astrocyte marker glial fibrillary acidic protein (GFAP). As described herein, the human marrow stromal cells (hMSCs) have been found to integrate and migrate in the rat brain in a manner similar to neural stem cells.

It has been axiomatic that in the early stages of development, neuronal and glial cells in the central nervous system are derived from multipotential precursor cells in the periventricular germinal zone (McKay, 1997, Science 276:66-71; Lundberg et al., 1997, Exp. Neurol. 145:342-360). Glia and neurons (Gould et al.,1998, Proc. Natl. Acad. Sci. (USA) 95:3168-3171; Kempermann et al., 1998, Nat Med 4:555-557) continue to proliferate and turn over. Bone marrow may be one source of generation of glia (Eglitis et al., 1997, Proc. Natl. Acad. Sci. (USA) 94:4080-4085).

The effects of direct implantation into rat brain of human marrow stromal cells (hMSCs) is described elsewhere herein. The hMSCs are a subset of multipotential cells in bone marrow that can be isolated by their adherence to plastic and can differentiate into osteoblasts, adipocytes, chondrocytes and myotubes (Owen, 1988, J. Cell Science (Supp)10:63-76; Caplan,1991, J. Orthopaedic Res. 9:641-650).

The objective of the experiments presented in this Example was to assess whether the hMSCs could be used as donor cells for grafting into the brain, and thus whether these cells could be used as vehicles for gene transfer into the CNS. A further objective was to determine whether a population of these cells, given the appropriate environmental and differentiation signals, could develop into brain cells. These experiments have implications for furthering the goals of regeneration and repair in the nervous system

Neurotransplantation has been used previously to achieve this goal and to study the ontogeny and lineage of brain cells (McKay, 1997, Science 276:66-71; Bjorklund, 1993, Research Publications - Association for Research in Nervous & Mental Disease 71:361-374; Olson, 1997, Nat Med 3:1329-1335; Bjorklund,1993, Nature 362:414-415). Both neural and non-neural cells have been used for grafting. For example, in Parkinson's disease, mesencephalic cells from human fetuses have been used as a source of dopaminergic tissue for treatment of humans (Bjorklund, 1993, Research Publications- Association for Research in Nervous & Mental Disease 71:361-374; Spencer et al., 1992, N. Engl. J. Med. 327:1541-1548; Freed et al., 1992, N. Engl. J. Med. 327:1549-1555; Kordower et al., 1997, J. Comp. Neurol. 387:96-113). A number of the patients exhibited significant improvement both in clinical symptoms and in objective measures such as increased synthesis of dopamine as assayed by fluorodopa uptake by positron-emission tomograpy (Spencer et al., 1992, N. Engl. J. Med. 327:1541-1548; Freed et al., 1992, N. Engl. J. Med. 327:1549-1555; Kordower et al., 1997, J. Comp. Neurol., 387:96-113; Defer et al., 1996, Brain 119:41-50). However, procuring the fetal tissue has presented major logistic and ethical problems (Rosenstein,1995, Exp. Neurol. 133:1-6; Turner et al., 1993, Neurosurg. 33:1031-1037) and therefore a series of attempts have been made to develop alternative cells for therapy of Parkinsonism and other neurologic diseases.

Most of the cells tested, however, have had difficulties associated with them. For example, they have been either as difficult to obtain as human fetal cells or they did not integrate and migrate after infusion to the brain (Kang et al., 1993, J. Neurosc. 13:5203-5211; Andersson et al., 1993, Int. J. Dev. Neurosc. 11:555-568; Sanberg et al., 1997, Nat. Med. 3:1129-1132; Isacson et al., 1997, Nat. Med. 3:964-969). In addition, many of the implanted cells do not survive in the host. For example, only about 0.1 % of the donor cells survived 7 months after mesencephalic neural cells from fetal pig were neurotransplanted into a patient with Parkinsonism (Isacson et al., 1997, Nat. Med. 3:964-969).

Experiments described elsewhere herein have revealed that after infusion into rat brains, hMSCs were found to migrate from the injection site for several millimeters. The experiments presented in the present Example expand upon that observation and demonstrate that a fraction of hMSCs can be recovered as cells expressing a marker for astrocytes (GFAP) after infusion into the brains of rats.

### Isolation of rat and human MSCs

Rat MSCs were isolated as previously described herein, by dissecting tibias and femurs from 8 to 12 week old Lewis/SsNHsd female rats (Harlan-Sprague-Dawly). The ends of the bones were cut and the marrow was extruded with 5 ml ofDMEM (GIBCO/BRL, Grand Island, NY) using a needle and syringe. Whole marrow cells (between 100-200 X 10⁶⁾ were plated on a ^{175 cm2} tissue culture flask in DMEM/10% FBS. Non-adherent cells were removed after 24 hours by replacing the medium. The medium was replaced every 2 to 3 days as the cells were grown to confluency. The cells were lifted by incubation with 0.25% trypsin and 1 mM EDTA, passed three or four times, and stored frozen.

Human MSCs were grown from aspirates taken from the iliac crest of normal male and female volunteers aged 19 to 46 years, as previously described herein. Nucleated cells were isolated by centrifugation through a density gradient (Ficoll-Plaque Plus; 1.077 g/ml; Pharmacia LKB Biotechnology, Inc., Piscataway, NJ), suspended at a concentration of 1 x 10⁷/ml in α-MEM/10% FBS, and plated at 3 x 10⁶/cm² in 25 cm² culture dishes. Non-adherent cells were removed after 3 days. The cells were lifted by incubation with 0.25% trypsin and 1 mM EDTA at 37°C for 3 to 4 minutes after the cultures reached confluency. The suspension of cells was diluted 1:2 or 1:3 and replated. This procedure was repeated for 3 to 5 passages. Beginning with the second passage, 5 ng/ml of platelet-derived growth factor αα (PDGF-AA; GIBCO/BRL, Grand Island, NY) was added to the medium.

### Antibody staining and fluorescent labeling

The cells were subcultured in chambered slides for subsequent antibody staining. Fluorescent labeling of nuclei was performed by incubating hMSCs with 1-10 µg/ml *bis*-benzamide (Sigma Chemical Co., St. Louis, MO) for 24 hours before implantation. The cultures were washed three times with sterile buffered saline and lifted by incubation with 0.25% trypsin for 1-3 minutes 1-2 hours before infusion into rat brains. Trypsin was neutralized by adding media containing 20% serum and the cells were isolated by centrifugation. The cells were suspended as a slurry of about 10,000 cells per µl in α-MEM without serum.

### Infusion of hMSCs into rat brain

Adult Sprague-Dawly albino rats (200 to 300 g) were anesthetized using 3% halothane in oxygen and maintained by intramuscular injection of a mixture of 6 mg/kg xylozine and 60 mg/kg ketamine. Rats were then placed in a sterotaxic apparatus, and a burr hole was made 3 mm lateral to the bregma. The hMSCs were administered by injecting about 10 µl of the cell suspension slowly over a 30 minute period into the striatum at a depth of 4 to 5 mm from the surface of the brain. The wound was closed with interrupted surgical sutures, and the animals were treated with 0.2 mg/kg of Buprenorphine and allowed to recover. The rats were sacrificed by intracardiac perfusion using ice-cold phosphate buffered saline, followed by 3% buffered paraformaldehyde, and then 10% sucrose while under deep anesthesia with xylozine and ketamine. The brains were removed, the forebrains were trimmed, and the samples were immediately frozen.

### Assessment of human cell (hMSC) survival in the rat brain

Brain tissue sections were prepared at a ten-micron thickness using a cryostat. The transplant site was located by microscopically identifying the fluorescently labeled cells in the tissue sections. Frozen sections were attached to gelatin coated slides and quickly immersed in cold acetone for 5 minutes and then stored at -20°C for further processing and counting. The number of fluorescently labeled nuclei was counted in 8 to 10 tissue sections cut from rostral to caudal limits of the striatum. This procedure was repeated on each brain by two investigators who used alternate sections. Only the clearly labeled nuclei were counted. Dead and lysed cells were characterized by a bluish hue in the surrounding tissue and no clear nuclear staining. The observed numbers were extrapolated to total number of sections in order to estimate the number of surviving engrafted cells.

### Preparation of Cell cultures

Rat brain cell cultures were prepared by mechanically dissociating the forebrain into small pieces and incubating the resulting tissue at 37°C for 15 minutes in a mixture of 2.4 units/ml of dispase and 45 units/ml of collagenase (GIBCO/BRL, Grand Island, NY). The tissue was dissociated at 5 minute intervals by flushing through a large bore pipette. The dissociated tissue was centrifuged, and the supernatant was discarded. The residue from each brain was suspended in α-MEM and plated in two 175 cm² culture flasks in α-MEM/10% FBS. Nonadherent cells and debris were removed by changing the medium after 48 hours, and then every 4 days for about 2 weeks until the cultures were confluent To remove loosely adherent cells, the confluent cultures were treated with 2.4 units/ml dispase for 15 min at 37°C, and shaken on a tilted rotary shaker at 6-7 rpm for 2 hours. The detached cells were discarded and fresh media was added to the adherent cells and the cultures were reincubated. The cells were then lifted using trypsin (0.5%) and were subcultured in chambered slides for counting and immunocytochemical staining. The total number of cells and the fraction of cells having fluorescently labeled nuclei were counted using a hemocytometer.

### Immunohistochemical staining

Cells in chambered slides were fixed with acetone for 5 minutes. Immunocytochemistry was performed at room temperature. Cells were treated with appropriate blocking antibodies of 1% goat serum and 1% fetal bovine serum for 30 minutes. Primary antibodies were applied for 1 to 2 hours in chambered slides. The secondary antibodies used were species-specific IgGs coupled to either FITC or rhodamine. The resulting fluorescently labeled cells were visualized and photographed using a fluorescent microscope.

### Results

Both rat and human MSCs were readily isolated from aspirates of whole bone marrow by their tight adherence to tissue culture plastic, as previously described herein (see also Caplan, 1991, J: Orthopaedic Res. 9:641-650). Figure 8 depicts rat MSCs cultured on poly L-lysine coated glass cover-slips. The cells stained heavily for Vimentin, moderately for Nestin (40-60% of cells) and slightly for GFAP (1% of cells), neural S100 protein, and p75 nerve growth factor receptor (p75 NGFr). Human MSCs which were treated under the same conditions were positive for Vimentin. However, examination of over fifteen samples of human MSCs (approx. x 10⁵ cells/sample) did not reveal any cells that stained with antibodies to GFAP.

The number of fluorescently labeled hMSCs in rat brains at selected days after implantation is shown in Table 4. Cultured hMSCs were fluorescently labeled with *bis*-benzamide, and about 100,000 cells in 10 µl of buffer were infused slowly into the striatum of adult Sprague-Dawly albino rats. After select time periods, ranging from 5 to 120 days, the rats were killed and the brains were removed. Consistent with the results described elsewhere herein, examination of brain sections did not reveal any evidence of gliosis or infiltration of leukocytes around the site of cell infusion. Table 4 contains the results following counting fluorescently labeled cells in sections of the brain. The data indicate that about 30% of the human cells were present after 5 to 30 days and about 15% were present after 120 days. As described herein, the labeled cells were seen along the needle track, but the cells also migrated into multiple areas of the brain, including the contralateral cortex and the temporal lobe regions.

In another series of experiments, rat brains were removed 7 to 90 days after infusion of about 100,000 hMSCs (Table 5), and the forebrains containing the injection sites were cultured in MEM and 20 % FBS. After the establishment of confluent primary cultures (Figure 9a), the cells were lifted with trypsin and counted using a hemocytometer and phase microscope (Figure 9b). Fluorescently labeled nuclei were counted using a hemocytometer and fluorescent microscope (Figure 9c), and the ratio of fluorescently labeled cells to unlabeled cells was determined. Subcultures were plated in chamber slides (20% FBS in DMEM) and the cells were fixed in cold acetone or 3% paraformaldehyde for indirect fluorescence immunocytochemistry. Sixty samples from 12 brains were examined. Human cells in the cultures were identified both by staining with HLA-ABC and by fluorescent labeling of nuclei.

The results of these experiments are presented in Table 5. These results indicate that 3 to 7% of the cells in the cultures were human cells. About 1% of the human cells in the cultures (about 0.1% of the total cells) stained with antibody to GFAP. This ratio remained relatively constant with time. The GFAP positive cells exhibited the morphology of epithelioid flat astrocytes (Figure 10). Human MSCs treated under these culture conditions did not stain with antibody to ED-1, a marker on the surface of microglia, and therefore were not microglia (Wu et al., 1997, Neurosc. Res. 28:67-75).

The data presented in this Example confirm and expand upon data described herein demonstrating that hMSCs can be stably implanted into the rat brain. The data in this Example establish that hMSCs survive in the brain environment and can be detected in large numbers up to at least 150 days after implantation. Previous studies have suggested that results from transplantation of tissues in the rat brain is a good predictor of the behavior and survival of these tissues in the human brain (Zawada et al., 1998, Nat. Med. 4:569-574). The fact that a large number of these cells were stably implanted without histological signs of immune reaction in the brain or behavioral deterioration of the recipient animal after implantation raises the possibility that hMSCs can safely be used for transplantation into the human brain.

The results also suggest that the culture conditions described herein either selected for the infused cells or caused them to proliferate disproportionately. This can be concluded because the number of human cells recovered in the astrocyte-enriched cultures (Table 5) was much larger than could be predicted from the relatively small number of hMSCs infused into the rat brains (rat brain contains over 2 x 10⁷ cells and only 10⁵ hMSCs were infused). The results of the experiments in this Example therefore provide evidence that astrocytes can arise from a small fraction of hMSCs that are implanted directly into the brains of rats. Thus, the micro-environment of the brain either selects for astrocyte lineages in MSCs or induces differentiation of MSCs into astrocytes.

The data presented in this Example demonstrate that a fraction of human marrow stromal cells implanted into rat brains can express GFAP. As described herein, the hMSCs integrated and migrated in rat brain, in a manner similar to neural stem cells. These data therefore also establish that bone marrow derived stromal cells have the capacity to differentiate into macroglial cells in the brain.

These features support other data provided herein that MSCs are useful for either direct cell therapy or as vectors for ex vivo gene therapy of a number of diseases of the central nervous system.

Previous reports indicated that peripheral blood macrophages can give rise to microglia of the central nervous system, particularly early in development or after injury (Ling et al., 1993, GLIA 7: 9-18; Bauer et al., 1996, Histoch. J. 28:83-97; Rinner et al.,1995, GLIA 14:257-266). It is likely that the culture methods used by these investigators excluded these cells and selected for astrocytes. Although a definite, albeit slow, turnover in the glial population of the adult mammalian brain can occur (Rakic, 1985, Science 227:1054-1056), astrocytes have been generally assumed to be endogenous to the adult brain.

### Example 10: Use of Marrow Stromal Cells to treat Gliomas

Malignant brain tumors are devastating diseases that affect about 15,000 individuals in the U.S. each year and that kill 90% of the patients in less than two years (Levine et al., 1989, In Cancer: principles and practice of oncology. Davita et al. (eds.) (Lippincoff, Philadelphia) pp. 1557-1611; Azizi, et al., 1998, J. Neurovirol. 4:204-216; Chung et al., 1998, Surg. Oncol. Clin. N. Am., 7:589-602; Liang et al., 1998, Curr. Opin. Oncol. 10:201-206). Therefore, a number of attempts are currently underway to develop improved therapies. As described herein, it has been shown that rat brains can be engrafted with a subset of cells from human bone marrow that are referred to as either mesenchymal stem cells or marrow stromal cells (hMSCs). After infusion into rat brains, the hMSCs integrated and migrated along known pathways for migration of neural stem cells. Human MSCs did not produce any immune or inflammatory reactions and about 15% of the cells were recovered in the brain after three months. Also, analysis of astrocyte-enriched cultures prepared from the brains of the recipient rats indicated that a small fraction of the engrafted hMSCs had differentiated into early astrocytes that expressed glial fibrillary acidic protein. Because hMSCs are relatively easy to isolate from marrow and to genetically engineer to express exogenous genes as described herein (see also Owen et al., 1988, In Cell and Molecular Biology of Vertebrate Hard Tissues, Ciba Foundation Symposium 136, Chichester, UK, pp. 42-60; Caplan, 1991, J. Orthop. Res., 9:641-650), they appear to be attractive vehicles with which to deliver therapeutic gene products to the brain.

This Example contains a description of experiments performed in cell culture and in rats to test the possibility that hMSCs can be used to express tumor-toxic proteins and then be injected into gliomas so as to provide a potential therapy for these diseases. Essentially the same experimental strategies that other investigators have successfully used to genetically engineer cells to secrete tumor-toxic proteins have been used herein. Such protocols are well known to the skilled artisan. The principal novelty of these experiments is that they employed hMSCs as producer cells that could be engrafted into brain and, therefore, serve as a long-term source of tumor-toxic proteins that are delivered locally to a glioma.

In this Example, hMSCs are genetically engineered to express and secrete one of three tumor-toxic proteins (Figure 11): (a) The Fas ligand (FasL) that has recently been demonstrated to selectively kill some-glioblastoma cells (Weller et al., 1994, J. Clin. Invest., 94:954-964; Weller et al., 1995, Cancer Res., 55:2936-2944; Frei et al., 1998, J. Neuro Immunol., 87:105-113; Weller et al., 1998, Brain Pathol. 8:285-293; Nagata, 1998, Intern. Med, 37:179-181 ; Suda et al., 1993, Cell, 75:1169-1178; Li et al.,1998, Transpl. Proc., 30:943; Tanaka et al.,1997, J. Immunol., 158:2303-2309); (b) A bispecific single chain antibody to both epidermal growth factor receptor (EGFR) and to CD3. The design is based on the principle that the anti-EGFR antibody will target the secreted protein to glioma cells and the anti-CD3 antibody on the other end of the same molecule will attract activated T lymphocytes to kill the glioma cells. Similar bispecific single chain antibodies are currently being tested for a large number of cancers (Kufer et al., 1997, Cancer Immunol. Immunother. 45:193-197; De Jonge et al., 1997, Cancer Immunol. Immunother. 45:162-165; Bookman, 1998, Semin. Oncol. 25:381-396; Helfrich et al. 1998, Int. J. Cancer, 76:232-239; Jost et al., 1996, Molec. Immunol. 33:211-219; Wickham et al., 1997, J. Virol. 71:7663-7669); and (c) a mouse single-chain antibody to epidermal growth factor receptors (EGFR) that are abundant on malignant glioma cells obtained from about 60% of patients and that can potentially be used together With ¹²⁵I-labeled antibodies to the mouse single chain antibody to kill glioma cells (Snelling et al., 1995, Hybridoma 14:111-114; Faillot et al., 1996, Neurosurg. 39:478-483; Stragliotto et al., 1996, Eur. J. Cancer, 32A:636-640). The protocol of these experiments is modeled on a similar protocol currently used in clinical trials using ¹²⁵I-labeled monoclonal antibodies to an EGFR (Snelling et al., 1995, Hybridoma 14:111-114; Faillot t al.,1996, Neurosurg. 39:478-483; Stragliotto et al., 1996, Eur. J. Cancer, 32A:636-640).

### Conventional Therapy for Malignant Gliomas

The current therapy for malignant gliomas largely consists of surgical resection followed by radiation therapy (Levine et al., 1989, In Cancer: principles and practice of oncology. Davita et al. (eds.) (Lippincott, Philadelphia) pp. 1557-1611; Azizi et al., 1998, J. Neurovirol. 4:204-216; Chung et al., 1998, Surg. Oncol. Clin. N. Am. 7:589-602; Liang et al., 1998, Curr. Opin. Oncol., 10:201-206). At times, these therapies are combined with chemotherapy for high grade gliomas. The treatment protocols can prolong the survival of patients by a few months, but the rate of recurrence of the malignant gilomas is greater than 90% and the post-surgical quality of life is poor. The results have improved somewhat with recent technical improvements in radiotherapy such as hyper-fractionation, altering the quality of the radiation, stereotaxic treatment to spare normal tissues, and addition of radio sensitizing substances. A large number of chemotherapeutic agents have been tested but the results have not been impressive (Levine et al., 1989, In Cancer: principles and practice of oncology. Davita et al. (eds.) (Lippincott, Philadelphia) pp. 1557-1611; Azizi, et al. 1998, J. Neurovirol. 4:204-216; Chung et al.,1998, Surg. Oncol. Clin. N. Am. 7:589-602; Liang et al., 1998, Curr. Opin. Oncol. 10:201-206).

### Fas and FasL for Therapy of Gliomas

Fas (CD95/APO-1) and FasL (CD95L) have recently attracted considerable attention as molecular targets and reagents for the therapy of malignancies (Trauth et al., 1989, Science 245:301-305; Nagata et al., 1995, Science 267:1449-1456). Fas is a member of a growing family of cytokine receptors that include receptors for TNF-α and NGF. FasL is a cytotoxic cytokine homologous to TNF and induces apoptotic cell death in susceptible target cells (Nagata et al., 1995, Science 267:1449-1456). The FasL/Fas system has been implicated in T cell-mediated cytotoxicity, the regulation of T cell homeostasis, and thymocyte selection during development. Membrane bound FasL can exert its cytotoxicity by cell to cell contacts, but significant cytotoxicity is also found in the supernatant of COS cells transfected with FasL cDNA and secreting soluble FasL (Rensing-Ehl et al., 1995, Eur. J. Immunol. 25:2253-2258). Antibodies to Fas induce apoptosis in cells bearing the FasL receptor. A single injection of the antibodies into nu/nu mice produced rapid regression of the human B cell tumor (Trauth et al., 1989, Science 245:301-305). However, administration of antibodies to Fas to wildtype mice was lethal because of apoptosis of the cells in the liver (Ogasawara et al., 1993, Nature 364:806-809).

Several recent reports have suggested exploitation of the Fas/FasL system for the therapy of malignant gliomas (Weller et al., 1994, J. Clin. Invest. 94:954-964; Weller et al., Cancer Res. 55:2936-2944; Frei 1998, J. Neuroimmunol. 87:105-113; Weller et al., 1998, Brain Pathol. 8:285-293). The critical findings were that Fas is not expressed in normal brain tissue (Weller et al.,1998, Brain Pathol., 8:285-293; Gratas et al., 1997, Brain Pathol. 7:863-869). The only exception is endothelial cells but these resist apoptosis induced by Fas antibodies in vitro ( Weller et al., 1994, J. Clin. Invest. 94:954-964). In contrast, both Fas and FasL are expressed in malignant gliomas (Weller, M., et al.,1998, Brain Pathol., 8:285-293). For reasons that are not apparent, the presence of both Fas and FasL in the same glioma cell does not produce apoptosis. However, Fas mRNA is detected in all primary glioblastomas, and about one-fifth of secondary glioblastomas. Systematic analysis of grade I to IV astrocytomas indicated that the levels of mRNA for Fas correlate with malignant progression of the tumors (Weller et al., 1998, Brain Pathol. 8:285-293). Assays for the Fas protein indicated that expression is observed primarily in glioma cells surrounding large areas of necrosis.

Based on these observations, antibodies to Fas and soluble FasL have been tested with gliomas in culture (Weller et al., 1994, J. Clin. Invest. 94:954-964; Weller et al., 1995, Cancer Res. 55:2936-2944; Frei et al., 1998, J. Neuroimmunol. 87:105-113; Weller et al., 1998, Brain Pathol. 8:285-293). Both produced apoptosis, but FasL was more effective than antibodies to Fas. FasL induced apoptosis in all of 19 tumor cell lines analyzed (Frei et al., 1998, J. Neuroimmunol. 87:105-113). Also, the long-term effect of FasL on tumor colony formation was more striking in that there was more than 90% inhibition of colony tumor growth with all of 8 high grade gliomas analyzed (Frei et al., 1998, J. Neuroimmunol. 87:105-113). On the basis of their results, the authors suggested that intrathecal or intratumor application of FasL was a promising approach for the treatment of Fas-expressing malignant tumor cells (Frei et al., 1998, J. Neuroimmunol. 87:105-113).

### Bispecific scFv-anti-EGFR and scFv-anti-CD3 for Therapy of Gliomas

Bispecific single chain antibodies (scFv) are now being explored for the therapy of a number of malignancies such as ovarian cancer (Bookman, 1998, Semin. Oncol. 25:381-396) or colorectal cancer (Kufer et al., 1997, Cancer Immunol. Immunother. 45:193-197). For example, a bispecific scFv to both CD3 and the transferrin receptor redirected activated mouse cytotoxic T-lymphocytes to specifically lyse human cells containing the transferrin receptor (Jost et al., 1996, Molec. Immunol. 33:211-219). The bifunctional reagent was effective at concentrations of a few ng/ml (Jost et al., 1996, Molec. Immunol. 33:211-219). Similarly, a bispecific scFv for both CD3 and an epithelial cell antigen has been prepared to treat colorectal cancers (Kufer et al., 1997, Cancer Immunol. Immunother. 45:193-197). Also, a bispecific antibody has been prepared to both CD3 and the idiotype of a membrane-bound IgM from tumor cells. The antibody was shown to eliminate tumors in BCL1 B cell lymphoma mice (De Jonge et al., 1997, Cancer Immunol. Immunother. 45:162-165). In addition, a bispecific scFv has been prepared that binds both CD3 and an antigen found on pancreatic carcinoma cells (EGP2; epithelial glycoprotein-2 C017-1A antigens, KSA). The resulting protein was effective in mediating tumor lysis in vitro (Helfrich et al., 1998, Int. J. Cancer 76:232-239).

These reports provide the basis for the strategy of using specific scFv for both CD3 and EGFR. The bispecific antibody to EGFR targets glioma cells, since EGFRs are not found in normal brain tissues but are found on about half of high grade gliomas (Snelling et al., 1995, Hybridoma 14:111-114; Faillot et al., 1996, Neurosurg. 39:478-483; Stragliotto et al., 1996, Eur. J. Cancer 32A:636-640). The other end of the bispecific scFv will bind to activated CD8⁺ T-lymphocytes to kill the glioma cells (see for example, Strategy II in Figure 11). In developing this strategy, it should be noted that previous publications have shown that T-cells enter the central nervous system following trauma (Hirschberg et al., 1998, J. Neuroimmunol. 89:88-96) and have been observed there in patients having multiple sclerosis and meningitis as well as in normal controls (Vrethem et al., 1998, Acta Neurol. Scand. 97:215-220). Therefore, the bispecific antibody used should recruit cytotoxic T cells to gliomas. Also, administration of interleukin-2 together with activated T cells appeared to be of benefit to some patients (Haynes et al., 1995, Cancer 75:840-852). Therefore, the strategy is unlikely to produce adverse effects.

### Antibodies to EGFR for Therapy of Gliomas

Antibodies to EGFR for therapy of gliomas have been studied extensively and have been used in both Phase I (Faillot et al., 1996, Neurosurg. 39:478-483; Stragliotto, G., et al.,1996, Eur. J. Cancer, 32A:636-640) and Phase II ( Snelling et al., 1995, Hybridoma 14:111-114) clinical trials. The rationale for the therapy is based on the observations that many glioma cells are rich in EGFRs and antibodies to EGFR are concentrated in gliomas after intravenous administration. Based on these observations, ¹²⁵I-labeled monoclonal antibodies to EGFR were prepared and shown to be internalized by human glioma cells. Also, a preliminary Phase II clinical trial has been carried out (Snelling et al., 1995, Hybridoma 14:111-114) in which ¹²⁵I-labeled monoclonal antibody to EGFR was administered systemically to 60 patients with glioblastoma multiforme.

The results indicated that therapy was relatively safe and may have some benefit in the management of primary glioblastoma multiforme. However, in these studies, it was apparent that only about 1% of systemically administered antibody entered the brain (Snelling et al., 1995, Hybridoma 14:111-114).

Since this clinical trial was initiated, a tumor-specific variant of EGFR has been identified (Hills et al., 1995, Int. J. Cancer 63:537-543; Wikstrand et al., 1995, Cancer Res. 55:3140-3148; Wikstrand et al., 1997, Cancer Res. 57:4130-4140; Okamoto et al., 1996, Br. J. Cancer 73:1366-1372; Wikstrand et al., 1998, J. Neurovirol. 4:148-158; Schmidt et al., 1998, Int. J. Cancer 75:878-884; Reist et al., 1997, Nucl. Med. Biol. 24:639-647). The tumor-specific EGFR (named EGFRvIII) has an internal deletion and is present in about 50% of gliomas. Four groups of investigators have prepared monoclonal antibodies to EGFRvIII (Hills et al., 1995, J. Cancer 63:537-543; Okamoto et al., 1996, Br. J. Cancer 73:1366-1372; Wikstrand et al., 1998, J. Neurovirol. 4:148-158; Schmidt et al., 1998, Int. J. Cancer 75:878-884). There is considerable interest in initiating clinical trials with such antibodies that more specifically target the EGFR found on many glioma cells. These experiments could provide more effective and safer therapies for malignant gilomas.

The following experiments provided the foundation for the studies reported in this Example:

### Demonstration of successful transfection of test cells resulting in secretion of soluble Fas ligand

The successful transfection of COS cells was carried out to generate cells having a gene encoding and expressing a soluble form of FasL wherein the soluble FasL is secreted by the cells. Figure 12 is an image of a Western blot assay demonstrating synthesis and secretion of soluble FasL by COS cells transfected with a cDNA encoding soluble (truncated) FasL. The cDNA was contained in the plasmid pSecTag²/Hyg (Invitrogen, Carlsbad, CA; see Example 10 at Design and synthesis of gene constructs, herein). The cells were transiently transfected with lipofectamine and assayed by Western blotting as described by Rensing-Ehl et al. (see Figure 2 in Rensing-Ehl et al., 1995, Eur. J. Immunol. 25:2253-2258). Lane 1 depicts cell lysate (14 µg protein) obtained from transfected cells (about 5 x 10⁵ in 2 ml medium) incubated for 2 days. Lane 2: Same as in lane 1 following 4 days of incubation. Lane 3: cell lysate obtained from mock transfected cells incubated for 4 days. Lane 4: Medium (18 µl) not concentrated from transfected cells after incubation for 1 day. Lane 5: same as lane 4 following 2 days of incubation. Lane 6: Same as lane 4 following 4 days of incubation. Lane 7: Medium obtained from mock transfected cells after incubation for 4 days. The diffuse white bands in lanes 4 to 7 are large concentrations of albumin in the samples of medium that reduce the background for the Western blot.

It is evident from the data obtained in this experiment that a test cell line (COS) can be transfected with a gene to result in the expression and secretion by that cell line of a soluble version of Fas ligand.

### Fas ligand secreted by the test cells kills cells that contain Fas

In order to demonstrate the feasibility of transfecting hMSCs for the purpose of generating a glioma toxic protein in hMSCs for use in killing glioma cells in brain tissue, the following experiment was conducted. The results of this experiment are shown in Figure 13 as follows. Figures 13 a-d are images of a series of photomicrographs depicting inhibition of growth and killing of 293 cells (Fas⁺) by medium obtained from COS cells transiently expressing a cDNA encoding soluble FasL. About 5 x 10⁵ 293 cells were incubated with 1 ml fresh medium plus 1 ml of medium obtained from transfected COS cells at 48 hours following transfection. The 293 cells are shown at 3 days of incubation. The treatment reduced the viable cells by over 90%. Figure 13a depicts control 293 cells incubated with 1 ml of medium obtained from mock transfected-COS cells. Figure 13b depicts test 293 cells incubated with 1 ml of medium from COS cells expressing soluble FasL. Phase contrast magnification was x 100. Figures 13c and 13d depict the same cultures as Figure 13b at the higher magnification of x 200. The appearance of the cells suggests that they are apoptotic.

### Demonstration of successful transduction of mouse and human MSCs to express an exogenous gene.

An experiment using a trial gene, tyrosine hydroxylase, was carried out to show that mouse and human MSCs could be genetically engineered to express a test exogenous gene. Figures 14 and 15 depict the results of those experiments and demonstrate that mouse and human MSCs could be successfully genetically engineered using a retroviral vector to express a test exogenous gene, tyrosine hydroxylase. Figure 14 is an image of a Western blot of rat MSCs transduced with a retrovirus (pLNCX) containing a test gene (tyrosine hydroxylase). Conditions for retrovirus transduction were as described herein at Example 10. The Western blot assay was carried out using cell lysates and an antibody for tyrosine hydroxylase. Lane 1: MW markers. Lanes 2-5: Cell lysates of transduced MSCs (5 or 10 µg protein). Lane 6: Cell lysates of untransduced MSCs. Lane 7: Cell lysate of control cells (293). Figure 15 is a graph depicting the synthesis and secretion of the product (L-DOPA) by human MSCs transduced with a retrovirus containing a test cDNA (tyrosine hydroxylase). The cells were incubated with the essential cofactor 50 µM tetrahydropteridine, and L-DOPA produced in the medium was assayed by HPLC. No L-DOPA was detected in medium obtained from control MSCs.

By transferring the promoter and cDNA for the soluble FAS ligand used in the experiments described in Figures 12 and 13 to the retrovirus used in the experiment described in Figures 14 and 15, MSCs can be prepared which secrete a toxic protein such as FasL or another glioma toxic protein as described in Figure 11. The effectiveness of such MSCs genetically engineered to secrete a glioma toxic protein in both inhibiting growth and killing glioma cells in culture can be evaluated as described in Example 10 herein: Further, the effectiveness of such MSCs genetically engineered to secrete a glioma toxic protein in both inhibiting growth and killing glioma cells in rats can be evaluated as described in Example 10 herein.

### Engraftment and migration of hMSCs in rat brain

As reported herein in Example 9, hMSCs obtained from human bone marrow engraft into rat brain and migrate along known pathways for the migration of neural stem cells (Figure 16). More recently, it was discovered that if astrocyte enriched cultures were prepared from the brains of the recipient rats, one could recover a small fraction of the human cells in the cultures. Moreover, a smaller fraction of the human cells have the morphology of early astrocytes and contain glial fibrillary acid protein, a marker for early astrocytes (Figure 10). As indicated in Table 4, assessment of brain sections obtained from the rats which received the bone marrow engraftment indicated that 15% of the human cells were still present after 120 days. Assessment of astrocyte-enriched cultures obtained from the rat brains indicated that about 5% of the cells in the cultures were human cells. Of the human cells in the cultures, about 1% stain positively with an antibody for glial fibrillary acidic protein (Table 5). None of the cells stained positively with an antibody to ED-1, and therefore the human cells were not microglia.

### Infused MSCs propagate during the growth of the brain in neonatal mice

In other experiments, mouse marrow stromal cells (mMSCs) were prepared from mice and infused into the brains of 7-day old neonatal mice. About 50,000 of the mMSCs were infused into the subventricular germinal zone. The donor cells were detected by prelabeling of the nuclei with *bis*-benzamide (Sigma Chemical Co., St Louis, MO). After 12 and 45 days, donor cells were recovered at the injection site, but as in the rat experiments with hMSCs, they were also seen to migrate to large regions of the brain (Figure 16). At higher magnification, the cells could be seen to be aligning themselves in linear arrays and appeared to be migrating along white matter tracks within the external capsule, corpus callosum, anterior commissure, ventral hippocampal commissure, and along fiber bundles of the striatum (Figures 16 and 17). They were also evident in the striatum of the contralateral hemisphere. The number of cells present in the brain of one mouse killed after 12 days and another killed after 45 days was estimated by counting fluorescent nuclei in frozen sections of the brain as described herein. As indicated in Table 6, the number of donor cells increased about 40-fold as the brain of the neonatal mice grew. Assays of the DNA content in brains of mature mice indicated that they contained about 10⁸ cells. Therefore, at 45 days, the donor cells accounted for about 2% of the total cells of the brain.

Analysis of the mouse brains established that there was no evidence of tumor formation, and the mice did not develop any neurological signs or symptoms of tumors. Therefore, the propagation of the donor cells appears to be a process that parallels the propagation of the endogenous neural stem cells in the developing mouse brain.

### Assays of hMSCs for mRNA for Fas. FasL and EGFR

To determine whether or not hMSCs contain mRNA encoding Fas, FasL or EGFR, ³²P-labeled poly A RNA obtained from the hMSCs was used in a dot blot assay. There was no detectable mRNA specific for Fas, FasL or EGFR in hMSCs (data not shown). To confirm these results, RT-PCR assays were carried out on the mRNA. The results presented in Figure 18 demonstrate that low levels of mRNA specific for FasL were detected, but there was no evidence of mRNA specific for Fas.

### Experiments conducted in the C6 glioma model in rats

C6 rat glioma cells (about 100,000) were injected into the margin of the white and grey matter junction of the cerebral hemisphere on one side of a rat brain. After 2 weeks, frozen sections were prepared and stained with hemylotoxin and eosin. The C6 glioma cells were successfully implanted into the rat brain, as depicted in Figure 19. Figure 19 illustrates how the margins of the glioma were sharply delineated and that occasional glioma cells with bizarre nuclei were seen.

### Genetic engineering of hMSCs to express and secrete proteins toxic to glioma cells

The overall strategy of these experiments may be outlined as follows: (a) retroviral vectors are prepared which are designed to express secreted forms of three different tumor-toxic proteins (Figure 11); (b) the retroviral vectors are used to infect hMSCs; © stably infected hMSCs are screened to identify clones that secrete high levels of the recombinant tumor-toxic protein; and (d) high producing clones are assayed for their ability to differentiate *in vitro* into osteoblasts, chondrocytes and adipocytes to ensure that cells retain their pluripotentiality. The clones are then used in assays for glioma toxicity *in vitro* and *in vivo* as described herein.

Three different glioma-toxic proteins are used by way of example. The rationale for choosing each are described below.

### Fas Ligand (FasL) as a Glioma-Toxic Protein

FasL is a preferred gene for use in the present invention because: a) soluble FasL has been shown to induce apoptosis in malignant glioma cells but not in normal cells of the CNS (Weller et al., 1994, J. Clin. Invest. 94:954-964; Weller et al., 1995, Cancer Res. 55:2936-2944; Frei et al., 1998, J. Neuroimmunol. 87:105-113; Weller et al., 1998, Brain Pathol. 8:285-293);(b) cytotoxic soluble FasL was secreted by gene-transfected COS cells herein and, therefore, hMSCs are likely amenable to genetic engineering to generate cells which secrete soluble FasL; c) hMSCs express low levels of FasL mRNA and do not express Fas mRNA (Figure 18). Therefore hMSCs are unlikely to undergo apoptosis once genetically engineered to synthesize and secrete higher levels of FasL; (d) a therapy based on local infusion and engraftment of hMSCs to secrete soluble FasL avoids systemic toxicity but provides a long-term source of the protein. These experiments also provide the basis for subsequent experiments in which the expression of FasL in hMSCs and secretion of FasL therefrom can be regulated either by the presence in the cell of a suicide gene such as the herpes simplex thymidine kinase (Uckert, 1998, Hum. Gene Ther. 9:855-865) or a conditional promoter such as a tetracycline sensitive promoter (Steinmann et al., 1998, Arch. Virol. 143:3548).

### A Bispecific scFv specific for both EGFR and CD3 as a Glioma-toxic Gene/Protein

A bispecific scFv specific for both EGFR and CD3 as a glioma toxic gene is one preferred construct for use in the present invention because (a) the protein expressed thereby has been used to treat other malignancies and promising results have been obtained (Kufer et al., 1997, Cancer Immunol. Immunother. 45:193-197; De Jonge et al., 1997, Cancer Immunol. Immunother. 45:162-165; Bookman, Semin. Oncol. 25:381-396; Helfrich et al., 1998, Int. J. Cancer 76:232-239; Jost et al., 1996, Molec. Immunol. 33:211-219) and (b) the use of the gene is as applicable to the CNS as it is to peripheral tissues since T lymphocytes appear in the CNS in increased numbers during pathological changes in the CNS (Hirschberg et al., 1998, J. Neuroimmunol. 89:88-96) and further, infusion of activated T cells was beneficial in some patients (Haynes et al., Cancer 76:840-852). This gene can be further refined to regulate the production of the bispecific scFv using either a suicide gene or an inducible promoter as described above (Uckert et al., 1998, Hum. Gene Ther. 9:855-865; Steinmann et al., 1998, Arch. Virol. 143:3548).

### scFv antibodies to EGFRvIII as Glioma-Toxic Gene/Protein

This glioma toxic gene is also preferred because a) a large body of information about the potential dangers and feasibility of the use of this gene has already been obtained in the current phase II clinical trial using a ¹²⁵I-labeled monoclonal antibody specific for EGFR (Snelling et al., 1995, Hybridoma 14:111-114; Faillot et al., 1996, Neurosurg. 39:478-483; Stragliotto, Eur. J. Cancer 32A:636-640); b) this two-stage strategy in which engrafted transfected hMSCs are used to secrete scFv antibody to EGFR followed by administration of ¹²⁵I-labeled antibodies to mouse scFv, should provide more specific therapy than the currently used monoclonal ¹²⁵I-labeled antibody to EGFR; c) the use of the protein is likely to be more specific than the use of ¹²⁵I-labeled antibodies to EGFRvIII, since specificity is achieved both in the binding of the mouse scFv to the EGFR and in the binding of ¹²⁵I-labeled antibody to the mouse scFv.

In addition to the above, a retroviral vector may be used because such vectors reproducibly yield high levels of stable gene expression in cells into which they are introduced. Further, a wide variety of retroviral constructs are commercially available, and can be used in hMSCs. Marrow aspirates from the iliac crests of normal volunteers and marrow from vertebrate bodies obtained at autopsy of organ donors may be used to isolate hMSCs. Marrow from vertebral bodies may also be used because they provide a larger number of hMSCs (about 2 x 10⁹ per body instead of 10⁸ per marrow aspirate). Also, if hMSCs obtained from donor-vertebral bodies engraft in brain as hMSCs from iliac aspirates, they may be the preferred source for potential clinical trials.

### Design and Synthesis of Gene Constructs

In the case of the FasL construct, rat FasL cDNA in a plasmid (pBL-KA15) is described in Suda et al. (1993, Cell 75:1169-1178). The extracellular region of the gene (ATG plus nt 371 to 910; Suda et al., 1993, Cell 75:1169-1178) is PCR amplified, and the sequence is cloned. The cDNA is then transferred to a secretion vector (pSecTag2/Hygro; Invitrogen, Carlsbad, CA) that contains an Igκ chain secretion signal, CMV promoter, *myc* tag, and a hygromycin resistance gene. The sequences including the flanking genes are then transferred to a standard retrovirus vector (pLNCX; ClonTech Inc., Palo Alto, CA).

In the case of the scFv directed against EGFR, the retroviral vector pBabeNeo/scFv-225S is described in (Jannot et al., 1996, Oncogene 13:275-282). To improve secretion of the protein from cells, a glycosylation site is introduced as described by Jost et al. (Jost et al., 1994, J. Biol. Chem. 269:26267-26273). The scFv antibody gene directed against EGFRI is replaced with the gene for scFv against EGFRvIII because it is a glioma tumor specific variant of EGFR, as described in the references recited herein.

The bispecific scFv which is directed against both EGFR and CD3, is described in the references recited herein. This bispecific scFv directed against both CD3 and the FLAG epitope (Wickham et al.,1997, J. Virol. 71:7663-7669) is modified by replacing the anti-FLAG scFv sequences with sequences for scFv anti-EGFR or scFv anti-EGFRvIII in the retrovirus vector pLNCX for use herein.

### Retrovirus Transfection

Standard protocols are used for the retrovirus transfection. The conditions are comparable to conditions used to stably transfect mouse MSCs and the conditions others have used to stably transfect hMSCs (Gordon et al., 1997, Hum. Gene Ther. 8:1385-1394; Chuah et al., 1998, Hum. Gene Ther. 9:353-365; Bulabois et al., 1998, J. Hematother. 7:225-239). Vectors are stably transfected into the amphotropic murine packaging cell line (Retro-Pack; PT67; ClonTech, Inc. Palo Alto, CA). Constitutive virus producer clones are isolated by selection with G418 or hygromycin and viral titers are assayed on NIH-3T3 cells. The supernatant of high titer clones is used to infect hMSCs. Primary cultures are infected on three successive days, and the cells are placed under selection for 5 to 14 days to obtain stably expressing clones.

Recombinant gene expression is assayed first in RT-PCR assays using the known gene sequences for primers. The cell-free supernatant obtained from resistant cell clones is pooled and concentrated 45-fold using an Amicon stirring apparatus and then assayed by cytotoxicity for LN-229 and C6 glioblastoma cells (Frei et al., 1998, J. Neuroimmunol. 87:105-113; Barth 1998, J. Neurooncol. 36:91-102) to assess secretion of active FasL. To assess cells expressing scFv antibodies, the presence of specific antibodies directed against *myc* (Invitrogen) is detected in concentrated medium to detect the *myc* epitope that is expressed from the retroviral vector. Antisera to mouse scFv is prepared using the protein synthesized by transfected hMSCs and is used in the assays. Any problem of inadequate protein obtained from the hMSCs to prepare antisera in rabbits can be circumvented by preparing the recombinant protein in E. coli.

### Isolation and Expansion of hMSCs

The method for isolation of hMSCs is as described above in Example 9. The procedure is repeated for 3-15 passages. Similar conditions are used to isolate and expand hMSCs obtained from vertebral bodies from human donors.

There are life span variations in cultures of samples of hMSCs obtained from aspirates of iliac crests apparently due to sampling problems. The life-span is closely correlated with the efficiency of the initial samples to generate colony-forming units (CFUs), and CFU assays can be used to identify samples of hMSCs that will undergo up to 15 doublings in culture. To characterize CFUs, hMSCs expanded in culture to 70-90% confluency were harvested using trypsin-EDTA (GIBCO, Grand Island, NY) for 5 minutes at 37°C and were counted using a hemacytometer. A glass Pasteur pipetter was flamed at the tip to reduce its diameter. Cells were drawn through the narrowed pipette several times to ensure cell separation. Cells were then diluted 1:100 in complete medium (α-MEM; GIBCO; 20% lot selected FCS), 1 X Penicillin-Streptomycin (GIBCO), and 2 mM L-glutamine (GIBCO). About 100-200 cells were plated in 100 mm tissue culture dishes (Falcon, Becton Dickenson, Lincoln Park, NJ) and were incubated for 10-14 days at 37°C in 5% humidified CO₂. Media was aspirated and cells were washed with 1 x PBS and stained with 5% Crystal Violet in methanol for 10 minutes at room temperature. The stain was aspirated from the cells which were then washed twice with distilled water. Visible colonies were counted and CFUs were calculated as the percent of total cells plated.

### Assays for Differentiation/Pluripotentiality

The following conditions were used to assay differentiation of hMSCs into osteoblasts: hMSCs were cultured to 70-90% confluency in complete medium. Medium was replaced every 3 days with osteogenic medium (complete medium, 10⁻⁸ M dexamethasone, 0.2 mM ascorbic acid; Sigma Chemical Co., St. Louis, MO), and 10 mM betaglycerol phosphate (Sigma Chemical Co., St. Louis, MO). Deposition of mineral was usually observed after 2-3 weeks. Cultures were washed with PBS and fixed in a solution of ice-cold 70% ethanol for 1 hour for staining with Alizarin red (AR-S) and to quantify calcium mineral content. Cultures were rinsed with-Milli-Q purified water and stained for 10 minutes with 40 mM Alizarin red-S, pH 4.2 (1 ml/35 mm plate) with rotation. The cultures were then rinsed 5 times with water, followed by a 15 minute wash with PBS (with rotation) to reduce non-specific AR-S stain. The stained cultures were photographed followed by a quantitative extraction procedure using 10% w/v cetylpyridinium chloride (CPC) in 10 mM sodium phosphate, pH 7.0 for 15 minutes at room temperature. Aliquots of these AR-S extracts were diluted 10-fold in the 10% CPC solution and the AR-S concentration determined by absorbance measurement at 562 nm on a spectrophotometer. Values were normalized for total DNA content assayed with a binding dye (Hoechst 33258, Hoechst Marion Roussel, Kansas City, MO).

Differentiation of hMSCs into adipocytes was assessed using the following method: hMSCs were cultured to 95% confluence in complete medium. Medium was replaced every 3 days with MHI cocktail containing 0.5 µM hydrocortisone, 125 mM IBMX, and 35 mM indomethacin. Cells containing lipid vacuoles were observed after 2-3 weeks. The presence of fat was detected by Oil red-O staining (Sigma Chemical Company, St. Louis, MO). Cells were washed with PBS and fixed in 10% formalin for 10 minutes and were stained in fresh Oil red-O solution (3 parts Oil red-O stock [0.5% Oil red-O in 99% isopropyl alcohol], 2 parts distilled water, mixed for 5 minutes and filter sterilized for 10-15 minutes. Plates were washed 3 times with water. The number of colonies containing one or more adipocytes and the number of adipocytes per colony (mean ± S.D.) were counted.

The results are evaluated on the basis of whether adequate levels of secretion of the desired recombinant protein sufficient to kill glioma cells or to limit their life span, are observed in the transfected cells.

### Testing the effectiveness of genetically engineered hMSCs in culture

Standard protocols that have been used extensively by others are used in these experiments, but instead of adding a potentially tumor toxic reagent to the cultures, hMSCs engineered to secrete the tumor-toxic protein are added.

### Glioma Cell sources

Rat C6 glioma cells, with and without the β-galactosidase (lac Z) gene inserted therein, and human T98G glioma cells were obtained from the American Type Culture Collection (ATCC). In addition, primary cultures of 13 human glioblastoma multiforme tumors with a confirmed pathologic diagnosis were prepared for use. Additional glioma cell lines LN-18, LN-215, LN-229 LN-308, LN-319 and LN-405 which can be used are described in Weller et al. (1995, Cancer Res. 55:2936-2944).

### Assays of Tumor Toxicity

Standard assay conditions are used such as those described in Frei et al. (1998, J. Neuroimmunol. 87:105-113). Ratios of hMSCs to human glioma cells ranging from 1:10,000 to 1:1 are used. The killing of hMSCs is distinguished from killing of the gliomas by prior nuclear-labeling of either cell type with *bis*-benzamide (Sigma Chemical Co., St. Louis, MO) as previously described herein. To assess the effects of hMSCs secreting scFv anti-EGFR (Strategy III in Figure 11), a more complex assay may be used. The effects of the cells secreting scFv anti-EGFR are examined with and without the addition of ¹²⁵I-labeled rabbit IgG against mouse scFv. The ¹²⁵I-labeled IgG is added at a concentration of 0.039 to 20 µCi/ml (Reist et al., 1995, Cancer Res. 55:4375-4382) at 12 or 48 hours after the beginning of the incubation of the cell mixtures. Cell binding of the anti-mouse scFv is assessed using an FITC tag and immunofluoresence microscopy and radiolabeling as described by Reist et al. (Reist et al., 1995, Cancer Res. 55:4375-4382). The hMSCs which secrete the scFv anti-EGFR are not assayed for apoptosis because ¹²⁵I alone causes chromosomal damage.

To assess cytotoxicity (Frei et al., 1998, J. Neuroimmunol. 87:105-113), the cell mixtures are seeded in 24-well plates (Falcon) at a density of 2 x 10⁵ cells per well in 0.5 ml DMEM containing 10% heat-inactivated FSC. After incubation for 2 days to 1 week, the medium is discarded and the remaining adherent viable cells are stained with 0.3 ml of crystal violet (0.5% in 20% methanol) for 15 minutes, washed in tap water and air-dried. Cell viability is determined by eluting the dye with 0.3 ml 0.05 M sodium citrate/50% ethanol and 0.1 ml solution, the eluted dye solution is transferred to the well of a 96-well plate (Falcon), and measuring absorbance at 540 nm of the dye using a Titertek Multiskan plate reader (Flow, Rockville, MD). Survival is expressed as the percentage of cells surviving relative to control cultures and the killing rate is calculated using the following formula: % killing = 100% - % survival.

To assess the effects (size of the colony and cell death) on tumor colony formation in soft agarose (Frei et al., 1998, J. Neuroimmunol. 87:105-113), each well of a 24-well culture plate (Falcon) is coated with a 0.4 ml base layer containing 0.7% low gelling seaplaque agarose. Cell mixtures (6 x 10⁵) are resuspended in 1.5 ml DMEM containing 20% FCS, 2 mM N-acetyl-L-glutamine, 1% nonessential amino acids, 20 µg/ml gentamycin, 0.3% low gelling seaplaque agarose before plating 0.5 ml over replicate base culture layers. Culture plates are then transferred to a refrigerator (4°C) for 15 minutes, then to room temperature for 10 minutes and then into a culture incubator at 37°C. Duplicate cultures are prepared for each tumor and sample tested. After 4 to 7 weeks of incubation, the colonies are stained with neutral red and counted using an inverted microscope. Clones containing more than 30 cells are counted as colonies and those having less than 30 cells are counted as clusters. Neutral red uptake is also measured. Before staining the colonies, the agarose cultures are carefully rinsed with 0.1 N HCl. Neutral red solution (3.3 mg/ml; Sigma Chemical Co., St. Louis, MO) is used at 0.1 % in 0.2 N HCl and the colonies are incubated for 15 minutes at room temperature. The colonies are then carefully rinsed twice with 0.1 N HCl and the neutral red positive colonies are counted as described above. The plating efficiency is calculated using the following formula: % plating efficiency = [number of colonies grown/number of cells plated] x 100.

Apoptosis in the cells is also assessed using the conditions of Frei et al. (1998, J. Neuroimmunol. 87:105-113) as follows: The detection of single-and double-stranded DNA breaks in the ex vivo tumor cells is performed using the *in situ* cell death detection kit (Boehringer Mannheim, Indianapolis, IN) according to the manufacturer's instructions. Briefly, 3 x 10⁶ cells are cultured in 25 cm² flasks (Falcon). Thereafter, the floating cells as well as the adherent cells are transferred to a conical 50 ml tube (Falcon) and washed in PBS (pH 7.4) by centrifugation for 5 minutes at 300 X g. The pelleted cells are fixed in 0.2 ml PBS-buffered 2% formaldehyde solution for 30 minutes at room temperature on a horizontal shaker. The cells are washed in PBS and then permeabilized by incubating them in 0.1 ml Triton-X100 (0.1%, w/v)/sodium citrate (0.1%, w/v) for 2 minutes at 4°C. Prior to commencing the TUNEL reaction-(Boehringer-Mannheim), the cells are washed two times. The TUNEL reaction mixture (50 µl) contains 0.3 nmol FITC-12 dTP, 3 nmol dATP, 25 units TdT and cobalt chloride. Negative control reactions are performed by omitting cobalt chloride. The reaction is performed at 37°C for 1 hour and stopped with the addition of 2 µl 0.5 M EDTA. The cells are washed once in PBS and analyzed using an Epics profile analyzer.

The effects of sample treatments are compared by student's t-test. Composite treatments are compared by ANOVA. The critical parameter is the ratio of hMSCs to glioma cells required to obtain significant effects.

### Testing the effectiveness of the genetically engineered hMSCs in a rat model for the disease

In this section, standard protocols which have been extensively employed by others (Barba et al., 1993, J. Neurosurg. 79:729-735) are used, but with the modification that varying amounts of genetically engineered hMSCs are injected at various time points from 0 to 14 days after glioma cells are injected into the brains of rats.

### Rat Model and administration of hMSCs

Brain-tumors are created using essentially the same protocols previously described herein. Adult Sprague Dawley albino rats (200 to 300 g) are anesthetized using 3% halothane in oxygen and maintained by intramuscular injection of a mixture of 6 mg/kg xylozine and 60 mg/kg ketamine. The rats are placed in a stereotaxic apparatus and two burr holes are made 3 mm lateral and 2 mm anterior to the bregma. About 10 µl of the glioma cell suspension (50,000 to 75,000) is slowly infused into each site over a 30 minute period into the margin of the grey and white matter junction of the cerebral hemisphere. The wound is closed with interrupted surgical sutures, and the animals are treated with 0.6 mg/kg of xylozine and 6 mg/kg of ketamine. At time points ranging from 0 to 14 days later, 10 µl of genetically engineered hMSCs (10,000 to 100,000 cells) are injected into one site and an equal injection of wildtype hMSCs is made into the second tumor site to serve as a control. The rats are examined daily for neurological symptoms of lethargy and paresis. The rats are killed after 14 to 21 days at which time the control C6 gliomas are 6-10 mm in diameter. The rats are anesthetized with xylozine and ketamine and then perfused intracardially with ice-cold phosphate buffered saline, followed by 3% buffered paraformaldehyde, and 10% sucrose. The brains are removed, the forebrains are trimmed, and the samples are immediately frozen.

### Assays

The effectiveness of the genetically engineered MSCs in reducing or treating gliomas is measured by (a) stereological analysis of the tumor volume; (b) long time survival of the rats; (c) counting fluorescently pre-labeled glioma cells or hMSCs in serial sections of the brain; and (d) isolation of nuclei from the brains and counting of fluorescently pre-labeled glioma nuclei. The recombinant proteins produced therein can be detected by immunohistology.

### Assessment of tumor volume

This assessment is performed using previously described protocols (Barba et al., 1993, J. Neurosurg. 79:729-735). The rats are killed and perfused with 4% paraformaldehyde. Individual brains are harvested, placed in 4% paraformaldehyde for 24 hours and then stored in 30% sucrose at 4°C until saturated. The brains are serially sectioned using 40 µm sections; every fifth section is mounted and treated with Nissl stain to aid in tumor identification. Stained brain sections having tumor tissue are examined and tumor volumes are quantified stereologically. By examining every fifth serial 40 µm section, each 100 µm section of the brain is scanned for the presence of tumor. The entire volume of tumor within the brain is quantified microscopically by counting grid dots over the tumor from a calibrated dot-matrix grid overlay. The volume represented by each dot is calculated as the product of the area covered by each dot multiplied by the thickness of brain represented by each slice, and the total tumor volume is calculated as the sum of the tumor volumes measured in the individual brain sections. Tumor volumes are plotted in graph form and compared via Mann-Whitney U-test nonparametric statistical analysis.

### Assay for long-term survival

These experiments (Barba et al., 1993, J. Neurosurg. 79:729-735) are carried out using rats in which giloma cells are infused into one site only followed by infusion of either genetically engineered or wildtype hMSCs into the same site. The rats are evaluated over 90 days. The animals are observed daily, the day of death is recorded, and the presence of tumor is verified histologically. Results are plotted as a survival curve.

### Detection and characterization of glioma cells and hMSCs in brain sections

Either the glioma cells or the hMSCs are prelabeled with 1 µg/ml *bis*-benzamide for 24 hours before infusion into the brain as previously described herein. The rats are killed and perfused as described above. The brains are frozen in isopentane dry ice and 10 µm sections are prepared. Frozen sections are attached to gelatin coated slides and are quickly immersed in cold acetone for 5 minutes and are stored at -20°C for further processing. Fluorescently labeled cells are visualized and photographed using a fluorescent microscope. The number of fluorescently labeled nuclei are counted in 8-10 tissue sections cut from rostral to caudal limits of the striatum. The procedure is repeated on each brain by two investigators who use alternate sections. Only the clearly labeled nuclei are counted. Dead and lysed cells leave a bluish hue in the surrounding tissue and no clear nuclear staining. The observed numbers are extrapolated to total number of sections to estimate the number of surviving engrafted cells.

To confirm the assays of glioma cells or hMSCs, nuclei are isolated from the brains (Ausubel, 1998, Current Protocols in Molecular Biology, 1, 4.10.6, John Wiley and Sons, Inc.). After infusion of either labeled glioma cells or labeled hMSCs the brains are cut into small pieces at 4°C and transferred to an ice-cold Dounce Homogenizer. The tissue and cells are broken with 5-10 strokes of a B pestle or until the nuclei appear to be free of cytoplasmic tags as measured by phase contrast microscopy. The nuclei are transferred to a clean 50 ml conical polypropylene centrifuge tube and 4 ml of sucrose buffer II is added (2 M sucrose, 5 mM magnesium acetate, 0.1 mM EDTA, 1 mM DTT in 10 mM Tris-HCl buffer, pH 8.0). The sample is mixed by gentle pipetting and inversion. Sucrose buffer II (4.4 ml) is added to a swinging bucket centrifuge tube (polyallomer SW 40.1 tube), the nuclear layer is added onto the sucrose cushion and the tube is topped off with sucrose buffer I (0.32 M sucrose, 3 mM CaCl₂, 2 mM magnesium acetate, 0.1 mM EDTA and 1 mM DTT and 0.5% NP-40 and 10 mM Tris-HCl buffer, pH 8.0). The sample is centrifuged for 45 minutes at 30,000 x g in an SW 40.1 rotor at 4°C. The pellet containing nuclei is suspended in 0.2 ml ice-cold glycerol storage buffer at about 5 X 10⁷ nuclei per ml. The sample is stored frozen at -70°C in liquid nitrogen. For the assay, the thawed suspension of nuclei is placed in a hemocytometer. The labeled nuclei are counted using an ultraviolet microscope and total nuclei are counted using phase contrast microscopy.

### Assay for recombinant protein secretion

To detect the glioma toxic protein secreted by the hMSCs, the brains are snap frozen by placing them in isopentane-dry ice (-22°C), sectioned, and the sections are fixed in cold acetone. The sections are then stained with primary antibodies such as goat anti-rat FasL (Santa Cruz) or goat anti-mouse IgG (Jackson). These antibodies may be obtained from Jackson Labs, Chester, PA) The secondary antibodies are the appropriate FITC-labeled antibodies (Sigma Chemical Co., St. Louis, MO).

The experiments can be repeated under the same conditions after longer time periods. For example, the rats can be evaluated and killed after 1 to 6 months for gene-engineered hMSCs shown to be effective in these experiments.

Treated gliomas and mock-treated control gliomas are consistently compared from the same rats. Data from all the assays is evaluated using the student's t-test.

**Table 4: The Number of Fluorescently Labeled hMSCs in Rat Brains**

| Donor cells were assayed by pre-labeling the cells with bis-benzamide and then counting fluorescently labeled nuclei in brain sections | | |
|---|---|---|
| Days after Implantation | Number of Rats | Labeled Human Cells/Brain |
| 5 | 3 | 28,500 |
| 14 | 4 | 27,000 |
| 30 | 4 | 30,500 |
| 72 | 5 | 18,000 |
| 120 | 2 | 14,000 |

**Table 5: Recovery of Human Cells from Astrocyte-enriched Cultures from Brain Recipient Rats**

| Human cells were detected both by the presence of fluorescently labeled nuclei and positive staining with antibodies to HLA-ABC | | | | |
|---|---|---|---|---|
| Days after Implantation of | Number Rats | Cells per Flask^{a} | Human cells (% of total) | GFAP positive Human Cells (% human cells) |
| 7 | 3 | 5 x 10₆ | 3.5 x 10⁵ (7%) | 3,500 (1%) |
| 14 | 2 | 3 x 10₆ | 1.5 x 10⁴ (5%) | 1,500(1%) |
| 30 | 2 | 4 x 10₆ | 1.2 x 10⁴ (3%) | 1,200 (1%) |
| 60 | 3 | 1 x 10₆ | 5 x 10⁴ (5%) | 250 (0.5%) |
| 90 | 2 | 2 x 10₆ | 6 x 10⁴ (3%) | 600 (1%) |

| | | | | |
|---|---|---|---|---|
| ^{a} = avg. of 4 to 6 flasks obtained from 2 or 3 rats | | | | |

**Table 6: Propagation of mMSCs after Infusion into the Brains of Neonatal Mice**

| Donor cells were detected by pre-labeling with bis-benzamide and counting fluorescently labeled nuclei in brain sections | |
|---|---|
| Days After Infusion | Donor Cells Recovered |
| 0 | 50,000 |
| 12 | 500,000 |
| 45 | 2,000,000 |

### Example 11: Marrow stromal cells behave as neural stem cells following injection into neonatal mouse brains

Stem cells are characterized by a capacity to self-renew and generate progeny capable of differentiating into multiple, yet distinct cell lineages. While stem cells derived from early embryos can differentiate into all somatic cell types (Evans and Kaufman, 1981, Nature 292:154; Martin, 1981, Proc. Natl. Acad. Sci. USA 78:7634; Pederson, 1994, Reprod. Fertil. Dev. 6:543), those derived from adult tissues are thought to produce only the cell lineages characteristic of the tissues wherein they reside. For example, hematopoietic stem cells resident in bone marrow give rise to only blood elements (Morrison, 1994, Annu. Rev. Cell Dev. Biol. 11:35). Stem cells are also restricted in their distribution within adult tissue, and are found only in bone marrow, gut, gonads, skin, and brain (Hall and Watt, 1989, Development 106:619; Morrison et al., 1997, Cell 88:287). In the latter case, lack of accessibility limits the utility of these stem cells for treating diseases of the central nervous system.

Recently, several reports have challenged the view that the differentiation potential of stem cells in adult tissues is lineage restricted (Tajbakhsh et al., 1994, Neuron 13:813; Bjornson et al., 1999, Science 283:534). If, in fact, such stem cells are totipotent, and their differentiation potential manifested by a response commensurate to specific cues in the local microenvironment, then it may be possible to reconstitute a tissue using stem cells from a separate dermal origin.

The experiments of this Example were designed to determine whether stem cells from bone marrow can adopt neural cell fates in brain by injecting marrow stromal cells (MSCs) into the subventricular germinal zone (SVZ) of neonatal mice.

### Immunodepletion method for isolating a uniform population of MSCs

Murine MSC cultures were established from the bone marrow of female FVB/N mice (Jackson Labs, Bar Harbor, ME) and cultured for 7 to 10 days (Phinney et al., 1999, J. Cell. Biochem. 72:570). For immunocytochemistry, cells cultured in single-well chamber slides (Falcon) were washed with PBS, air dried, fixed for 2 minutes in ice cold acetone, air dried again, and then either stained with Geimsa, or incubated with a rat anti-mouse CD 16/CD32 (Pharmigen Fc block) at a dilution of 1:50 for 30 minutes, then with a biotinylated CD11b antibody (Pharmigen) at a dilution of 1:200, and finally with a FITC-conjugated anti-biotin antibody (Dako) at a dilution of 1:100. Slides were counterstained with ethidium bromide.

Alternatively, cells were treated with 0.25% trypsin for 5 minutes at room temperature and then harvested by gentle scraping. Approximately 2-2.5 x 10⁶ cells were incubated for 60 minutes at 4°C with a slurry of avidin coated paramagnetic beads (PGC) conjugated to a biotinylated anti-CD11b antibody (Pharmigen). The final concentration of the beads was 0.05 mg/ml and the ratio of antibody to beads was 10 µg/mg. Cells not bound to the beads were harvested, washed with α-minimal essential media (MEM), and then plated on single-well chamber slides. After 48 hours cells were stained as described above. All photographs were produced using a Nikon Otpiphot-2 microscope.

### Fluorescent labeling of mMSCs with bis-benzimide

Seven day old mouse MSC cultures were incubated overnight with 10 µg/ml bis-benzimide (Sigma), and then MSCs isolated by immuno-depletion were counted on a hemocytometer and resuspended in serum free α-MEM at a concentration of 10,000 cells/µl. Using bregma as a landmark, approximately 50,000 cells in a total of 5 µl were slowly injected over a period of 5 minutes into the SVZ of cryo-anesthetized 4 day old mice using a steroetactic device. At the time points indicated, mice were sacrificed, perfused with 4% paraformaldehyde, and cryoprotected with 30% sucrose in PBS. Brains were excised and grossly dissected into four equal sized coronal sections. Each section was placed in OCT compound, frozen, and cryosectioned at 10 µm thickness. A total of three animals were taken at each time point for histological analysis. In more than 18 animals that received MSC injections, only. one was found to exhibit no donor cell engraftment in brain.

### Calculating the amount of donor cells in mouse forebram

The area covered by labeled nuclei in coronal serial sections was first calculated using a microscopy reticule at 100x magnification. This area was then divided by the area of an ocular field at 400x magnification. The resulting number was then multiplied by the number of in-phase, fluorescent nuclei counted per ocular field (n+5) at 400x magnification for every fifth, 10 µm section. Based on this method, the estimated number of donors cells in forebrain were found to equal 100,000 at 3 days, 600,000 at 12 days, and 2 x 10⁶ at 45 days post-injection following examination of a single animal for each time point.

### Cell Labeling with 8-bromo-2-deoxyuridine (BrdU)

MSC cultures grown for 5 days were pulsed with 5 µM 8-bromo-2-deoxyuridine in α-MEM supplemented with 10% FBS for 48 hours, washed several times with PBS and immuno-depleted as described above. Three animals were used for each time point in the histological analysis.

### Assessment of side effects from the injection of mMSCs

A minimal reactive gliosis was evident in animals receiving mMSC injections, but was confined to the margins of the injection tract. This gliosis was also evident in sham experiments wherein animals received injections of saline only. None of the animals injected with MSCs showed any overt behavioral abnormalities up to 90 days post-injection, which is the longest timepoint yet established. Moreover, sister brother matings of experimental animals confirmed that females receiving MSC injections could successfully rear offspring.

### Staining for GFAP and BrdU

Mice were perfused with 4% paraformaldehyde and processed for paraffin embedding. Immunohistochemistry for BrdU was carried out using a 1:500 dilution of anti-BrdU antibody (Dako, Inc.) as described previously (Gage et al., 1995, Proc. Natl. Acad. Sci. 92:897) except that the formic acid pre-treatment was omitted. BrdU stained sections were then double labeled for GFAP. Sections were treated for 10 minutes with 0.25% trypsin, followed by a 15 minute blocking step with 20% normal goat serum. Rabbit anti-GFAP at a 1:250 dilution (Dako, Inc.) was then applied for 30 minutes at room temperature. Primary antibody was detected by gold labeled goat anti-rabbit immunoglobulin at a 1:10 dilution followed by silver enhancement for 30 minutes (Polysciences, Inc.)

MSCs are capable of expansion, renewal, and differentiation into multiple mesenchymal. cell lineages (Owen, 1988, J. Cell Sci. Suppl. 10:63; Aubin, 1998 J. Cell. Biochem. Suppl. 30:73), and thus fulfill the basic criteria of stem cells. Typically, MSCs are isolated from bone marrow by their adherence, to plastic (Prockop, 1997, Science 276:71). We previously showed that murine MSC cultures established from various inbred mouse strains by this method represent a heterogeneous population dominated by myelopoietic cells that express the cell surface receptor CD11b (Phinney et al., 1999, J. Cell. Biochem. 72:570). These observations enabled us to develop a reliable method based on immunodepletion for isolating a uniform population of fibroblastoid-shaped MSCs as depicted in (Figure 20). Following purification, these cells showed no immunoreactivity with CD45, indicating that the population is devoid of hematopoietic cells (data not shown).

Three days after the injection into mouse brain of approximately 50,000 mMSCs labeled with the fluorescent DNA binding dye bis-benzimide, direct examination of cryostat sections by fluorescence microscopy revealed dye-labeled donor cells in the SVZ and lining the later and third ventricles along the neuraxis, extending rostrally into the olfactory bulb. A small number of cells were also evident lining the corpus collosum and choroid plexus. These results are depicted in (Figure 21A). Occasional donor cells were also found in the contralateral hemisphere. Frozen sections from animals that received injections of 50,000 bis-benzimide, labeled cells that were lysed prior to injection contained no detectable fluorescent nuclei, which indicated that bis-benzimide is not passively transferred from donor to host cells.

At 12 days after injection, donor cells were most widely distributed proximal to the injection tract at +0.9 bregma, and had expanded into the ipsilateral striatum, spanning from the anterior commissure up through the external capsule and into the cingulate cortex, as depicted in (Figures 21a-c). Many donor cells were also still evident lining the lateral ventricle and the choroid plexus. At 45 days after transplantation, donor cells had disseminated throughout the striatum-as depicted in (Figure 21d). Moving rostrally from the injection site, donor cells extended beyond the external capsule, through the sematosensory cortex, and along the arachnoid matter, as depicted in (Figure 21a).

Donor cells were also seen in the corpus collosum and the interior and ventral hippocampal commisures as depicted in (Figure 21e). Within these established white matter tracts, donor cells were aligned in parallel, radial arrangements as depicted in (Figure 22), indicating that their spaciotemporal distribution throughout the brain was mediated by an ordered process of migration rather than simple diffusion. Increasing numbers of dye-labeled cells were evident in the contralateral hemisphere, as well. Estimates based on direct counting of fluorescent nuclei indicated that donor cell numbers expanded greater than 40-fold.

The marked proliferation of MSCs, together with their migration from the SVZ into striatal and cortical regions, mimics the events of gliogenesis; the post-natal colonization of forebrain by NSCs from the SVZ and their subsequent differentiation into macroglia (Levison et al., 1993, Development 119:611; Levison and Goldman, 1993, Neuron 10:201; Zerlin et al., 1995, J. Neurosci. 15:7238). Therefore, MSCs emulate aspects of neural stem cell behavior.

To probe this issue further, these experiments were repeated using cells labeled with 8-bromo-2-deoxyuridine (BrdU). At 12 days post-injection, BrdU-Iabeled donor cells were evident throughout the subventricular zone, striatum and external capsule of the ipsilateral hemisphere, as depicted in (Figure 23a-c), a distribution similar to that seen with bis-benzimide labeled cells.

Double-labeling revealed that glial fibrillary acid protein (GFAP) expression was virtually absent in donor cells within the SVZ, but was strongly expressed in a subset of donor cells within the striatum and the molecular layer of the hippocampus, as depicted in (Figure 23d). Based on morphology and their association with neurons, BrdU-labeled cells also appeared to differentiate into oligodendrocytes. These result extend previous findings which suggested that cells derived from bone marrow engraft in brain and give rise to macroglia (Eglitis and Mezey, 1997, Proc. Natl. Acad. Sci. USA 95:4080; Azizi et al., 1998, Proc. Natl. Acad. Sci. USA 95:3908) by demonstrating that mouse MSCs migrate along established routes and differentiate into neural cell lineages in a manner consistent with ongoing developmental events in neonatal forebrain.

In addition to donor-derived astrocytes and oligodendrocytes, BrdU-Iabeled cells were also found clustered within the Islands of Calleja, a neuron rich region in the ventral forebrain cortex, as depicted in (Figures 24a and b), suggesting that MSCs also differentiate into neurons. To confirm this, the distribution of MSCs in known regions of post-natal neurogenesis (Luskin, 1993, Neuron 11:173; Lois and Alvarez-Buylla, 1994, Science 264:1145; Jankovski and Sotelo, 1996, J. Comp. Neurol. 371:376; Altman, 1972, J. Comp. Neurol. 145:353; Hatten and Heintz, 1995, Ann. Rev. Neurosci. 18:385) was analyzed, and a significant proportion of BrdU-Iabeled cells were found in both the olfactory bulb and cerebellum, as depicted in (Figure 24c-f). In the latter case, donor cells were found specifically within the subependyma of the fourth ventricle, the external granular layer (EGL), and the internal granular layer (IGL) as depicted in (Figures 24e and f). The majority of these cells did not express GFAP, and therefore were not astrocytes. Their specific localization and abundance indicates that they are not microglia, which are sparsely populated in these regions. Rather, the distinct distribution pattern of BrdU-labeled cells in this region is analogous to that expected for neuroprogenitors, which during post-natal cerebellar development undergo clonal expansion within and subsequent migration from the EGL to the IGL where they become neurons (Jacobson,1991, Developmental Neurobiology, Plenum Press, New York; Zhang and Goldman, 1996, Neuron 16:47). This is also consistent with the finding that perkinje cells adjacent to the IGL, which mature during embryogenesis and are non-mitotic in post-natal brain (Altman and Bayer, 1978, J. Comp. Neurol. 179:23; Altman and Bayer, 1985, J. Comp. Neurol. 231:42), were exclusively of host origin.

### Summary

Collectively, these data demonstrate that mouse MSCs mimic the behavior of neural stem cells in mouse brain by participating in aspects of normal brain development, including proliferation, migration along established routes, nondisruptive interspersion within striatal, cortical, and cerebellar regions, and differentiation into neural cell lineages. The latter also demonstrates that mMSCs are capable of producing differentiated progeny of a different dermal origin; a potential apparently unmasked by exposure to the developing neonatal brain microenvironment.

One factor in brain that may mitigate this behavior of MSCs is FGF2. This polypeptide growth factors is expressed in germinal zones within developing brain where it induces the proliferation of bi-potential neuronal/astroglial precursors as well as multi-potential NSCs (Caday et al., 1990, Brain Res. Develop. Brain Res. 52:241; Grothe and Meisinger, 1995, Neurosci. Lett. 197:175; Hattori et al., 1997, Brain Res. Mol. Brain Res. 47:262). The fact that FGF2 is mitogenic for MSCs and also prevents their differentiation in vitro (Phinney et al., 1999, J. Cell. Biochem. 72:570; Oliver et al., 1990, Growth Factors 3:231; Locklin et al., 1995, Clin. Orthop. Rel. Res. 313:27), may be partially responsible for the dramatic expansion of these cells in brain. Other neurotrophic facts for which MSCs express receptors, such as NGF (Caneva et al., 1995, Blood Cells, Molecules & Disease 21:73), may also play a role.

The non-disruptive, widespread interspersion of MSCs in brain has important implications for cell therapy of CNS disease. MSCs can be readily isolated from small volume aspirates from the illiac crest, providing a replenishable source of autologous cells for transplantation. Moreover, the ability of these cells to differentiation into neural cell lineages suggests engraftment may occur in a functional manner.

### Example 12: Use of Genetically engineered rat MSCs for treatment in a rat model of Parkinsonism.

Rat MSCs (rMSCs) were genetically engineered to secrete L-DOPA as previously described herein (see Example 10) for mouse and human MSCs. Briefly, rMSCs were transduced with one retrovirus containing the gene for tyrosine hydroxylase (TH) and then a second retrovirus with the gene for GTP-cyclohydrase (GC). The GC gene provides tetrahydropteridine, an essential cofactor for tyrosine hydroxylase. Approximately 100,000 transduced cells were then engrafted into rat brain as previously described herein. Experiments were then conducted to determine whether the engrafted rMSCs would continue to synthesize and secrete L-DOPA after engraftment into the corpus striatum of rats with parkinsonism induced by 6-hydroxydopamine.

The levels of L-DOPA production after engraftment into rat brain were assessed by microdialysis in the striatum of a Sprague Dawley 6-hydroxydopamine lesioned rat followed by HPLC analysis at 3 days after engraftment of the genetically engineered rMSCs described above (TH+GC+). Dialysates were taken every 30 minutes for 3 hours. The results of these experiments are described in Figures 25 and 26. The data presented in Figure 27 indicate the metabolites of L-DOPA which are indicative of L-DOPA production. The results of these experiments indicate that L-DOPA was produced in the brain of the treated rat after engraftment of the transduced rMSCs at levels comparable to or higher than that obtained by others in comparable experiments in which either skin fibroblasts or astrocytes were employed (Lundberg et al., 1996, Exp. Neurol. 139:39-53; Beners et al.,1996, J. Neurosci. 16:4449-4456; Leff et al., 1998, Exp. Neurol. 151:249-264). No L-DOPA was detected in the brains of control rats.

The data presented in Figures 25, 26 and 27 further indicate that the L-DOPA synthesized by the transduced rMSCs in the rat brain was converted to the expected metabolites of both L-DOPA and dopamine.

A reduction in apomorphine-induced rotations, one of the accepted phenotypes of the rat model for parkinsonism, was also observed in the 6-hydroxydopamine lesioned rats after engraftment of the transduced rMSCs. Figure 28 contains the data obtained in these experiments. The rats were prepared by infusion of 6-hydroxydopamine into the corpus striatum to produce the parkinsonism model. Rotation was induced by injection of apomorphine (0.05 mg/kg, sc). Values shown were obtained 2 to 5 rats infused with rMSCs transduced to synthesize L-DOPA (+GC+TH) and 3 control rats infused with rMSCs transduced only with the gene for tyrosine hydroxylase (TH), rendering the cells unable to produce L-DOPA in culture without added tetrahydropteridine. The graph indicates that the effect on rotation disappeared after about 7 days, the same time at which L-DOPA was no longer detected in the microdialysis experiments shown in Figure 25.

Similar experiments can be carried out using rMSCs that are genetically engineered by methods such as electroporation of naked DNA or the use of self-inactivated retroviruses to result in rMSCs that do not lead to shutdown of the expressed gene in the central nervous system. This provides a method for prolonging the treatment for parkinsonism.

The disclosures of each and every patent, patent application and publication cited herein are hereby incorporated herein by reference in their entirety.

While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

## Claims

1. A method of treating a human patient having a disease, disorder or condition of the central nervous system, the method comprising obtaining a bone marrow sample from a human donor, isolating stromal cells from said bone marrow sample, and administering said isolated stromal cells to the central nervous system of said human patient, wherein the presence of said isolated stromal cells in said central nervous system effects treatment of said disease, disorder or condition.

2. The method of claim 1, wherein said human donor is not suffering from a disease, disorder or condition of the central nervous system and wherein said human donor is synergeneic with said patient.

3. The method of claim 1, wherein said human donor is said human patient.

4. The method of claim 1, wherein said disease, disorder or condition of the central nervous system is selected from the group consisting of a genetic disease, a tumor, trauma and stroke.

5. The method of claim 4, wherein said disease, disorder or condition is injury to the tissues or cells of said central nervous system.

6. The method of claim 4, wherein said disease, disorder or condition is a brain tumor.

7. The method of claim 1, wherein said isolated stromal cells administered to said central nervous system remain present or replicate in said central nervous system.

8. The method of claim 1, wherein prior to administering said isolated stromal cells, said cells are cultured in vitro.

9. The method of claim 1, wherein prior to administering said isolated stromal cells, said isolated stromal cells are transfected with an isolated nucleic acid encoding a therapeutic protein, wherein when said protein is expressed in said cells said protein serves to effect treatment of said disease, disorder or condition.

10. The method of claim 9, wherein said therapeutic protein is selected from the group consisting of a cytokine, a chemokine, a neurotrophin and antibody and a glioma-toxic protein.

11. The method of claim 1, wherein prior to administering said isolated stromal cells, said isolated stromal cells are transfected with an isolated nucleic acid encoding a therapeutic protein wherein when such protein is secreted by said cells said protein serves to effect treatment of said disease, disorder or condition.

12. The method of claim 11, wherein said isolated nucleic acid is operably linked to a promoter/regulatory sequence.

13. The method of claim 11, wherein said therapeutic protein is selected from the group consisting of a cytokine, a chemokine, a neurotrophin, an antibody and a glioma-toxic protein.

14. The method of claim 9, wherein said isolated nucleic acid is a wild type copy of a mutated, non-functioning or under-expressed gene, wherein said isolated nucleic acid is operably linked to a promoter/regulatory sequence and is expressed in said isolated stromal cells.

15. The method of claim 11, wherein said isolated nucleic acid is a wild type copy of a mutated, non-functioning or under-expressed gene, wherein said isolated nucleic acid is operably linked to a promoter/regulatory sequence and is expressed in said stromal cells to generate a protein which is secreted from said isolated stromal cells.

16. The method of claim 1, wherein prior to administrating said stromal cells, said cells are pre-differentiated by coculturing said stromal cells in the presence of a substantially homogeneous population of differentiated cells, or in the presence of an inducer of cell differentiation, whereby said isolated stromal cell differentiates and acquires the phenotypic characteristics of the differentiated cells or of the differentiation phenotype characteristic of a cell treated with the inducer.

17. The method of claim 1, wherein prior to administration of said isolated stromal cells at least one of the steps of culturing said cells in vitro, introducing isolated nucleic acid into said cells, and pre-differentiating said cells, is performed.

18. The method of claim 1, wherein said isolated stromal cells are immunologically isolated.

19. A method of directing the differentiation of an isolated stromal cell, said method comprising culturing said isolated stromal cell in the presence of a substantially homogeneous population of differentiated cells or in the presence of an inducer of cell differentiation, whereby said isolated stromal cell differentiates and acquires the phenotypic characteristics of the differentiated cells or of the differentiation phenotype characteristic of a cell treated with the inducer.

20. The method of claim 19, wherein said differentiated cells are selected from the group consisting of astrocytes, macroglial cells and neurons.

21. The method of claim 6, wherein said brain tumor is a glioma.

22. The method of claim 11, wherein said therapeutic protein is a glioma-toxic protein.

23. The method of claim 22, wherein said glioma-toxic protein is Fas ligand.

24. The method of claim 22, wherein said glioma-toxic protein is a bispecific single chain antibody directed against epidermal growth factor receptor (EGFR) and CD3.

25. The method of claim 22, wherein said glioma-toxic protein is an antibody directed against epidermal growth factor receptor (EGFR).

26. The method of claim 11, wherein said transfection is conducted using a retroviral vector.

27. The method of claim 9, wherein said method of administering said isolated stromal cells is by engraftment of the cells directly into the brain.

28. The method of claim 11, wherein said method of administering said isolated stromal cells is by engraftment of the cells directly into the brain.

29. The method of claim 7, wherein said isolated stromal cells administered to said central nervous system remain present or replicate in said central nervous system for up to about 150 days.

30. The method of claim 1, wherein after administering said isolated stromal cells to the central nervous system, about 0.1 % of said isolated stromal cells administered to the central nervous system are capable of expressing a marker for differentiation into macroglial cells, astrocytes or neurons.

31. The method of claim 30, wherein said marker for differentiation is glial fibrillary acidic protein.

32. The method of claim 1, wherein said disease of the central nervous system is Parkinson's disease.

33. The method of claim 9, wherein said therapeutic protein is an enzyme required in the biosynthesis of a neurotransmitter that is deficient or not produced in a disease, disorder or condition of the central nervous system.

34. The method of claim 11, wherein said therapeutic protein is an enzyme required in the biosynthesis of a neurotransmitter that is deficient or not produced in a disease, disorder or condition of the central nervous system.

35. The method of claim 33, wherein said neurotransmitter is L-DOPA.

36. The method of claim 34, wherein said neurotransmitter is L-DOPA.

37. The method of claim 35, wherein said treatment results in a decrease in an observed phenotype for Parkinson's disease.

38. The method of claim 37, wherein said observed phenotype for Parkinson's disease is apomorphine-induced rotation in a rat model for Parkinson's disease..

39. The method of claim 36, wherein said isolated stromal cells continue to synthesize and secrete L-DOPA for at least about 3 days.

40. The method of claim 36, wherein said L-DOPA is converted to metabolites of L-DOPA.
